(19) Europäisches Patentamt
European Patent Office
Office européen des brevets

(11) EP 4 392 412 B1

(12) **EUROPEAN PATENT SPECIFICATION**

(45) Date of publication and mention of the grant of the patent:
**17.06.2026 Bulletin 2026/25**

(21) Application number: **22768920.5**

(22) Date of filing: **26.08.2022**

(51) International Patent Classification (IPC):
***C07D 401/06*** (2006.01) ***C07D 403/06*** (2006.01)
***C07D 409/06*** (2006.01) ***C07D 413/06*** (2006.01)
***A61K 31/401*** (2006.01) ***A61K 31/404*** (2006.01)
***A61K 31/41*** (2006.01) ***A61K 31/498*** (2006.01)
***A61P 25/28*** (2006.01) ***A61P 35/00*** (2006.01)

(52) Cooperative Patent Classification (CPC):
**A61P 25/28; A61P 35/00; C07D 401/06; C07D 403/06; C07D 409/06; C07D 413/06**

(86) International application number:
**PCT/GB2022/052204**

(87) International publication number:
**WO 2023/026060 (02.03.2023 Gazette 2023/09)**

(54) **ABAD INHIBITOR COMPOUNDS**
ABAD-INHIBITORVERBINDUNGEN
COMPOSÉS INHIBITEURS D’ABAD

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.08.2021 GB 202112240**

(43) Date of publication of application:
**03.07.2024 Bulletin 2024/27**

(73) Proprietor: **University Court of The University of St Andrews**
**St Andrews, Fife KY16 9AJ (GB)**

(72) Inventors:
- **AITKEN, Laura**
  **Dairsie Fife KY15 4SU (GB)**
- **GUNN-MOORE, Frank**
  **Edinburgh EH10 4BR (GB)**
- **SMITH, Terry K**
  **St Andrews Fife KY16 9ST (GB)**

(74) Representative: **Marks & Clerk LLP**
**15 Fetter Lane**
**London EC4A 1BW (GB)**

(56) References cited:
**WO-A1-2007/024944** **WO-A1-2013/173206**
**WO-A2-2008/100457**

- **HROCH LUKAS ET AL: "Synthesis and evaluation of frentizole-based indolyl thiourea analogues as MAO/ABAD inhibitors for Alzheimer’s disease treatment", BIOORGANIC & MEDICINAL CHEMISTRY, ELSEVIER, AMSTERDAM, NL, vol. 25, no. 3, 27 December 2016 (2016-12-27), pages 1143 - 1152, XP029902227, ISSN: 0968-0896, DOI: 10.1016/J.BMC.2016.12.029**
- **XIE Y ET AL: "Identification of small-molecule inhibitors of the A@b-ABAD interaction", BIOORGANIC & MEDICINAL CHEMISTRY LETTERS, ELSEVIER, AMSTERDAM NL, vol. 16, no. 17, 1 September 2006 (2006-09-01), pages 4657 - 4660, XP027966727, ISSN: 0960-894X, [retrieved on 20060901]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## FIELD OF THE INVENTION

The present invention concerns Amyloid Binding Alcohol Dehydrogenase (ABAD) inhibitors that interact non-competitively with nicotinamide adenine dinucleotide + H (NADH). Inhibition of ABAD is useful in the treatment or prophylaxis of disease, including Alzheimer's disease (AD) and cancer. Accordingly, the present invention also concerns ABAD inhibitors for use as a medicament, specifically for use in the treatment of Alzheimer's disease and/or cancer.

## BACKGROUND OF THE INVENTION

Dementia is a neurological disease, which is currently estimated to affect over 850,000 people in the UK, with a cost to the economy of over £26 billion (current figures from www.dementiastatistics.org). At present there are no disease modifying therapies available to treat dementia. Rather, current treatments act to slow down the progression of the disease without treating the underlying causes.

Changes in the way our bodies produce energy are well reported in AD. As a person ages, the energy sources used by the person's brain use can switch from solely using glucose to utilising other sources. In AD, glucose metabolism is significantly decreased and stored fat is relied upon for energy production. The main function of ABAD, also known as 17β-hydroxysteroid type 10 (17β-HSD10), is to produce energy for the brain, and it does so via β-fatty acid oxidation when glucose levels are low, as is the case in AD. Energy production requires the correct functioning of structures within nerve cells but in the early stages of AD, such structures do not function efficiently. This abnormality, called mitochondrial dysfunction, can be caused by a build-up of amyloid (a hallmark of AD) within mitochondria, allowing amyloid to attach to ABAD. This does not occur in normal aging. When amyloid attaches to ABAD, it alters its function and causes a build-up of toxins and the activation of specific genes, all of which results in damaging changes in synapses, which impairs the memory capabilities of the brain.

ABAD was first identified as an amyloid beta peptide (Aβ) binding protein in 1997 (S. D. Yan et al. Nature, 1997, 389, 689-695). This finding was subsequently confirmed in a number of later studies (see, for example, J. W. Lustbader et al., Science, 2004, 304(5669), 448-452 and J. Yao et al., Mol. Cell. Neurosci, 2007, 35(2), 377-382). ABAD is known to interact with the two major plaque forming isoforms of Aβ, namely Aβ(1-40) and Aβ(1-42), leading to distortion of the enzyme structure and inhibition of its normal function as an energy provider for cells (see U. C. Oppermann et al., FEBS lett., 1999, 451(3), 238-242 and S. D. Yan et al., J. Biol. Chem., 1999, 274, 2145-2156). In vitro experiments have shown that the interaction between ABAD and Aβ is cytotoxic and ABAD's function is diminished resulting in a build-up of reactive oxygen species (ROS) and toxins, leading to mitochondrial dysfunction.

Using site directed mutagenesis and surface plasmon resonance, Lustbader et al. (2004, supra) identified the binding site for Aβ as the LD loop of ABAD, and subsequently synthesised a 28-amino acid peptide to mimic this region. This peptide is termed the 17β-HSD10-decoy peptide, or 17β-HSD10-DP. It was shown using surface plasmon resonance that 7β-HSD10-DP can bind to Aβ(1-40) and Aβ(1-42), thereby inhibiting their binding to ABAD. This translated into a cytoprotective effect in cell culture experiments: cultured wild type cortical neurons exposed to Aβ(1-42) showed a significant increase in cell death, as measured by cytochrome-c release, whilst those pre-incubated with 17β-HSD10-DP did not.

Direct modulation of ABAD activity is also reported to be effective in treating AD. In vitro experiments with SH-SY5Y (neuroblastoma cells) administered with the ABAD inhibitor, AG18051, show a reduction in mitochondrial dysfunction and oxidative stress associated with the interaction between ABAD and Aβ, and protect cultured SH-SY5Y cells from Aβ mediated cytotoxicity (Lim et al., PLoS One, 2011, 6(12), e28887).

Not only is ABAD an important target in AD, it has also been implicated in various forms of cancer, such as prostate cancer and notably in cases deemed castration resistant prostate cancer (CRPC). See P. R. Dillard, M. F. Lin and S. A. Khan, Mol. Cel. Endocrinol., 2008, 295(1-2), 115-120; E. Jernberg et al., PLoS One, 2013, 8(11), e77407; and E. Carlson et al., BMC Cancer, 2015, 15:166 for more details. Prostate cancer has the second highest mortality rate and accounts for approximately 29% of cancer cases in men, but the underlying causes for the disease are yet to be established. Prostate cancer is a hormone sensitive cancer, where androgens, namely testosterone and dihydrotestosterone (DHT), play a pivotal role in its development and progression. Testosterone and **DHT** promote cancer cell growth through their interaction with androgen receptors within the prostate (A. Zhang et al., Horm. Cancer, 2016, 7(2), 104-113). Current treatments of prostate cancer typically focus on reducing testosterone and DHT levels through chemical or surgical castration. However, such treatment is limited in patients suffering from metastatic CRPC. In these cases, levels of ABAD are significantly increased. ABAD is reported to oxidise the DHT metabolite 5α-androstane-3,17-diol (3α-androstanediol or 3α-diol) back to DHT, thereby generating significantly increased levels of DHT (Zhang *et al.,* 2016, *supra*). High amounts of DHT can increase the growth of prostate cancer and make it more difficult to treat.

In WO 2008/100457, a class of indolines as inhibitors of α2C adrenergic receptor agonists are described, in

addition to methods of preparing such compounds, pharmaceutical compositions containing one or more such compounds, methods of preparing pharmaceutical formulations comprising one or more such compounds, and methods of treatment, prevention, inhibition, or amelioration of one or more conditions associated with the α2C adrenergic receptors using such compounds or pharmaceutical compositions.

In WO 2007/024944, imidazole derivatives are described and reported to be inhibitors of alPha2C adrenergic receptor agonists. Methods of preparing the imidazole derivatives, pharmaceutical compositions containing them, methods of preparing such pharmaceutical formulations, and methods of treatment, prevention, inhibition or amelioration of one or more conditions associated with the alpha2C adrenergic receptors using the imidazole derivatives or pharmaceutical compositions are described.

In WO 2013/173206, benzothiazole phosphonate analogues and methods of using such analogues to inhibit the activity of Amyloid Binding Alcohol Dehydrogenase, and in the amelioration or treatment of Alzheimer's disease are described.

In Bioorganic & Medicinal Chemistry, 25 (2017), 1143-1152, L. Hroch et al. describe the design and synthesis of a class of asymmetrical disubstituted indolyl thioureas, designed to interact with amyloid-binding alcohol dehydrogenase (ABAD). ABAD activity evaluation was reported to not show any highly potent compound.

In Bioorganic & Medicinal Chemistry, 16 (2005), 4657-4660, Y. Xie et al. describe frentizole as an inhibitor of the Aβ-ABAD interaction and go on to identify a more potent benzothiazole urea inhibitor.

The present disclosure provides alternative ABAD inhibitors useful in the treatment or prophylaxis of conditions such as those disclosed herein. The ABAD inhibitors disclosed herein are especially useful in the treatment or prophylaxis of AD and prostate cancer (CRPC in particular).

## SUMMARY OF THE INVENTION

The inventors have found that the compounds disclosed herein are surprisingly effective ABAD inhibitors. Subsequently, the compounds disclosed herein have the potential to reverse memory deficits attributed to amyloid-ABAD interactions. The compounds disclosed herein interact non-competitively with NADH, offering a potentially greater enzyme specificity than some known ABAD inhibitors.

The skilled person is aware that any reference to an aspect of the current disclosure includes that aspect and/or any embodiment of that aspect. For example, any reference to the first aspect includes the first aspect and/or any embodiment of the first aspect.

Viewed from a first aspect, there is provided a compound of formula (I):

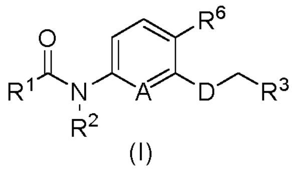

(I) ;

wherein $R^1$ is any one selected from the group consisting of $C_6$-$C_{10}$aryl, $C_3$-$C_5$heteroaryl, $C_{10}$-$C_{16}$biaryl, $C_6$-$C_{14}$biheteroaryl, $C_5$-$C_8$cycloalkyl, $C_3$-$C_7$heterocycloalkyl, $C_1$-$C_6$alkyl, $C_6$-$C_{10}$aryl$C_1$-$C_3$alkyl, $C_{10}$-$C_{16}$biaryl$C_1$-$C_3$alkyl and $C_3$-$C_8$cycloalkyl$C_1$-$C_3$alkyl optionally substituted one or more times with any one or a combination selected from the group consisting of halo, $C_1$-$C_4$haloalkyl, $C_1$-$C_4$alkyl, $C_1$-$C_4$alkoxy, $C_1$-$C_4$haloalkoxy, hydroxy and methylsulfonyl;

$R^2$ is any one selected from the group consisting of iso-butyl, iso-propyl, cyclobutyl, cyclopropyl, cyclobutylmethyl, cyclopropylmethyl, methoxyethyl, isopropoxyethyl, and di($C_{1-4}$alkyl)amino$C_{1-3}$alkyl;

A is CH or N;

$R^3$ is any one selected from the group consisting of $C_2$-$C_5$heteroaryl optionally substituted one or more times with any one or a combination selected from the group consisting of $C_1$-$C_4$alkyl, $C_3$-$C_5$cycloalkyl, halo, hydroxy and $C_{1-3}$alkoxy; ethyne, hydroxymethyl, amido, N-methylamido and cyano;

D is $NR^7$ or O, wherein $R^7$ is $C_{1-3}$alkyl or $C_{1-3}$alkylol; and

$R^6$ is H; or

$R^6$ and D together form a 5 or 6 membered heterocycle optionally substituted one or more times with any one or a combination selected from the group consisting of oxo, halo, $C_{1-3}$alkyl, $C_{1-3}$haloalkyl, hydroxy and $C_{1-3}$alkoxy.

Viewed from a second aspect, there is provided a composition comprising the compound of the first aspect and one or more pharmaceutically acceptable excipients.

Viewed from a third aspect, there is provided a compound of the first aspect or a composition of the second aspect for use as an ABAD inhibitor.

Viewed from a fourth aspect, there is provided a compound of the first aspect or a composition of the second aspect for use as a medicament.

Viewed from a fifth aspect, there is provided a compound of the first aspect or a composition of the second aspect for use in a method of treatment or prophylaxis of dementia or cancer.

## BRIEF DESCRIPTION OF THE FIGURES

Figure 1: Chemical modifications made to the basic cluster backbone.

Figure 2: Box plot summarising compound type versus ABAD/17β-HSD10 activity.

Figure 3: Key interactions between 17β-HSD10 and ESC1002033. A) H-bond from the imidazole to GLN162 and also a H-bond to a well-ordered water molecule with an edge to face interaction between the central aryl ring and TYR168 of the catalytic triad. B) A small hydrophobic pocket close to the imidazole and water.

Figure 4: Top: Indicates the previously published AG18051, its interaction with ABAD/17β-HSD10 (ABAD) and (bottom left) the key residues for interaction are defined as GLN162, GLN165 and TYR168. Bottom right is a superposition of ESC1002033 over bottom left.

Figure 5: A) Targeting the edge to face interaction at Tyrosine 168; B) targeting Leucine 205.

Figure 6: Confirmation of an inactive singleton enantiomer.

Figure 7: Crystal structures depicting the key interactions of ESC1002421 (not of the invention claimed herein) and 17β-HSD10.

Figure 8: A summary of lipophilicity vs potency improvements of the singleton series (not of the invention claimed herein).

Figure 9: Thermal shift data for ESC1002033 and ESC1002082 (not of the invention claimed herein) giving representative examples of the derivative plots and showing the correlation between $pIC_{50}$ and $\Delta T_m$.

Figure 10: 17β-HSD10 activity in HEK293 mtsABAD cell line using the fluorogenic probe -(-)CHANA (Values shown are mean n=6 $\pm$ SEM).

Figure 11: Example electron density map, with initial electron density mFo-DFc in green on the left (ligand superimposed for reference) and the 2mFo-Dfc density for the fitted ligand and cofactor on the right.

Figure 12: ESC1002033 bound in a deep cleft with some notable features in its conformation. Binding site shown in (A) and surface (B).

Figure 13: A) ESC1002332 (not of the invention claimed herein) bound in same site as ESC1002033. B) Differences in ESC1002332 and ESC1002033 binding.

## DETAILED DESCRIPTION OF THE INVENTION

The inventors have found that the compounds disclosed herein are surprisingly effective ABAD inhibitors, with some of the inhibitors exhibiting $IC_{50}$ values of less than 100 nM. The compounds disclosed herein interact non-competitively with NADH, offering potentially greater enzyme specificity than some known ABAD inhibitors. The compounds are now disclosed in detail.

In the discussion that follows, reference is made to a number of terms, which have the meanings provided below, unless a context indicates to the contrary. The nomenclature used herein for defining compounds, in particular the compounds according to the invention, is in general based on the rules of the IUPAC organisation for chemical compounds, specifically the "IUPAC Compendium of Chemical Terminology (Gold Book)". For the avoidance of doubt, if a rule of the IUPAC organisation is contrary to a definition provided herein, the definition herein is to prevail. Furthermore, if a compound structure is contrary to the name provided for the structure, the structure is to prevail.

The term "aromatic" defines a cyclically conjugated molecular entity with a stability (due to delocalisation) significantly greater than that of a hypothetical localised structure. The Hückel rule is often used in the art to assess aromatic character; monocyclic planar (or almost planar) systems of trigonally (or sometimes digonally) hybridised atoms that contain (4n+2) $\pi$-electrons (where n is a non-negative integer) will exhibit aromatic character. The rule is generally limited to n = 0 to 5.

The term "conjugated" or variants thereof defines a molecular entity whose structure may be represented as a system of alternating single and multiple bonds. In such systems, conjugation is the interaction of one p-orbital with another across an intervening $\pi$-bond in such structures. In appropriate molecular entities d-orbitals may be involved. The term is also extended to the analogous interaction involving a p-orbital containing an unshared electron pair.

The term "delocalised" defines the $\pi$-bonding in a conjugated system where the bonding is not localised between two atoms, but instead each link has a fractional double bond character, or bond order.

The term "heteroaromatic" defines a cyclically conjugated molecular entity comprising heteroatoms, with a stability (due to delocalisation) significantly greater than that of a hypothetical localised structure.

The term "cyclic" or variants thereof defines a compound in which one or more series of atoms in the compound is connected to form a ring. Whereas, the term "acyclic" defines a compound containing no rings of atoms.

The term "aryl" defines a group derived from an arene by removal of a hydrogen atom from a ring carbon atom, wherein an arene is a monocyclic or polycyclic aromatic hydrocarbon.

The term "heteroaryl" defines a group derived from a heteroarene by removal of a hydrogen atom from a ring carbon or heteroatom, wherein a heteroarene is a monocyclic or polycyclic aromatic hydrocarbon comprising one or more heteroatoms.

The term "biaryl" defines a group derived from a biarene by removal of a hydrogen atom from a ring carbon atom, wherein a biarene is an assembly of two aryl groups, joined by a single bond.

The term "biheteroaryl" defines a group derived from a biheteroarene by removal of a hydrogen atom from a ring carbon or heteroatom, wherein a biheteroarene is an assembly of two heteroaryl groups, joined by a single bond.

The term "cycloalkyl" defines all univalent groups derived from cycloalkanes by removal of a hydrogen atom from a ring carbon atom. The term "cycloalkane" defines saturated monocyclic and polycyclic hydrocarbons. $C_3$-$C_5$cycloalkyl consists of cyclopropyl, cyclobutyl and cyclopentyl; and $C_5$-$C_8$cycloalkyl consists of cyclopentyl, cyclohexyl, cycloheptyl and cyclooctyl.

The term "heterocycloalkyl" defines all univalent groups derived from heterocycloalkanes by removal of a hydrogen atom from a ring carbon atom or heteroatom. The term "heterocycloalkane" defines monocyclic and polycyclic hydrocarbons comprising heteroatoms. $C_3$-$C_7$heterocycloalkyl may correspond to cyclopentyl, cyclohexyl, cycloheptyl and cyclooctyl, wherein at least one carbon atom is replaced with a heteroatom. In order to satisfy covalency, the heteroatom may be bound to one or more hydrogen atoms.

The term "heterocycle" defines all cyclic compounds comprising atoms of at least two different elements in the ring. For the avoidance of doubt, heterocycloalkanes and heteroarenes are included within the term "heterocycle".

The term "comprising" or variants thereof will be understood to imply the inclusion of a stated element, integer or step, or group of elements, integers or steps, but not the exclusion of any other element, integer or step, or group of elements, integers or steps.

The term "consisting" or variants thereof will be understood to imply the inclusion of a stated element, integer or step, or group of elements, integers or steps, and the exclusion of any other element, integer or step or group of elements, integers or steps.

The term "alkyl" is well known in the art and defines univalent groups derived from alkanes by removal of a hydrogen atom from any carbon atom, wherein the term "alkane" is intended to define cyclic or acyclic branched or unbranched hydrocarbons having the general formula $C_nH_{2n+2}$, wherein n is an integer $\geq 1$. $C_1$-$C_4$alkyl consists of methyl, ethyl, n-propyl, iso-propyl, n-butyl, sec-butyl, iso-butyl and tert-butyl.

The term "halo" is well known in the art and defines a halogen radical that, when bonded to a carbon radical makes a fluoride, chloride, bromide or iodide compound.

The term "haloalkyl" defines an alkyl in which one or more hydrogen atoms have been replaced with a halo.

The term "alkyloxy" is synonymous with "alkoxy" and when used herein defines a univalent group comprising an alkyl singly bonded to an oxygen atom, derived from the corresponding alcohol by removal of the hydrogen atom bonded to the oxygen atom.

The term "haloalkoxy" defines an alkoxy in which one or more hydrogen atoms have been replaced with a halo.

The term "alkylether" defines a univalent group derived from an ether group (an oxygen atom connected to two alkyl groups), by removal of a hydrogen atom from any carbon atom.

The term "alkanol" defines a univalent group comprising an alkylene singly bonded to a hydroxy group, derived from the corresponding alcohol by removal of a hydrogen atom bonded to a carbon atom.

The term "treatment" defines the therapeutic treatment of a subject that may be a human or non-human animal, in order to impede or reduce or halt the rate of progress of a condition, or to ameliorate or cure the condition. Prophylaxis of the

condition as a result of treatment is also included. References to prophylaxis are intended herein not to require complete prevention of a condition: its development may instead be hindered through treatment in accordance with the invention.

By an "effective amount" herein defines an amount of any one or a combination of the compounds described herein that is sufficient to impede a condition and thus produces the desired therapeutic or inhibitory effect.

The term "stereoisomer" is used herein to refer to isomers that possess identical molecular formulae and sequence of bonded atoms, but which differ in the arrangement of their atoms in space.

The term "enantiomer" defines one of a pair of molecular entities that are mirror images of each other and non-superimposable, i.e. cannot be brought into coincidence by translation and rigid rotation transformations. Enantiomers are chiral molecules, i.e. are distinguishable from their mirror image.

The term "racemic" is used herein to pertain to a racemate. A racemate defines a substantially equimolar mixture of a pair of enantiomers.

The term "diastereoisomers" (also known as diastereomers) defines stereoisomers that are not related as mirror images.

The term "solvate" is used herein to refer to a complex comprising a solute, such as a compound or salt of the compound, and a solvent. If the solvent is water, the solvate may be termed a hydrate, for example a mono-hydrate, di-hydrate, tri-hydrate etc, depending on the number of water molecules present per molecule of substrate.

The term "isotope" is used herein to define a variant of a particular chemical element, in which the nucleus necessarily has the same atomic number but has a different mass number owing to it possessing a different number of neutrons.

The term "prodrug" is used herein to refer to a compound which acts as a drug precursor and which, upon administration to a subject, undergoes conversion by metabolic or other chemical processes to yield a compound disclosed herein.

The term "pharmaceutically acceptable excipient" defines substances other than a pharmacologically active drug or prodrug, which are included in a pharmaceutical product.

The term "enteral" is used to refer to administration of a compound through the gastrointestinal tract. Enteral administration may be oral administration, i.e. administration through the mouth.

The term "parenteral" is used to refer to administration of a compound into the body *via* means other than the gastrointestinal tract. Parenteral administration includes intravenous administration (directly into a vein), intramuscular administration (into the muscle), intradermal administration (beneath the skin) or subcutaneous administration (into the fat or skin). Parenteral administration may be carried out *via* a bolus injection, in which a discrete amount of compound is administered in one injection.

As described above, in a first aspect, there is provided a compound of formula (I):

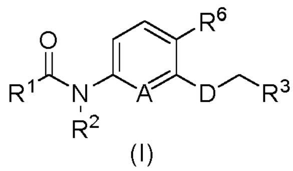

(I) ;

wherein $R^1$ is any one selected from the group consisting of $C_6$-$C_{10}$aryl, $C_3$-$C_5$heteroaryl, $C_{12}$biaryl, $C_6$-$C_{10}$biheteroaryl, $C_5$-$C_8$cycloalkyl, $C_3$-$C_7$heterocycloalkyl, $C_1$-$C_6$alkyl, $C_6$-$C_{10}$aryl$C_1$-$C_3$alkyl, $C_{12}$biaryl$C_1$-$C_3$alkyl and $C_5$-$C_8$cycloalkyl$C_1$-$C_3$alkyl optionally substituted one or more times with any one or a combination selected from the group consisting of halo, $C_1$-$C_4$haloalkyl, $C_1$-$C_4$alkyl, $C_1$-$C_4$alkoxy, hydroxy and methylsulfonyl;

$R^2$ is any one selected from the group consisting of iso-butyl, iso-propyl, cyclobutyl, cyclopropyl, cyclobutylmethyl, cyclopropylmethyl, methoxyethyl, isopropoxyethyl, and di($C_{1-4}$alkyl)amino$C_{1-3}$alkyl;

A is CH or N;

$R^3$ is any one selected from the group consisting of $C_2$-$C_5$heteroaryl optionally substituted one or more times with any one or a combination selected from the group consisting of $C_1$-$C_4$alkyl, $C_3$-$C_5$cycloalkyl, halo, hydroxy and $C_{1-3}$alkoxy; ethyne, hydroxymethyl, amido, N-methylamido and cyano;

D is $NR^7$ or O, wherein $R^7$ is $C_{1-3}$alkyl or $C_{1-3}$alkylol;

$R^6$ is H; or

$R^6$ and D together form a 5 or 6 membered heterocycle optionally substituted one or more times with any one or a combination selected from the group consisting of oxo, halo, $C_{1-3}$alkyl, $C_{1-3}$haloalkyl, hydroxy and $C_{1-3}$alkoxy.

Compounds of formula (I) are also referred to herein as "cluster compounds". $R^1$ of such cluster compounds is any one selected from the group consisting of $C_6$-$C_{10}$aryl, $C_3$-$C_5$heteroaryl, $C_{12}$biaryl, $C_6$-$C_{10}$biheteroaryl, $C_5$-$C_8$cycloalkyl, $C_3$-$C_7$heterocycloalkyl, $C_1$-$C_6$alkyl (such as $C_{3-6}$alkyl), $C_6$-$C_{10}$aryl$C_1$-$C_3$alkyl, $C_{12}$biaryl$C_1$-$C_3$alkyl and $C_5$-$C_8$cycloalk-

yl$C_1$-$C_3$alkyl, each optionally substituted one or more times with any one or a combination selected from the group consisting of halo, $C_1$-$C_4$haloalkyl, $C_1$-$C_4$alkyl, $C_1$-$C_4$alkoxy, hydroxy and methylsulfonyl.

$C_6$-$C_{10}$aryl includes phenyl, naphthalenyl, cyclooctatetraenyl and cyclodecapentaenyl. In some embodiments, $C_6$-$C_{10}$aryl is phenyl or naphthalenyl, such as phenyl.

$C_3$-$C_5$heteroaryl includes thiophenyl, pyridyl, pyrimidinyl, pyrazinyl, imidazolyl, pyrazolyl, thiazolyl and triazolyl. In some embodiments, the $C_3$-$C_5$heteroaryl of $R^1$ is thiophenyl, pyridyl, pyrimidinyl or pyrazinyl. For example, the $C_3$-$C_5$heteroaryl of $R^1$ may be thienyl or pyridyl.

$C_{12}$biaryl may be biphenyl.

$C_6$-$C_{10}$biheteroaryl includes bithienyl, bipyridyl, thienylpyridyl, thienylpyrimidinyl, pyridylpyrimidinyl, thienylpyrazinyl, pyridylpyrazinyl, bipyrimidinyl, bipyrazinyl and pyrimidinylpyrazinyl. In some embodiments, $C_6$-$C_{10}$biheteroaryl is bithienyl or bipyridyl.

$C_5$-$C_8$cycloalkyl includes cyclopentyl, cyclohexyl, cycloheptyl and cyclooctyl. In some embodiments, $C_5$-$C_8$cycloalkyl is cyclopentyl or cyclohexyl, such as cyclohexyl.

$C_3$-$C_7$heterocycloalkyl includes morpholino, tetrahydropyranyl, piperidinyl, tetrahydrofuranyl, pyrrolidinyl, azepinyl and diazepinyl. In some embodiments, $C_3$-$C_7$heterocycloalkyl is morpholino, tetrahydropyranyl or piperidinyl.

$C_1$-$C_6$alkyl includes methyl, ethyl, *n*-propyl, *iso*-propyl, *n*-butyl, *sec*-butyl, *tert*-butyl, *n*-pentyl, 2-methylbutan-2-yl, 2,2-dimethylpropyl, 3-methylbutyl, pentan-2-yl, pentan-3-yl, 3-methylbutan-2-yl, 2-methylbutyl, n-hexane, hexan-2-yl, hexan-3-yl, 4-methylpentanyl, 4-methylpentan-2-yl, 2-methylpentan-2-yl and 2-methylpentanyl. In some embodiments, $C_{1-6}$alkyl is $C_{3-6}$alkyl. In some embodiments, $C_3$-$C_6$alkyl is *n*-propyl or *iso*-propyl, such as *iso*-propyl.

$C_6$-$C_{10}$aryl$C_1$-$C_3$alkyl includes phenyl$C_1$-$C_3$alkyl, naphthalenyl$C_1$-$C_3$alkyl, cyclooctatetraenyl$C_1$-$C_3$alkyl and cyclodecapentaenyl$C_1$-$C_3$alkyl. In some embodiments, $C_6$-$C_{10}$aryl$C_1$-$C_3$alkyl is selected from the group consisting of phenylmethyl, phenylethyl, phenylpropyl, naphthalenylmethyl, naphthalenylethyl and naphthalenylpropyl. For example, $C_6$-$C_{10}$aryl$C_1$-$C_3$alkyl may be phenylmethyl, phenylethyl or phenylpropyl.

$C_{12}$biaryl$C_1$-$C_3$alkyl includes biphenyl$C_1$-$C_3$alkyl. In some embodiments, $C_{12}$biaryl$C_1$-$C_3$alkyl is biphenylmethyl, biphenylethyl or biphenylpropyl.

$C_5$-$C_8$cycloalkyl$C_1$-$C_3$alkyl includes cyclopentyl$C_1$-$C_3$alkyl, cyclohexyl$C_1$-$C_3$alkyl, cycloheptyl$C_1$-$C_3$alkyl and cyclooctyl$C_1$-$C_3$alkyl. In some embodiments, $C_5$-$C_8$cycloalkyl$C_1$-$C_3$alkyl is selected from the group consisting of cyclopentylmethyl, cyclopentylethyl, cyclopentylpropyl, cyclohexylmethyl, cyclohexylethyl and cyclohexylpropyl.

As described above, the $C_6$-$C_{10}$aryl, $C_3$-$C_5$heteroaryl, $C_{12}$biaryl, $C_6$-$C_{10}$biheteroaryl, $C_5$-$C_8$cycloalkyl, $C_3$-$C_7$heterocycloalkyl, $C_3$-$C_6$alkyl, $C_6$-$C_{10}$aryl$C_1$-$C_3$alkyl, $C_{12}$biaryl$C_1$-$C_3$alkyl and $C_5$-$C_8$cycloalkyl$C_1$-$C_3$alkyl may each be substituted one or more times with any one or a combination selected from the group consisting of halo, $C_1$-$C_4$haloalkyl, $C_1$-$C_4$alkyl, $C_1$-$C_4$alkoxy, hydroxy (-OH) and methylsulfonyl ($-SO_2CH_3$).

In some embodiments, the halo substituent is fluoro or chloro, e.g. fluoro.

The $C_1$-$C_4$haloalkyl is any one or a combination selected from the group consisting of halomethyl, haloethyl, halo-*n*-propyl, halo-*iso*-propyl, halo-*n*-butyl, halo*sec*-butyl, halo-*iso*-butyl and halo-*tert*-butyl. In some embodiments, the halo of the $C_1$-$C_4$haloalkyl is fluoro. In some embodiments, the $C_1$-$C_4$haloalkyl is any one or a combination selected from the group consisting of trifluoromethyl, difluoromethyl and monofluoromethyl, such as trifluoromethyl.

In some embodiments, the $C_1$-$C_4$alkyl substituent is any one or a combination selected from methyl and ethyl.

The $C_1$-$C_4$alkoxy substituent is any one or a combination selected from the group consisting of methoxy, ethoxy, *n*-propoxy, *iso*-propoxy, *n*-butoxy, *sec*-butoxy, *iso*-butoxy and *tert*-butoxy. In some embodiments, the $C_1$-$C_4$alkoxy is any one or a combination selected from the group consisting of methoxy and ethoxy, such as methoxy.

In some embodiments, $R^1$ is any one selected from the group consisting of $C_6$-$C_{10}$aryl, $C_3$-$C_5$heteroaryl, $C_{12}$biaryl, $C_5$-$C_8$cycloalkyl, $C_3$-$C_6$alkyl and $C_6$-$C_{10}$aryl$C_1$-$C_3$alkyl, each optionally substituted one or more times with any one or a combination selected from the group consisting of halo, $C_1$-$C_4$haloalkyl, $C_1$-$C_4$alkyl, $C_1$-$C_4$alkoxy, hydroxy and methylsulfonyl. In such embodiments, $R^1$ is not an optionally substituted $C_6$-$C_{10}$biheteroaryl, $C_3$-$C_7$heterocycloalky, $C_{12}$biaryl$C_1$-$C_3$alkyl or $C_5$-$C_8$cycloalkyl$C_1$-$C_3$alkyl.

In some embodiments, $R^1$ is any one selected from the group consisting of $C_6$-$C_{10}$aryl, $C_3$-$C_5$heteroaryl, $C_5$-$C_8$cycloalkyl and $C_3$-$C_6$alkyl, each optionally substituted one or more times with any one or a combination selected from the group consisting of halo, $C_1$-$C_4$haloalkyl, $C_1$-$C_4$alkyl, $C_1$-$C_4$alkoxy and hydroxy and methylsulfonyl. In such embodiments, $R^1$ is not an optionally substituted $C_{12}$biaryl, $C_6$-$C_{10}$biheteroaryl, $C_3$-$C_7$heterocycloalky, $C_6$-$C_{10}$aryl$C_1$-$C_3$alkyl, $C_{12}$biaryl$C_1$-$C_3$alkyl or $C_5$-$C_8$cycloalkyl$C_1$-$C_3$alkyl.

In some embodiments, $R^1$ is any one selected from the group consisting of phenyl, thiophenyl, pyridinyl, cyclohexyl and *iso*-propyl, each optionally substituted one or more times with any one or a combination selected from the group consisting of halo, $C_1$-$C_4$haloalkyl, $C_1$-$C_4$alkyl, $C_1$-$C_4$alkoxy, hydroxy and methylsulfonyl.

In some embodiments, the optional substituents of $R^1$ are selected from the group consisting of halo, $C_1$-$C_4$haloalkyl, $C_1$-$C_4$alkyl, $C_1$-$C_4$alkoxy and hydroxy (i.e. the optional substituents are not methylsulfonyl). In some embodiments, the optional substituents are selected from the group consisting of fluoro, chloro, trifluoromethyl, methyl, ethyl, methoxy and hydroxy. For example, the optional substituents may be selected from the group consisting of fluoro, chloro,

trifluoromethyl, methyl, ethyl and methoxy.

In particular embodiments, $R^1$ is any one selected from the group consisting of phenyl, thiophenyl, pyridinyl, cyclohexyl and *iso*-propyl, each optionally substituted one or more times with any one or a combination selected from the group consisting of fluoro, chloro, trifluoromethyl, methyl, ethyl and methoxy.

As described above, $R^2$ is any one selected from the group consisting of *iso*-butyl, iso-propyl, cyclobutyl, cyclopropyl, cyclobutylmethyl, cyclopropylmethyl, methoxyethyl, isopropoxyethyl and di($C_{1-4}$alkyl)amino$C_{1-3}$alkyl.

In some embodients, the di($C_{1-4}$alkyl)amino$C_{1-3}$alkyl is dimethylamino$C_{1-3}$alkyl, such as dimethylaminoethyl.

In some embodiments, $R^2$ is any one selected from the group consisting of *iso*-butyl, *iso*-propyl, cyclobutyl, cyclopropyl, cyclobutylmethyl, cyclopropylmethyl, methoxyethyl, isopropoxyethyl.

In some embodiments, $R^2$ is any one selected from the group consisting of *iso*-butyl, *iso*-propyl, cyclobutylmethyl and cyclopropylmethyl. In some embodiments, $R^2$ is iso-butyl.

As described above, A is CH or N. When A is CH, the central ring of formula (I) may be a benzene ring, and when A is a nitrogen atom, the central ring of formula (I) may be a pyridine ring. In some embodiments, A is CH.

As described above, $R^3$ is any one selected from the group consisting of $C_2$-$C_5$heteroaryl optionally substituted one or more times with any one or a combination selected from the group consisting of $C_1$-$C_4$alkyl, $C_3$-$C_5$cycloalkyl, halo, hydroxy and $C_{1-3}$alkoxy; ethyne, hydroxymethyl, amido, N-methylamido and cyano.

In some embodiments, the $C_2$-$C_5$heteroaryl of $R^3$ comprises nitrogen and/or sulfur heteroatoms. As described above, $C_2$-$C_5$heteroaryl includes thiophenyl, pyridyl, pyrimidinyl, pyrazinyl, imidazolyl, pyrazolyl, thiazolyl and triazolyl. In some embodiments, the $C_2$-$C_5$heteroaryl of $R^3$ is any one selected from the group consisting of imidazolyl, pyrazolyl, thiazolyl, pyridyl and triazolyl. For example, the $C_2$-$C_5$heteroaryl of $R^3$ may be any one selected from the group consisting of imidazol-5-yl, imidazol-4-yl, imidazol-2-yl, pyrazol-4-yl, 1,3-thiazol-5-yl, pyrid-2-yl, pyrid-3-yl, 1,2,3-triazol-4-yl and 1,2,4-triazol-3-yl.

In some embodiments, the $C_2$-$C_5$heteroaryl of $R^3$ comprises nitrogen as the only heteroatom. For example, the $C_2$-$C_5$heteroaryl of $R^3$ may be any one selected from the group consisting of imidazolyl, pyrazolyl, pyridyl and triazolyl. In some embodiments, the $C_2$-$C_5$heteroaryl of $R^3$ is selected from the group consisting of imidazol-5-yl, imidazol-4-yl, imidazol-2-yl, pyrazol-4-yl, pyrid-2-yl, pyrid-3-yl, 1,2,3-triazol-4-yl and 1,2,4-triazol-3-yl.

In some embodiments, the $C_2$-$C_5$heteroaryl of $R^3$ is a $C_3$heteroaryl. For example, the $C_2$-$C_5$heteroaryl may include imidazolyl, pyrazolyl and thiazolyl, such as imidazol-5-yl, imidazol-4-yl, imidazol-2-yl, pyrazol-4-yl and 1,3-thiazol-5-yl. In some embodiments, the $C_2$-$C_5$heteroaryl of $R^3$ is any one selected from the group consisting of imidazolyl and pyrazolyl, such as imidazol-5-yl, imidazol-4-yl, imidazol-2-yl and pyrazol-4-yl.

The optional substituents of the $C_2$-$C_5$heteroaryl of $R^3$ may be any one or a combination selected from the group consisting of $C_1$-$C_4$alkyl, $C_3$-$C_5$cycloalkyl, halo, hydroxy and $C_{1-3}$alkoxy. In some embodiments, the $C_1$-$C_4$alkyl is any one or a combination selected from methyl and ethyl, such as methyl. As described above, $C_3$-$C_5$cycloalkyl includes cyclopropyl, cyclobutyl and cyclopentyl. In some embodiments, the $C_3$-$C_5$cycloalkyl is any one or a combination of cyclobutyl or cyclopropyl.

In some embodiments, the optional substituents of the $C_2$-$C_5$heteroaryl of $R^3$ are any one or a combination selected from the group consisting of $C_1$-$C_4$alkyl and $C_3$-$C_5$cycloalkyl.

In some embodiments, the optional substituents of the $C_2$-$C_5$heteroaryl of $R^3$ are one or more methyl.

In some embodiments, $R^3$ is an optionally substituted $C_2$-$C_5$heteroaryl.

In some embodiments, $R^3$ is a $C_3$heteroaryl, optionally substituted one or more times with methyl.

In some embodiments, $R^3$ is any one selected from the group consisting of imidazolyl, 1-methylimidazolyl, 2-methylimidazolyl, 4-methylimidazolyl and pyrazolyl. For example, $R^3$ may be any one selected from the group consisting of imidazol-5-yl, 1-methylimidazol-2-yl, 1-methylimidazol-4-yl, 2-methylimidazol-5-yl, 4-methylimidazol-5-yl and pyrazol-5-yl.

As described above, D is $NR^7$ or O, wherein $R^7$ is $C_{1-3}$alkyl. In some embodiments, D is $NR^7$, such as $NCH_3$.

As described above, $R^6$ may be H or, alternatively, $R^6$ and D may together form a 5 or 6 membered heterocycle optionally substituted one or more times with any one or a combination selected from the group consisting of oxo, halo, $C_{1-3}$alkyl, $C_{1-3}$haloalkyl, hydroxy and $C_{1-3}$alkoxy. In some embodiments, $R^6$ and D together form a 5 membered heterocycloalkene or a 6 membered heterocyclodialkene, each optionally substituted one or more times with any one or a combination selected from the group consisting of oxo, halo, $C_{1-3}$alkyl, $C_{1-3}$haloalkyl, hydroxy and $C_{1-3}$alkoxy. In such embodiments, the compound may be of formula (III) or (IV):

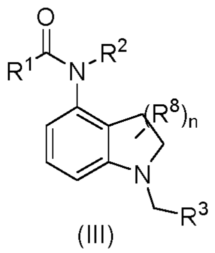
(III)

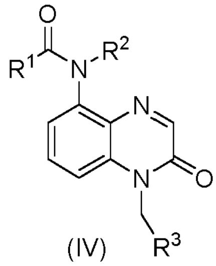
(IV)

,

wherein $R^1$, $R^2$ and $R^3$ are as described above and herein;
each $R^8$ is independently selected from the group consisting of oxo, halo, $C_{1-3}$alkyl, $C_{1-3}$haloalkyl, hydroxy and $C_{1-3}$alkoxy; and
n is 0 to 4.

In some embodiments, n is 0.

In some embodiments, $R^6$ is H.

In particular embodiments, there is provided a compound of formula (V) wherein:

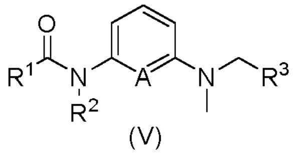
(V)

;

wherein $R^1$ is any one selected from the group consisting of $C_6$-$C_{10}$aryl, $C_3$-$C_5$heteroaryl, $C_{10}$-$C_{16}$biaryl, $C_6$-$C_{14}$biheteroaryl, $C_5$-$C_8$cycloalkyl, $C_3$-$C_7$heterocycloalkyl, $C_3$-$C_6$alkyl, $C_6$-$C_{10}$aryl$C_1$-$C_3$alkyl, $C_{10}$-$C_{16}$biaryl$C_1$-$C_3$alkyl and $C_3$-$C_8$cycloalkyl$C_1$-$C_3$alkyl optionally substituted one or more times with any one or a combination selected from the group consisting of halo, $C_1$-$C_4$haloalkyl, $C_1$-$C_4$alkyl, $C_1$-$C_4$alkoxy, hydroxy and methylsulfonyl;
$R^2$ is any one selected from the group consisting of *iso*-butyl, *iso*-propyl, cyclobutyl, cyclopropyl, cyclobutylmethyl, cyclopropylmethyl, and methoxyethyl, isopropoxyethyl;
A is a carbon or nitrogen atom;
$R^3$ is any one selected from the group consisting of $C_3$-$C_5$heteroaryl optionally substituted one or more times with any one or a combination selected from the group consisting of $C_1$-$C_4$alkyl $C_3$-$C_5$cycloalkyl, ethyne, hydroxymethyl, halo and N-methylacetamido;
$R^4$ is any one selected from the group consisting of $C_6$aryl, $C_3$-$C_7$heteroaryl and $C_6$-$C_{14}$biheteroaryl optionally substituted one or more times with any one or a combination selected from the group consisting of hydroxy, $C_1$-$C_4$alkoxy, $C_1$-$C_4$haloalkoxy, halo, $C_1$-$C_4$haloalkyl, $C_1$-$C_4$alkyl, $C_3$-$C_5$cycloalkyl, $C_4$-$C_5$heterocycloalkyl, $C_1$-$C_4$alkanol, cyano and methane sulfonato; and
$R^5$ is $C_3$-$C_5$heteroaryl, optionally substituted one or more times with any one or a combination selected from the group consisting of halo and $C_1$-$C_4$alkyl.

In particular embodiments, there is provided a compound of formula (V) wherein:

$R^1$ is any one selected from the group consisting of phenyl, thiophenyl, pyridinyl, cyclohexyl and *iso*-propyl, optionally substituted one or more times with any one or a combination selected from the group consisting of fluoro, chloro, trifluoromethyl, methyl, ethyl and methoxy;
$R^2$ is any one selected from the group consisting of *iso*-butyl, *iso*-propyl, cyclobutylmethyl and cyclopropylmethyl;
A is a carbon atom; and
$R^3$ is any one selected from the group consisting of imidazol-5-yl, 1-methylimidazol-2-yl, 1-methylimidazol-4-yl, 2-methylimidazol-5-yl, 4-methylimidazol-5-yl and pyrazol-5-yl.

In some embodiments, the compound of formula (I) is selected from the group comsisting of ESC1002456, ESC1002033, ESC1002575, ESC1002204 and ESC1002597. In some embodiments, the compound of formula (I) is represented by any one of structures (d, g, h, I and j).

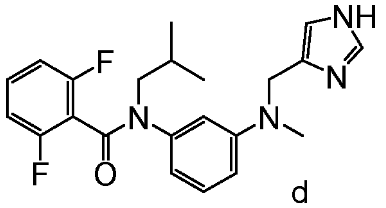

d

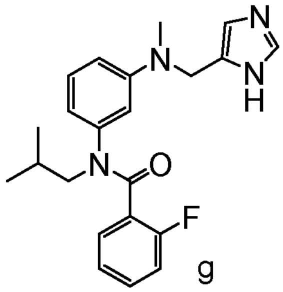

g

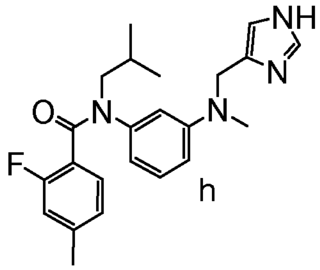

h

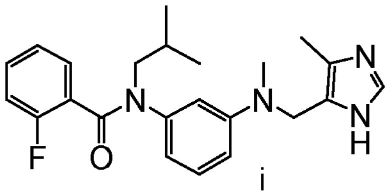

i

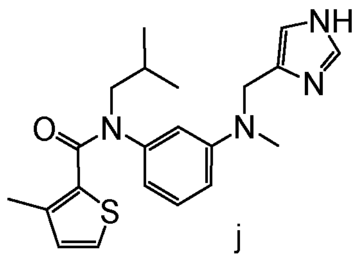

j

In some cases, the compounds described herein may be isolated or prepared in the form of a pharmaceutically acceptable salt. The term "pharmaceutically acceptable salt" is intended to define salts that may be administered to a patient or used in pharmacy. The pharmaceutically acceptable salt may be prepared by reacting the compound of the invention with a suitable acid, such as hydrogen chloride, hydrogen bromide, hydrogen iodide, sulfuric acid, phosphoric acid, acetic acid, trifluoroacetic acid, propionic acid, glycolic acid, maleic acid, malonic acid, methanesulfonic acid, fumaric acid, succinic acid, tartaric acid, citric acid, benzoic acid and ascorbic acid.

The compounds of the invention may exist in different stereoisomeric forms. All stereoisomeric forms and mixtures thereof, including enantiomers and racemic mixtures, are included within the scope of the invention. Individual stereoisomers of compounds of the invention, i.e. compounds comprising less than 5% 2% or 1% (e.g. less than 1%) of the other stereoisomer, are included. Mixtures of stereoisomers in any proportion, for example a racemic mixture comprising substantially equal amounts of two enantiomers are also included within the invention.

R- and S-enantiomers are distinguishable by the direction of priority of the substituents attached to the chiral centre. Priority is based on the atomic number (proton number) of the first atom of the substituent. For example, if the carbon atom at position 2 of the pyrrolidine centre of a compound of formula (II) is bound to a substituted phenyl, the nitrogen atom of the pyrrolidine, a proton and the carbon atom at position 3 of the pyrrolidine, the priority of the substituents (from lowest to highest) is in the order of proton < carbon at position 3 of pyrrolidine < substituted phenyl < nitrogen atom of pyrrolidine. In the case of carbon at position 3 of pyrrolidine and substituted phenyl, the first atom of the substituent is carbon. In order to distinguish priority of these two substituents, the second atoms of the substituents are taken into account. For the carbon at position 3 of pyrrolidine, two of the second atoms are hydrogen and one is carbon, whereas for substituted phenyl the second atoms are all carbon. Since carbon has a higher atomic number than hydrogen, it takes priority. Hence, substituted phenyl has a greater priority than the carbon at position 3 of pyrrolidine. To distinguish whether the chiral centre at position 2 of pyrrolidine is R or S, the chiral centre is oriented so that the lowest-priority of the four substituents (e.g. proton) is pointed away from the plane of view. If the priority of the remaining three substituents decreases in a clockwise direction, the enantiomer is R, and if the priority decreases in a counterclockwise direction, the enantiomer is S.

Also included are solvates and isotopically-labelled compounds of formula (I) or (II). Isotopically-labelled compounds are identical to those described herein, with the exception that one or more atoms are replaced by an atom having an atomic mass or mass number different from the atomic mass or mass number predominantly found in nature.

Examples of isotopes that can be incorporated into compounds of the invention include isotopes of hydrogen, carbon, nitrogen, oxygen, sulfur, fluorine and chlorine, such as $^2H$, $^3H$, $^{13}C$, $^{14}C$, $^{15}N$, $^{18}O$, $^{17}O$, $^{35}S$, $^{18}F$, and $^{36}Cl$, respectively.

Also provided herein is a composition comprising a compound of the first aspect and one or more pharmaceutically acceptable excipients. For the avoidance of doubt, the embodiments described herein in relation to the first aspect of the invention apply *mutatis mutandis* to the composition of the second aspect. For example, the composition of the second aspect may comprise a compound of formula (V), wherein:

$R^1$ is any one selected from the group consisting of phenyl, thiophenyl, pyridinyl, cyclohexyl and *iso*-propyl, optionally substituted one or more times with any one or a combination selected from the group consisting of fluoro, chloro, trifluoromethyl, methyl, ethyl and methoxy;
$R^2$ is any one selected from the group consisting of *iso*-butyl, *iso*-propyl, cyclobutylmethyl and cyclopropylmethyl;
A is a carbon atom; and
$R^3$ is any one selected from the group consisting of imidazol-5-yl, 1-methylimidazol-2-yl, 1-methylimidazol-4-yl, 2-methylimidazol-5-yl, 4-methylimidazol-5-yl and pyrazol-5-yl.

The composition of the second aspect comprises one or more pharmaceutically acceptable excipients. An extensive overview of pharmaceutically acceptable excipients is described in the Handbook of Pharmaceutical Excipients, 6th Edition; Editors R. C. Rowe, P. J. Sheskey and M. E. Quinn, The Pharmaceutical Press, London, American Pharmacists Association, Washington, 2009. Any suitable pharmaceutically acceptable excipient described within this document is within the scope of the invention.

The pharmaceutically acceptable excipient may be included within the composition for the purpose of long-term stabilization of the compound, bulking up solid formulations (often referred to as "bulking agents", "fillers", or "diluents"), or to enhance activity of the compound, such as by facilitating its absorption within the body, reducing its viscosity, or enhancing its solubility. The excipient may also enhance in vitro stability of the compound, such as prevention of denaturation or aggregation. Alternatively, the excipient may be used for identification purposes, or to make the compound more appealing to the patient, for example by improving its taste, smell and/or appearance. Typically, the excipient makes up the bulk of the composition.

Excipients include diluents or fillers, binders, disintegrants, lubricants, colouring agents and preservatives. Diluents or fillers are inert ingredients that may affect the chemical and physical properties of the final composition. If the dosage of the compound of the invention is small then more diluents will be required to produce a composition suitable for practical use. If the dosage of the compound of the invention is high then fewer diluents will be required.

Binders add cohesiveness to powders in order to form granules, which may form a tablet. The binder must also allow the tablet to disintegrate upon ingestion so that the compound of the invention dissolves. Disintegration of the composition after administration may be facilitated through the use of a disintegrant.

In a third aspect, there is provided a compound of the first aspect or composition of the second aspect for use as an ABAD inhibitor. As described above, the inventors have found that the compounds disclosed herein are surprisingly effective ABAD inhibitors. The main function of ABAD, is to produce energy for the brain. In AD, a build-up of amyloid within mitochondria, allows amyloid to attach to ABAD, altering its function and ultimately impairing the memory capabilities of the brain. Direct modulation of ABAD activity is reported to be effective in treating AD (Lim et al., PLoS One, 2011, 6(12), e28887). In addition, ABAD has been implicated in various forms of cancer, such as prostate, breast and bone cancer and notably in cases of prostate cancer deemed castration resistant prostate cancer (CRPC).

Accordingly, viewed from a fourth aspect, there is provided a compound of the first aspect or a composition of the second aspect for use as a medicament and in a fifth aspect, there is provided a compound of the first aspect or a composition of the second aspect for use in a method of treatment or prophylaxis of dementia or cancer. In some embodiments, the compound or composition described herein is for use in a method of treatment or prophylaxis of dementia, prostate cancer, bone cancer or breast cancer. In some embodiments, the use is in a method of treatment or prophylaxis of AD, prostate cancer, bone cancer or breast cancer.

The compounds or compositions described herein may be suitable for enteral (including oral, such as buccal or sublingual, and nasal administration) or parenteral (including subcutaneous, intravenous and intramuscular) administration. In some embodiments, the compounds or compositions described herein are suitable for parenteral administration.

The compounds or compositions described herein may be compressed into solid dosage units, such as tablets, or be processed into capsules. The compounds or compositions may be prepared in the form of a solution, suspension or emulsion for injection. Alternatively, the compounds or compositions may be administered as a spray, including a nasal or buccal spray. In some embodiments, the compounds or compositions are prepared in the form of a solution, suspension or emulsion for injection.

Viewed from a sixth aspect, there is provided a method of inhibiting ABAD activity in a subject, the method comprising administering an effective amount of a compound of the first aspect or a composition of the second aspect.

Viewed from a seventh aspect, there is provided a method of treatment comprising administering an effective amount of a compound of the first aspect or a composition of the second aspect to a subject.

Viewed from an eighth aspect, there is provided a method for the treatment or prophylaxis of dementia or cancer, the method comprising administering an effective amount of a compound of the first aspect or a composition of the second aspect to a subject.

An effective amount of the compound or composition may be administered to a subject enterally or parenterally. The compound or composition may be administered parenterally, sometimes by direct injection, which is typically intramuscular, subcutaneous or intraveneous, or enterally via a tablet, capsule or buccal spray.

The subject may be any animal, such as a human, a zoo or farm animal or a domestic animal (a pet), i.e. the compound or composition may be for veterinary use. The subject is typically a human, and may be suffering from or liable to suffer from AD or cancer.

The skilled person is aware that an effective amount is likely to vary with the particular compound or composition of the invention, the subject and the administration procedure used. The skilled person is able to identify the effective amount of the compounds and compositions of the invention via routine work and experimentation.

In some embodiments, the method is for the treatment or prophylaxis of dementia, prostate cancer, bone cancer or breast cancer. In some embodiments, the method is for the treatment or prophylaxis of AD, prostate cancer, bone cancer or breast cancer.

For the avoidance of doubt, the embodiments described herein in relation to the first aspect and second aspects apply *mutatis mutandis* to the compounds and compositions of the fourth to eighth aspects.

It will be appreciated by those skilled in the art that numerous variations and/or modifications may be made to the invention as described herein without departing from the scope of the invention as described. The present embodiments are therefore to be considered for descriptive purposes and are not restrictive, and are not limited to the extent of that described in the embodiment. The person skilled in the art is to understand that the present embodiments may be read alone, or in combination, and may be combined with any one or a combination of the features described herein.

The invention may be further understood with reference to the following clauses:

1. A compound of formula (I):

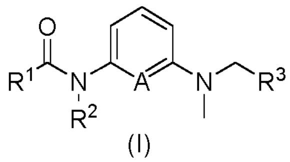

(I)

;

wherein $R^1$ is any one selected from the group consisting of $C_6$-$C_{10}$aryl, $C_3$-$C_5$heteroaryl, $C_{10}$-$C_{16}$biaryl, $C_6$-$C_{14}$biheteroaryl, $C_5$-$C_8$cycloalkyl, $C_3$-$C_7$heterocycloalkyl, $C_3$-$C_6$alkyl, $C_6$-$C_{10}$aryl$C_1$-$C_3$alkyl, $C_{10}$-$C_{16}$biaryl$C_1$-$C_3$alkyl and $C_3$-$C_8$cycloalkyl$C_1$-$C_3$alkyl optionally substituted one or more times with any one or a combination selected from the group consisting of halo, $C_1$-$C_4$haloalkyl, $C_1$-$C_4$alkyl, $C_1$-$C_4$alkoxy, hydroxy and methylsulfonyl;
$R^2$ is any one selected from the group consisting of *iso*-butyl, *iso*-propyl, cyclobutyl, cyclopropyl, cyclobutylmethyl, cyclopropylmethyl, methoxyethyl, isopropoxyethyl;
A is a carbon or nitrogen atom; and
$R^3$ is any one selected from the group consisting of $C_3$-$C_5$heteroaryl optionally substituted one or more times with any one or a combination selected from the group consisting of $C_1$-$C_4$alkyl $C_3$-$C_5$cycloalkyl, ethyne, hydroxymethyl, halo and N-methylacetamido;.

2. The compound of clause 1, wherein $R^1$ is any one selected from the group consisting of $C_6$-$C_{10}$aryl, $C_3$-$C_5$heteroaryl, $C_{10}$-$C_{16}$biaryl, $C_5$-$C_8$cycloalkyl, $C_3$-$C_6$alkyl and $C_6$-$C_{10}$aryl$C_1$-$C_3$alkyl, optionally substituted one or more times with any one or a combination selected from the group consisting of halo, $C_1$-$C_4$haloalkyl, $C_1$-$C_4$alkyl, $C_1$-$C_4$alkoxy, hydroxy and methylsulfonyl.

3. The compound of clause 1, wherein $R^1$ is any one selected from the group consisting of $C_6$-$C_{10}$aryl, $C_3$-$C_5$heteroaryl, $C_5$-$C_8$cycloalkyl and $C_3$-$C_6$alkyl, optionally substituted one or more times with any one or a combination selected from the group consisting of halo, $C_1$-$C_4$haloalkyl, $C_1$-$C_4$alkyl, $C_1$-$C_4$alkoxy and hydroxy.

4. The compound of clause 1, wherein $R^1$ is any one selected from the group consisting of phenyl, thiophenyl, pyridinyl, cyclohexyl and *iso*-propyl, optionally substituted one or more times with any one or a combination selected from the

group consisting of fluoro, chloro, trifluoromethyl, methyl, ethyl and methoxy.

5. The compound of any one of clauses 1 to 4, wherein $R^2$ is any one selected from the group consisting of *iso*-butyl, *iso*-propyl, cyclobutylmethyl and cyclopropylmethyl.

6. The compound of any one of clauses 1 to 5, wherein A is a carbon atom.

7. The compound of any one of clauses 1 to 6, wherein $R^3$ comprises nitrogen and/or sulfur as heteroatoms.

8. The compound of any one of clauses 1 to 6, wherein $R^3$ comprises nitrogen as the only heteroatom.

9. The compound of any one of clauses 1 to 8, wherein $R^3$ is a $C_3$heteroaryl, optionally substituted one or more times with methyl.

10. The compound of any one of clauses 1 to 6, wherein $R^3$ is any one selected from the group consisting of imidazolyl, 1-methylimidazolyl, 2-methylimidazolyl, 4-methylimidazolyl and pyrazolyl.

11. The compound of any one of clauses 1 to 6, wherein $R^3$ is any one selected from the group consisting of imidazol-5-yl, 1-methylimidazol-2-yl, 1-methylimidazol-4-yl, 2-methylimidazol-5-yl, 4-methylimidazol-5-yl and pyrazol-5-yl.

12. A composition comprising the compound of any one of clauses 1 to 11 and one or more pharmaceutically acceptable excipients.

13. A compound of any one of clauses 1 to 11 or a composition of clause 12 for use as an ABAD inhibitor.

14. A compound of any one of clauses 1 to 11 or a composition of clause 12 for use as a medicament.

15. A compound of any one of clauses 1 to 11 or a composition of clause 12 for use in a method of treatment or prophylaxis of cancer.

16. The compound or composition for use of clause 15, wherein the use is in a method of treatment of cancer.

17. The compound or composition for use of clause 15, wherein the use is in a method of prophylaxis of cancer.

18. A compound of any one of clauses 1 to 11 or a composition of clause 12 for use in a method of treatment or prophylaxis of dementia.

19. The compound or composition for use of clause 18, wherein the use is in a method of treatment of dementia.

20. The compound or composition for use of clause 18, wherein the use is in a method of prophylaxis of dementia.

The invention may be further understood with reference to the examples that follow. Any compounds not falling under the scope of Formula (I) are not part of the invention.

## EXAMPLES

### Materials and Methods

All aqueous solutions were prepared with deionized water (Millipore, UK) and all chemicals purchased from Sigma Aldrich, UK unless stated otherwise.

### Protein Purification for enzyme activity assays

See Aitken, L et al. 2016, ChemBioChem, vol. 17, no. 11, pp. 1029-1037
Cell pellets of *E. coli* BL21-CodonPlus cells containing Histev-17p3-HSD10 protein were re-suspended for 30 min, 4 °C, in lysis buffer (20 mM $NaH_2PO_4$, 30 mM imidazole, 500 mM NaCl, 10 % (v/v) glycerol, pH 7.5) with the addition of complete ethylenediaminetetraacetic acid (EDTA)-free protease inhibitor tablets (Roche), lysozyme (1 mg/mL), deoxyribonuclease (DNase) (20 μg/mL) and Triton X-100 (0.1 % (v/v)). Cells were lysed by passage through a cell disruptor at 30 kPSI

(Constant Systems Ltd) and the lysate was cleared by centrifugation (Sorvall Evolution RC, rotor S5-34 55-34 angle, 20500 rpm, 30 min, 4 °C). Cleared lysate was filtered (0.45 μm membrane; Whatman) then applied to a Ni- nitrilotriacetic acid (NTA) (GE Healthcare) column prewashed with lysis buffer and protein eluted with 300 mM imidazole buffer (20 mM $NaH_2PO_4$, 300 mM imidazole, 500 mM NaCl, 10 % (v/v) glycerol, pH 7.5). Tobacco etch virus (TEV) protease was added to the protein at a mass-to-mass ratio of 1:10, to cleave the histidine tag and the protein was then dialysed into 20 mM Tris-HCl, 30 mM imidazole, 500 mM NaCl, 10% (v/v) glycerol, pH 7.5 containing EDTA (1 mM) and dithiothreitol (DTT) (1 mM) to aid solubility. Protein digestion and dialysis was carried out at 4 °C for 16 h.

Complete digestion was firstly checked by sodium dodecyl sulfatepolyacrylamide gel electrophoresis (SDS-PAGE), then fully digested protein was passed over a second Ni-column and the flow-through, containing 17β-HSD10 protein, was concentrated using a Vivaspin column (10 kDa MWCO, GE Healthcare) to - 7 mL before final purification using gel filtration to remove imidazole (Hi-Load 16/60 Superdex 75 prep grade column, GE Healthcare, flow rate 1.5 mL/min). Protein was eluted in gel filtration buffer (10 mM trisaminomethane (Tris-HCl), 150 mM NaCl, 10% glycerol, pH 7.5) and concentrated (Vivaspin column (10 kDa molecular weight cut off (MWCO), GE Healthcare)) to 10 mg/mL. Aliquots (10 μL) were taken and flash frozen in liquid nitrogen before final storage at -80 °C.

**Compound Screening**

(See Aitken, L et al., 2017, SLAS Discovery, vol. 22, no. 6, pp. 676-685.)
The assay buffer used contains: 10 mM Tris.HCl (pH 7.4), 150 mM NaCl, 1 mM DTT, 0.005% Tween (polysorbate 20) and 0.01% bovine serum albumin (BSA). As indicated for some experiments the assay buffer was supplemented with 100 units/ml bovine liver catalase. All experiments were conducted in clear bottom 384-well or 1536-well low volume microplates (Corning) with a final assay volume of 20 μL and the reaction progress was monitored by oxidation of NADH as determined by reduction in absorbance at 340 nm read on the EnVision plate reader (PerkinElmer).

For primary screening an endpoint assay format was adopted in which an Echo acoustic liquid dispenser (Labcyte) was used to transfer 20 nL of either reference standard, dimethylsulfoxide (DMSO) control (0.5 % final assay concentration (FAC)) or test compounds (10 μM FAC) to the assay plate. 10 μL of 17β-HSD10 enzyme in assay buffer (2.5 nM, FAC) was then added to the plates using the Preddator liquid handling robot (Redd and Whyte) and plates were incubated at room temperature for 15 minutes. 10 μL of substrate mixture (100 μM acetoacetyl-CoA and 100 μM NADH, FAC) was added with the Preddator. To start the reaction and the plates were left to incubate for 35 minutes at room temperature before the absorbance at 340 nm was read on the EnVision plate reader (PerkinElmer). For kinetic assay format and dose response follow up experiments the same protocol was adopted but the absorbance at 340 nm was monitored constantly for 45 minutes from immediately after the reaction was started.

**Orthogonal Assays**

Previous studies during assay development (Aitken et al. 2017, supra) have shown that the primary screen may be susceptible to identifying redox cycling compounds and small molecule aggregating compounds as false positives. Thus, TritonX-100 aggregation assays were run to screen for compounds which could be causing inhibition *via* aggregation. The primary screen was repeated with the addition of TritonX-100 at a final assay concentration of 0.01 % (v/v). To identify potential redox cycling compounds, the primary screen was repeated with the addition of resazurin at a final assay concentration of 50 μM and no addition of protein. The known redox cycler, 9,10-phenanthrenequinone (9,10 PQ, 40 nM) was used as a positive control and DMSO (1 % v/v) acted as the negative control. Compounds were screened at a final assay concentration of 100 μM, in triplicate, in 96-well black flat-clear bottom plates. After the protein-substrate mixes with compounds were dispensed, the plate was left to incubate in the dark for 30 minutes. The change in fluorescence was then monitored over a 25-minute period (excitation = 560 nm, emission = 590 nm).

**Acetoacetyl-CoA Enzyme Kinetics (Aitken et al. 2017,** supra)

To determine the kinetics for acetoacetyl-CoA and the effect of 17β-HSD10 concentration upon reaction rate, a matrix titration experiment was set up. Previously determined conditions were used with a fixed concentration of 700 μM NADH and a starting concentration of up to 200 μM acetoacetyl-CoA. Doubling dilutions of acetoacetyl-CoA were then coupled with doubling dilutions of 17β-HSD10, which started with a maximum concentration of 40 nM. Data collected from the experiment was analysed using a standard template, from which reaction progress curves were analysed to calculate initial velocities and obtain Km values using the Michaelis-Menten equation (XLFit, ID Business Solutions).

**NADH Enzyme Kinetics (Aitken et al. 2017, supra)**

To determine the kinetics for NADH and the effect of 17β-HSD10 concentration upon reaction rate a matrix

titration experiment was set up. Previously determined conditions (Aitken et al. 2017, supra) were used, 120 μM acetoacetate and a starting concentration of up to 1000 μM NADH. Doubling dilutions of NADH were then coupled with doubling dilutions of 17β-HSD10, which started with a maximum concentration of 40 nM. Data were analysed as described previously (Aitken et al. 2017, supra).

**Physiochemical predictions (Aitken et al. 2017, supra)**

Published values were obtained (where available) from DrugBank (http://www.drugbank.com) and where there were no published values, predictions were made using ChemAxon Marvin suite (http://www.chemaxon.com).

**Thermal shift analysis**

Solutions containing compound at two concentrations (25 μM and 100 μM) added to assay buffer (50 mM Hepes, 0.5 M NaCl, pH 8.2, SYPRO orange 20 X, ± 1 mM NADH) were plated in triplicate (25 μl per well) in MicroAmp 96-well plates (Thermo Fisher: 4346907). The plates were then sealed with optical grade sealing film (Thermo Fisher: 4311971) and loaded into a ViiA 7 RT-PCR machine ((Thermo Fisher: 4453534) (ramp speed 0.015 °C/sec, 25-95 °C)). Data was exported to GraphPad PRISM and $T_m$ values were calculated using the Boltzmann sigmoidal curve fit equation.

**Lactate Dehydrogenase (LDH) cytotoxicity assay**

Cell cytotoxicity was assessed *via* the measurement of lactate dehydrogenase leakage into the culture medium using a commercially available kit from Pierce (Thermo Scientific cat no. 88953). This was carried out in accordance with the kit guidelines, with the activity of Lactate Dehydrogenase (LDH) being calculated from the change in absorbance at 340 nm as NADH is reduced. HEK293 cells overexpressing mts17β-HSD10 were cultured in phenol-red free media (10 % fetal bovine serum (FBS), 1 mM sodium pyruvate, 100 units penicillin, 0.1 mg/mL streptomycin and 2 mM L-glutamine) and seeded at a density of 10,000 cells per well (100 μL, 96 well plates). Cells were then treated with the compound of interest at 2 concentrations (25 μM and 100 μM in DMSO) in triplicate. Treated cells were incubated at 37 °C and $CO_2$ (5 %) for 24 hours before the LDH assay was performed as per the manufacturer's instructions. Spontaneous control (water) and maximum control (lysis buffer) used in accordance with the kit guide. Absorbance was measured at 490 nm and 680 nm using a SpectraMaxM2e spectrophotometer. The measured LDH activity was used to calculate %cytotoxicity using the following equation:

$$\%\ \boldsymbol{cytotoxicity} = \frac{(\boldsymbol{compound\ treated\ LDH\ Activity - Spontaneous\ LDH\ Activity})}{(\boldsymbol{Maximum\ LDH\ Activity - Spontaneous\ LDH\ Activity})}\ \boldsymbol{x100}$$

**Alamar Blue Cell viability Assay**

Cell viability was assessed *via* fluorescence change using the commercially available Alamar Blue from ThermoFisher. HEK293 cells overexpressing mts17β-HSD10 were cultured in phenol-red free media (10% FBS, 1 mM sodium pyruvate, 100 units penicillin, 0.1 mg/mL streptomycin and 2 mM L-glutamine) and seeded at a density of 10,000 cells per well (100 μL, 96 well plates). Cells were then treated with the compound of interest at 2 concentrations (25 μM and 100 μM in DMSO) in triplicate. Treated cells were then incubated at 37 °C and $CO_2$ (5%) for 24 hours before the assay was performed as per the manufacturer's instructions. Fluorescence was measured at 530 nm excitation and 590 nm emission using a SpectraMaxM2e spectrophotometer.

**CHANA assay - *In vitro* dose response and $IC_{50}$ determination**

HEK293 mts17β-HSD10 cells were seeded at a density of 10,000 cells per well (100 μL, 96 well black plates) in phenol-red free media (10% FBS, 1 mM sodium pyruvate, 100 units penicillin, 0.1 mg/mL streptomycin and 2 mM L-glutamine). After 24 hours the media was removed from the cells and replaced with fresh media containing varying concentrations of compound (100 μM - 0.098 μM). The fluorogenic probe (-)-CHANA was then added to each well to give a final assay concentration of 20 μM. Fluorescence was immediately measured using a FLUOstar Optima microplate reader (excitation = 380 nm, emission = 520 nm, orbital averaging = 3mm) and the initial reaction monitored for 3-4 hours. $IC_{50}$ was calculated from the control - subtracted triplicates using non-linear regression (four parameters) of GraphPad Prism 5 software. Final $IC_{50}$ and SEM value was obtained as a mean of at least 3 independent measurements.

## Chemical preparation and synthesis

Standard experimental procedures were followed for synthesis; chemicals and solvents were from commonly used suppliers and were used without further purification. Silica gel 60 F254 analytical thin layer chromatography (TLC) plates were from Merck (Darmstadt, Germany) and visualized under UV light and/or with potassium permanganate stain. Chromatographic purifications were performed using Merck Geduran 60 silica (40-63 μm) or prepacked SNAP columns using a Biotage SP1 Purification system (Uppsala, Sweden). Microwave assisted reactions were performed using a Biotage Initiator™ microwave synthesizer in sealed vials. Deuterated solvents were obtained from Cambridge Isotopes, Sigma-Aldrich, Goss Scientific Instruments Ltd. and Apollo Scientific Ltd. All $^{1}H$ and $^{13}C$ NMR spectra were recorded using a Bruker Avance 400 MHz spectrometer. All chemical shifts are given in ppm relative to the solvent peak and coupling constants (*J*) are reported in Hz. Low Resolution (LR) mass spectrometry data (m/z) were obtained from an Agilent 6140 series Quadrupole Mass Spectrometer with a multimode source attached to an Agilent 1200 series HPLC.

## Analytical Methods

### Liquid-chromatography-mass spectrometry (LC-MS)

*Analytical Method A*

LC-MS was performed using an Agilent 6140 Series Quadrupole Mass Spectrometer with a multimode source. Analysis was performed using either a Phenomenex Luna® C18 (2)-HST column (2.5 μm, 50 x 2.0 mm) or a Waters X-select® CSH ™ C18 column (2.5 μm, 50 x 2.1 mm). Mobile phase A contained 0.1% formic acid in 18 MΩ water and mobile phase B contained 0.1% formic acid in HPLC grade acetonitrile. A flow rate of 1.00 mL $min^{-1}$ was used over a 3.75 min gradient starting with 99% mobile phase A gradually increasing to 100% mobile phase B. The samples were monitored at 254 nm.

*Analytical Method B*

LC-MS was performed using an Agilent 6140 Series Quadrupole Mass Spectrometer with a multimode source. Analysis was performed using either a Phenomenex Luna® C18 (2)-HST column (2.5 μm, 50 x 2.0 mm) or a Waters X-select® CSH ™ C18 column (2.5 μm, 50 x 2.1 mm). Mobile phase A contained 0.1% formic acid in 18 MΩ water and mobile phase B contained 0.1% formic acid in HPLC grade acetonitrile. A flow rate of 1.00 mL $min^{-1}$ was used over a 5.5 min gradient starting with 99% mobile phase A gradually increasing to 100% mobile phase B. The samples were monitored at 254 nm.

*Analytical Method C*

LC-MS was performed using an Agilent 6140 Series Quadrupole Mass Spectrometer with a multimode source. Analysis was performed using either a Phenomenex Luna® C18 (2)-HST column (2.5 μm, 50 x 2.0 mm) or a Waters X-select® CSH ™ C18 column (2.5 μm, 50 x 2.1 mm). Mobile phase A contained 0.01M $NH_4OH$ in 18 MΩ water and mobile phase B contained 0.01M $NH_4OH$ in HPLC grade MeOH. A flow rate of 1.00 mL $min^{-1}$ was used over a 3.75 min gradient starting with 99% mobile phase A gradually increasing to 100 % mobile phase B. The samples were monitored at 254 nm.

### Preparative High Performance Liquid Chromatography (HPLC) Method

Preparative HPLC was carried out on a Waters HPLC comprising of a Waters 2767 Sample Manager, Waters 2545 Binary Gradient Module, Waters Systems Fluidics Organiser, Waters 515 ACD pump, Waters 2998 Photodiode Array Detector, using a Waters XBridge Prep OBD C18, 5 μm, 19 mm x 50mm i.d. column and a flow rate of 20 mL / minute. The general method that may be used to purify compounds are: acidic reverse phase HPLC (water / acetonitrile / 0.1% trifluoroacetic acid) using a standard gradient of 5% acetonitrile / 95% water to 100% acetonitrile or basic reverse phase HPLC ( water / acetonitrile / 0.01 M ammonia solution) using a standard gradient of 10% acetonitrile / 90% water to 100% acetonitrile. UV detection e.g. 254 nM is used for the collection of fractions from HPLC. This description gives general methods and variations in types of equipment, columns, mobile phase, detection wavelength, solvent gradient and run time may also be used to purify compounds.

## Synthesis of selected compounds

The chemical synthesis of an exemplified compound of formula (I) is outlined in Schemes 1 and 2.

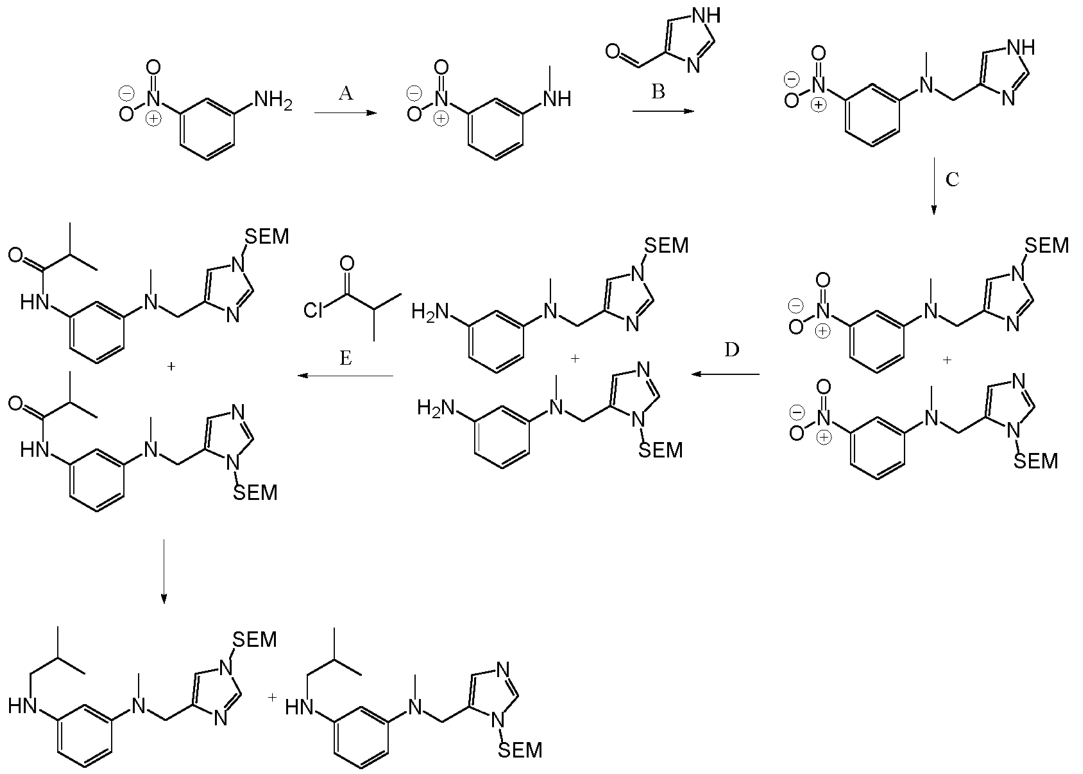


**Scheme 1:** Route to N1-isobutyl-N3-methyl-N3-((1-((2-(trimethylsilyl)ethoxy)methyl)-1H-imidazol-4-yl)methyl)benzene-1,3-diamine and N1-isobutyl-N3-methyl-N3-((1-((2-(trimethylsilyl)ethoxy)methyl)-1H-imidazol-5-yl)methyl)benzene-1,3-diamine 1:1 regioisomeric mixture. A: i) NaOMe, paraformaldehyde, MeOH, RT, 24h, ii) NaBH4, 80°C, 2h; B: AcOH, $Na(OAc)_3BH$, DCM, RT, 72h; C: NaH, SEM-Cl, THF, 0°C - RT, 2h; D: $H_2$, Pd/C, MeOH, 30°C, 20bar; E: DIEA, DCM, 0°C - RT, 1h; F: i) $BH_3$.THF, THF, RT - 70°C, 92h, ii) MeOH, 70°C, 24h.

Synthesis of N-methyl-3-nitroaniline

3-nitroaniline (5 g, 36.2 mmol) was added to a suspension of NaOMe (95%, 10.29 g, 181 mmol) in MeOH (30 mL) and the resulting suspension stirred and then added to a stirring suspension of paraformaldehyde (1.52 g, 50.68 mmol) in methanol (40 mL). The reaction was stirred at room temperature for 18 hours then $NaBH_4$ (1.37 g, 36.2 mmol) was added and the reaction heated to reflux for 2 hours. The reaction mixture was allowed to cool to room temperature then concentrated *in vacuo.* The mixture was partitioned between water (100ml) and EtOAc (150ml). The aqueous layer was separated and extracted with additional EtOAc (100ml). Organics were combined, washed with brine, dried over sodium sulphate, filtered and solvent was evaporated in *vacuo.* The crude product was purified by flash column chromatography (silica column, 0% to 100% DCM in *n*-heptane gradient) followed by concentration of the appropriate fractions *in vacuo* to afford N-methyl-3-nitro-aniline as an orange solid (4.84 g, 88 %).
$^1$H NMR (400 MHz, $CDCl_3$) δ 7.54 (dd, *J* = 1.51, 8.03 Hz, 1H), 7.41 (t, *J* = 2.26 Hz, 1H), 7.26 - 7.33 (m, 1H), 6.89 (dd, *J* = 2.01, 8.03 Hz, 1H), 4.10 (br. s., 1H), 2.93 (br. s., 3H).

Synthesis of N-((1H-imidazol-4-yl)methyl)-N-methyl-3-nitroaniline

N-methyl-3-nitro-aniline (4 g, 26.29 mmol) and 1H-imidazole-5-carbaldehyde (3.28 g, 34.18 mmol) were combined in DCM (75 mL) and acetic acid (2.26 mL, 39.43 mmol) was added. The suspension was stirred for 30 minutes then sodium triacetoxyborohydride (11.14 g, 52.58 mmol) was added. Stirring was continued at room temperature for 72 hours. The reaction mixture was diluted with DCM (75 mL) and washed with NaOH (2M aq., 100 mL). The aqueous wash was extracted with DCM (2 x 100 mL) then organics were combined, washed with brine, dried over sodium sulphate, filtered and solvent was evaporated *in vacuo* to afford crude product as an orange oil. Purification by flash column chromatography (silica column, DCM 0 % to 10 % gradient of a solution of 10 % $NH_4OH$ in MeOH) followed by

concentration of the appropriate fractions *in vacuo* afforded N-((1H-imidazol-4-yl)methyl)-N-methyl-3-nitroaniline as an orange gum (4.19 g, 69 %).
$^1$H NMR (400 MHz, $CDCl_3$) δ 7.64 (d, *J* = 0.75 Hz, 1H), 7.57 (t, *J* = 2.38 Hz, 1H), 7.51 (dd, *J* = 1.51, 8.03 Hz, 1H), 7.30 (t, *J* = 8.16 Hz, 1H), 7.05 (dd, *J* = 2.38, 8.41 Hz, 1H), 6.85 (s, 1H), 4.57 (s, 2H), 3.11 (s, 3H).

Synthesis of N-methyl-3-nitro-N-((1-((2-(trimethylsilyl)ethoxy)methyl)-1H imidazol-4-yl)methyl)aniline and N-methyl-3-nitro-N-((1-((2-(trimethylsilyl)ethoxy)methyl)-1H-imidazol-5-yl)methyl)aniline 1:1 regioisomeric mixture

NaH (60 %, 1.26 g, 31.39 mmol) was suspended in THF (20 mL) placed under argon and cooled to 0°C. A solution of N-(1H-imidazol-5-ylmethyl)-N-methyl-3-nitroaniline (6.08 g, 26.16 mmol) in THF (80 mL) was added slowly and the mixture stirred at 0°C for 45 minutes. 2-(Trimethylsilyl)ethoxymethyl chloride (6.95 mL, 39.24 mmol) was added and the reaction stirred at 0°C for 30 minutes and at room temperature for 2 hours. The reaction mixture was concentrated *in vacuo* to remove THF and the resulting suspension partitioned between EtOAc (150 mL) and NaOH (1 M, 100 mL). The aqueous layer was extracted with EtOAc (50 mL) then organics were combined, washed with brine, dried over sodium sulphate, filtered and concentrated *in vacuo* to afford crude product as an orange solid. Purification by flash column chromatography (silica column, DCM 0 % to 10 % gradient of a solution of 10 % $NH_4OH$ in MeOH) followed by evaporation of solvent from the appropriate fractions afforded a 1:1 regioisomeric mixture of the title compounds as an orange oil (6.02 g, 64 %).
LC-MS Analytical Method A: rt = 1.52, 1.58 min, *m*/*z* 363.2 $[M+H]^+$.

Synthesis of $N^1$-methyl-$N^1$-((1-((2-(trimethylsilyl)ethoxy)methyl)-1H-imidazol-4-yl)methyl)benzene-1,3-diamine and $N^1$-methyl-$N^1$-((1-((2-(trimethylsilyl)ethoxy)methyl)-1H-imidazol-5-yl)methyl)benzene-1,3-diamine 1:1 regioisomeric mixture

A solution of N-methyl-3-nitro-N-((1-((2-(trimethylsilyl)ethoxy)methyl)-1H-imidazol-4-yl)methyl)aniline and N-methyl-3-nitro-N-((1-((2-(trimethylsilyl)ethoxy)methyl)-1H-imidazol-5-yl)methyl)aniline 1:1 regioisomeric mixture (1.13 g, 3.12 mmol) in MeOH (100ml) was passed through a H-Cube hydrogenation system equipped with a 10% Pd/C cartridge at 1ml/min flow rate, 30°C and a hydrogen pressure of 20 bar. The eluant was concentrated *in vacuo* to afford a brown oil. Purification by flash column chromatography (silica column, DCM 0 % to 10 % gradient of a solution of 10 % $NH_4OH$ in MeOH) followed by evaporation of solvent from the appropriate fractions afforded the title compound regioisomeric mixture as a yellow oil (0.86 g, 83 %).
LC-MS Analytical Method A: rt = 1.29 min, *m*/*z* 333.2 $[M+H]^+$.

Synthesis of N-(3-(methyl((1-((2-(trimethylsilyl)ethoxy)methyl)-1H-imidazol-4-yl)methyl)amino)phenyl)isobutyramide and N-(3-(methyl((1-((2-(trimethylsilyl)ethoxy)methyl)-1H-imidazol-5-yl)methyl)amino)phenyl)isobutyramide 1:1 regioisomeric mixture

$N^1$-methyl-$N^1$-((1-((2-(trimethylsilyl)ethoxy)methyl)-1H-imidazol-4-yl)methyl)benzene-1,3-diamine and $N^1$-methyl-$N^1$-((1-((2-(trimethylsilyl)ethoxy)methyl)-1H-imidazol-5-yl)methyl)benzene-1,3-diamine 1:1 regioisomeric mixture (1.36 g, 4.08 mmol) was dissolved in DCM (20 mL) and DIEA (2.13 mL, 12.23 mmol) was added. The solution was cooled to 0°C, 2-methylpropanoyl chloride (0.56 mL, 5.3 mmol) was added and the reaction was stirred at 0°C for 1 hour. The reaction mixture was diluted with DCM (20 mL) and washed with water (20ml) saturated sodium bicarbonate solution (20 mL) and brine. Organics were dried over sodium sulphate, filtered and solvent was evaporated *in vacuo* to afford a brown oil. Purification by flash column chromatography (silica column, DCM 0 % to 10% gradient of a solution of 10% $NH_4OH$ in MeOH) followed by evaporation of solvent from the appropriate fractions afforded the title compound regioisomeric mixture as a pale brown oil (1.1 g, 67 %).
LCMS Analytical Method A: rt = 1.49 min, *m*/*z* 403.2 $[M+H]^+$.

Synthesis of $N^1$-isobutyl-$N^3$-methyl-$N^3$-((1-((2-(trimethylsilyl)ethoxy)methyl)-1H-imidazol-4-yl)methyl)benzene-1,3-diamine and $N^1$-isobutyl-$N^3$-methyl-$N^3$-((1-((2-(trimethylsilyl)ethoxy)methyl)-1H-imidazol-5-yl)methyl)benzene-1,3-diamine 1:1 regioisomeric mixture.

N-(3-(methyl((1-((2-(trimethylsilyl)ethoxy)methyl)-1H-imidazol-4-yl)methyl)amino)phenyl)isobutyramide and N-(3-(methyl((1-((2-(trimethylsilyl)ethoxy)methyl)-1H-imidazol-5-yl)methyl)amino)phenyl)isobutyramide 1:1 regioisomeric mixture (1.1 g, 2.73 mmol) was dissolved in 2-MeTHF (7.5 mL) and borane THF complex solution (1 M, 7.5 mL, 7.50 mmol) was added slowly at room temperature. The reaction was heated to 70°C for 20 hours then additional borane THF complex solution (1 M, 7.5 mL, 7.50 mmol) was added and heating continued for 72 hours. MeOH (20 mL) was added and heating continued at 70°C for 24 hours. Solvent was evaporated *in vacuo* and the resulting brown oil loaded onto silica and purified by flash column chromatography (silica column, DCM 0 % to 10 % gradient of a solution of 10 % $NH_4OH$ in

MeOH). Evaporation of solvent from the appropriate fractions afforded the title compound regioisomeric mixture as a pale brown gum (0.68 g, 64 %).
LC-MS Analytical Method A: rt = 1.54 min, *m/z* 389.2 [M+H]+.

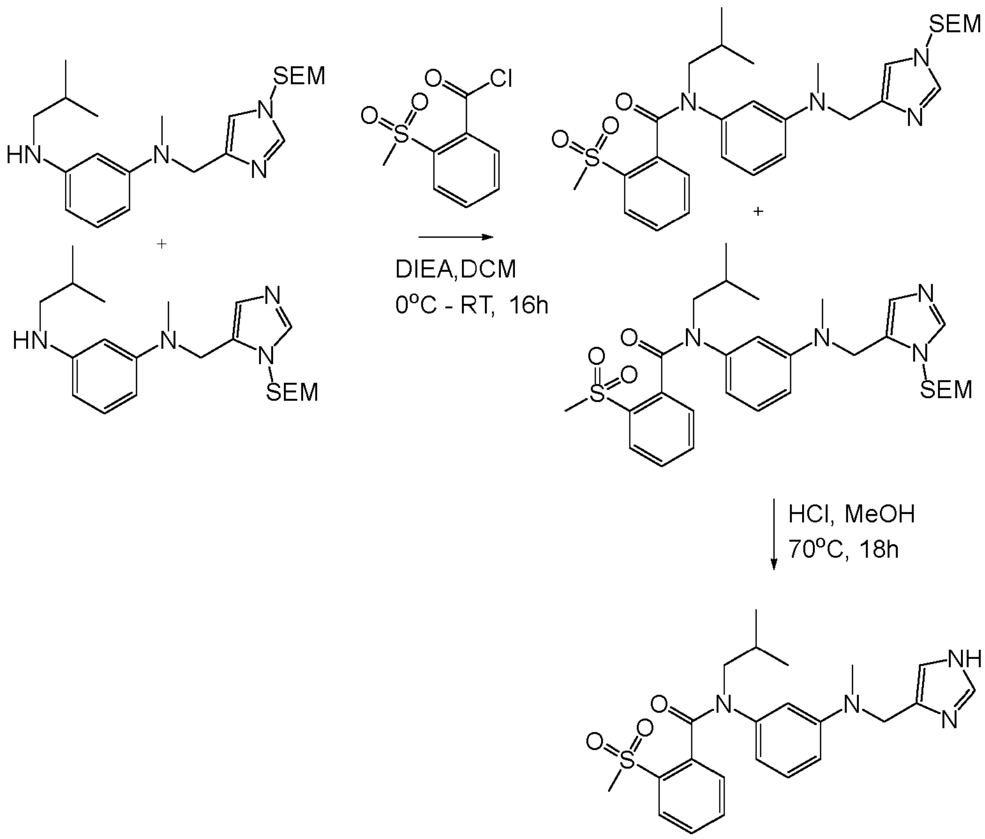


**Scheme 2:** General Procedure for amide formation with subsequent HCl deprotection.

Preparation of N-(3-(((1H-imidazol-4-yl)methyl)(methyl)amino)phenyl)-N-isobutyl-2-(methylsulfonyl)benzamide.

N-isobutyl-N-(3-(methyl((1-((2-(trimethylsilyl)ethoxy)methyl)-1H-imidazol-4-yl)methyl)amino)phenyl)-2-(methylsulfonyl) benzamide and N-isobutyl-N-(3-(methyl((1-((2-(trimethylsilyl)ethoxy)methyl)-1H-imidazol-5-yl)methyl)amino)phenyl)-2-(methylsulfonyl)benzamide 1:1 regioisomeric mixture.

$N^1$-isobutyl-$N^3$-methyl-$N^3$-((1-((2-(trimethylsilyl)ethoxy)methyl)-1H-imidazol-4-yl)methyl)benzene-1,3-diamine and $N^1$-isobutyl-$N^3$-methyl-$N^3$-((1-((2-(trimethylsilyl)ethoxy)methyl)-1H-imidazol-5-yl)methyl)benzene-1,3-diamine 1:1 regioisomeric mixture (47 mg, 0.12 mmol) was dissolved in DCM (1 mL) and DIEA (0.21 mL, 1.21 mmol. The solution was cooled to 0°C and 2-methylsulfonylbenzoyl chloride (52.89 mg, 0.24 mmol) was added. The reaction was stirred at 0°C for 30 minutes then 2-methylsulfonylbenzoyl chloride (52.89 mg, 0.24 mmol) was added and the reaction allowed to warm to room temperature and stirred for 16 hours. The reaction mixture was diluted with DCM (3 mL) and washed with HCl (0.5 M, 2 mL). Organics were filtered through a hydrophobic frit and solvent was evaporated *in vacuo* to afford crude product. Purification by flash column chromatography (silica column, DCM 0 % to 2.5 % gradient of a solution of 10 % $NH_4OH$ in MeOH) followed by evaporation of solvent from the appropriate fractions afforded the title compound regioisomeric mixture (60 mg, 87 %). LC-MS Analytical Method A: short method, rt = 1.56 min, *m/z* 571.0 [M+H]+.

N-(3-(((1H-imidazol-4-yl)methyl)(methyl)amino)phenyl)-N-isobutyl-2-(methylsulfonyl)benzamide

N-isobutyl-N-(3-(methyl((1-((2-(trimethylsilyl)ethoxy)methyl)-1H-imidazol-4-yl)methyl)amino)phenyl)-2-(methylsulfonyl)benzamide and N-isobutyl-N-(3-(methyl((1-((2-(trimethylsilyl)ethoxy)methyl)-1H-imidazol-5-yl) methyl)amino)phenyl)-2-(methylsulfonyl)benzamide 1:1 regioisomeric mixture (60 mg, 0.11 mmol) was dissolved in MeOH (1 mL) and HCl (5 M, 0.5 mL) added. The reaction was heated to 70°C for 18 hours then concentrated *in vacuo.* The

afforded residue was dissolved in DCM (2ml) and washed with saturated sodium bicarbonate solution (2ml). The 33queous wash was re-extracted with DCM (2 mL) then organics were combined, filtered through a hydrophobic frit and solvent was evaporated *in vacuo* to afford crude product, 54 mg.

Purification by preparative HPLC (basic) followed by evaporation of solvent and drying afforded N-(3-(((1H-imidazol-4-yl)methyl)(methyl)amino)phenyl)-N-isobutyl-2-(methylsulfonyl)benzamide (29 mg, 63 %).

$^1$H NMR at 298K (400 MHz, DMSO-$d_6$) δ 11.81 (br. S., 1H), 7.85 (d, *J* = 7.78 Hz, 1H), 7.52 (s, 1H), 7.37 - 7.49 (m, 2H), 7.14 (d, *J* = 6.53 Hz, 1H), 6.94 (t, *J* = 8.03 Hz, 1H), 6.83 (br. S., 1H), 6.68 (s, 1H), 6.60 (d, *J* = 7.53 Hz, 1H), 6.44 - 6.53 (m, 1H), 4.23 (br. S., 2H), 3.95 (br. S., 1H), 3.41 - 3.56 (br. S., 1H), 3.37 (s, 3H), 2.70 - 2.85 (m, 3H), 1.68 - 1.83 (m, 1H), 0.92 (br. S., 6H).

High T NMR obtained at 373K to confirm structure:

$^1$H NMR at 373K (400 MHz, DMSO-$d_6$) δ 11.52 (br. S., 1H), 7.87 (d, *J* = 7.28 Hz, 1H), 7.35 - 7.54 (m, 3H), 7.16 (br. S., 1H), 6.95 (br. S., 1H), 6.84 (s, 1H), 6.46 - 6.74 (m, 3H), 4.25 (br. S., 2H), 3.73 (br. S., 2H), 3.30 (s, 3H), 2.81 (br. S., 3H), 1.91 (br. S., 1H), 0.80 - 1.08 (m, 6H).

LC-MS Analytical Method B: rt = 1.69 min, *m/z* 441.2 $[M+H]^+$.

**Synthetic procedures for compounds referred to in Table 6**

Procedure 3 (ESC1002032)

tert-butyl 5-[[3-(diisobutylamino)-N-methyl-anilino]methyl]imidazole-1-carboxylate (37 mg, 0.09 mmol) was dissolved in HCl (4M in dioxane, 2 ml) and the reaction stirred at room temperature for 4 hours (LCMS). The reaction mixture was concentrated under reduced pressure then the residue dissolved in EtOAc (10ml) and washed with saturated sodium bicarbonate solution (10ml). Organics were dried over sodium sulphate, filtered and solvent was evaporated under reduced pressure to afford crude product (28mg). Purification by prep. HPLC (basic) followed by evaporation of solvent from the appropriate fractions afforded product as an off-white solid, 12 mg (42.8%) of N3-(1H-imidazol-5-ylmethyl)-N1,N1-diisobutyl-N3-methyl-benzene-1,3-diamine.

Procedure 4 (ESC1002033)

tert-butyl 5-[[3-(isobutylamino)-N-methyl-anilino]methyl]imidazole-1-carboxylate (85%, 65 mg, 0.15 mmol), 2-fluorobenzoic acid (32.39 mg, 0.23 mmol) and O-(7-Azabenzotriazol-1-yl)-N,N,N',N'-tetramethyluronium hexafluorophosphate (87.9 mg, 0.23 mmol) were charged to a reaction tube and dichloromethane (2 ml) added followed by DIEA (0.07 ml, 0.39 mmol). The reaction was stirred at room temperature for 16 hours. The reaction mixture was diluted with DCM (5ml) and washed with water (2ml). Organics were filtered through a hydrophobic frit and solvent was evaporated under reduced pressure to afford crude product (134mg). Purification by flash chromatography (silica column heptane 10% to 60% EtOAc gradient) followed by evaporation of solvent from the appropriate fractions afforded product 5923A (26mg, Boc-protected). Further eluting the column with a solution of DCM:MeOH:NH4OH (180:10:1) followed by evaporation of solvent from the appropriate fractions afforded product 5923C (6mg, de-protected, LCMS). Product 5923A was dissolved in HCl (4M diox., 1 ml) and stirred at room temperature for 4 hours, when no protected material remained. Solvent was evaporated under reduced pressure then the resulting residue dissolved in EtOAc (10ml) and washed with saturated sodium bicarbonate solution (10ml). Organics were dried over sodium sulphate, filtered and solvent was evaporated under reduced pressure to afford crude product 5923D (22mg). Products 5923C and 5923D were combined for purification by prep. HPLC (basic). Evaporation of solvent from the appropriate fractions and drying under vacuum afforded product as a colourless glass, 20 mg (34.1%) of 2-fluoro-N-[3-[1H-imidazol-5-ylmethyl(methyl)amino]phenyl]-N-isobutyl-benzamide.

ESC1002032

Procedure 4 with 4-methylbenzoic acid (31.48 mg, 0.23 mmol) in place of 2-fluorobenzoic acid and additional 4-methylbenzoic acid (15 mg) O-(7-Azabenzotriazol-1-yl)-N,N,N',N'-tetramethyluronium hexafluorophosphate (44 mg) and DIEA (40 μl) added after 16 hours, with stirring continued for 5 days. The reaction mixture was diluted with DCM (5ml) and washed with 0.5M HCl (aq., 5ml). Crude product (258mg). Flash chromatography (silica column, heptane 0% to 100% EtOAc gradient) afforded product 5922A (42mg, boc-protected product, contains tetramethylurea impurity). 5922A was dissolved in HCl (4M in dioxane, 1ml), stirred at room temperature for one hour and allowed to stand for 16 hours. Crude product (29mg) was extracted as in procedure D. Purification by prep. HPLC (basic method) followed by evaporation of solvent from the appropriate fractions under reduced pressure afforded product as a white solid, 23 mg (39.6%) of N-[3-[1H-imidazol-5-ylmethyl(methyl)amino]phenyl]-N-isobutyl-4-methyl-benzamide

Procedure 5 (ESC1002038)

tert-butyl 5-[[3-(isobutylamino)-N-methyl-anilino]methyl]imidazole-1-carboxylate (65 mg, 0.18 mmol) was dissolved in DCM (1ml) and DIEA (0.08 ml, 0.45 mmol) added. The solution was cooled to 0°C then a solution of 3-methoxybenzoyl chloride (46.4 mg, 0.27 mmol) in DCM (0.5ml) was added. The reaction was allowed to warm to room temperature and stirred for 16 hours (LCMS). The reaction mixture was partitioned between DCM (5ml) and HCl (aq. 0.5M, 5ml) then organics were filtered through a hydrophobic frit and solvent was evaporated to afford crude intermediate product (121mg). Purification by flash chromatography (silica column, heptane 0% to 100% EtOAc gradient) followed by evaporation of solvent afforded two products, 5933A and 5933B. Product 5933A consisting of mainly a side product but also some desired boc-protected product present, product 5933B being mainly desired boc-protected product. Each intermediate product was separately dissolved in HCl (4M in dioxane, 2ml) stirred at room temperature for one hour and allowed to stand for 16 hours. Solvent was evaporated from each under reduced pressure then each residue was dissolved in EtOAc (5ml) and washed with saturated sodium bicarbonate solution (2ml). Organics were dried over sodium sulphate, filtered and solvent was evaporated under reduced pressure to afford a yellow oil in each case. 5933A afforded 38mg (LCMS) and 5933B afforded 26mg (LCMS). Both residues were purified by prep. HPLC (basic). Product fractions from each were combined and solvent was evaporated under reduced pressure to afford product as a pale yellow solid, 20 mg (28.1%) of N-[3-[1H-imidazol-5-ylmethyl(methyl)amino]phenyl]-N-isobutyl-3-methoxy-benzamide.

(ESC1002034)

Procedure 5 with pyridine-4-carbonyl chloride hydrochloride (48.42 mg, 0.27 mmol) in place of a solution of 3-methoxybenzoyl chloride. Crude intermediate product (64mg, LCMS), mainly boc-protected desired product). The aqueous wash was basified with addition of NaOH then extracted with DCM (2 x 3ml). Organics were combined, filtered through a hydrophobic frit and solvent was evaporated under reduced pressure to afford a second crude product (26mg, LCMS, mainly de-protected desired product). The first (boc-protected) crude product was isolated as a yellow oil (LCMS) as described in procedure E for the intermediate products. The two crude residues were purified by prep. HPLC (basic) to afford product as a pale yellow solid, 17 mg (25.8%) of N-[3-[1H-imidazol-5-ylmethyl(methyl)amino]phenyl]-N-isobutyl-pyridine-4-carboxamide.

ESC1002042

Procedure 5 with 4-chloro-2-fluoro-benzoyl chloride (52.49 mg, 0.27 mmol) in place of 3-methoxybenzoyl chloride. Crude intermediate product (114mg). Purification by flash chromatography (silica column, heptane 0% to 50% EtOAc gradient) afforded two products, 5932A and 5932B. Product 5932A consisting of mainly a side product but also some desired boc-protected product present, product 5932B (LCMS) being mainly desired boc-protected product. Each intermediate product was isolated as a yellow oil following the methods described in procedure E. 5932A afforded 26mg (LCMS) and 5932B afforded 33mg (LCMS). Both residues were purified by prep. HPLC to afford product as a pale yellow solid, 28 mg (37.2%) of 4-chloro-2-fluoro-N-[3-[1H-imidazol-5-ylmethyl(methyl)amino]phenyl]-N-isobutyl-benzamide.

Procedure 8 (ESC1002078)

tert-butyl 5-[[3-(isopropylamino)-N-methyl-anilino]methyl]imidazole-1-carboxylate (45 mg, 0.13 mmol) was dissolved in DCM (2ml) and DIEA (0.07 ml, 0.39 mmol) was added. The solution was cooled to 0°C then a solution of 2-fluorobenzoyl chloride (31.07 mg, 0.2 mmol) in DCM (0.5ml) was added dropwise. The reaction was allowed to warm to room temperature and stirred for one hour, then allowed to stand for 16 hours. The reaction mixture was diluted with DCM (5ml) and washed with saturated sodium bicarbonate solution (5ml). Organics were filtered through a hydrophobic frit and solvent was evaporated under reduced pressure to afford crude intermediate as a yellow gum. The intermediate was dissolved in HCl (4M in dioxane, 1.5 ml) and stirred for 3 hours (LCMS). Solvent was evaporated under reduced pressure to afford a yellow gum. Purification by prep. HPLC (basic) followed by evaporation of solvent from the appropriate fractions and drying under vacuum afforded product, 18 mg (37.6%) of 2-fluoro-N-[3-[1H-imidazol-5-ylmethyl(methyl)amino]phenyl]-N-isopropyl-benzamide.

ESC1002080

Procedure 8 with 4-methylbenzoyl chloride (30.29 mg, 0.2 mmol) in place of 2-fluorobenzoyl chloride. Product isolated as 28 mg (59.1%) of N-[3-[1H-imidazol-5-ylmethyl(methyl)amino]phenyl]-N-isopropyl-4-methyl-benzamide.

Procedure 10 (ESC1002082)

[2-(2-bromophenyl)pyrrolidin-1-yl]-(3-methyl-2-thienyl)methanone (110 mg, 0.31 mmol) (6-methoxy-3-pyridyl) boronic acid (57.64 mg, 0.38 mmol) and 'first choice' catkit (158mg consisting of $Pd_2(dba)_3$ 0.05 eqiuv., $P(cy)_3$ 0.2 equiv., and $K_3PO_4$ 2 equiv.) were charged to a sealable vial. 1,4-Dioxane (1 ml) was added followed by water (0.2ml). The vial was sealed and placed under argon then the solution was de-gassed with a flow of argon for 10 minutes. The reaction was heated to 80°C for 20 hours (LCMS t = 5 hours, LCMS t = 20 hours). The reaction mixture was diluted with EtOAc (5ml) and water (2ml) and filtered through celite. Organics were separated, washed with water (5ml) and brine, dried over sodium sulphate, filtered and solvent was evaporated under reduced pressure to afford crude product, 126mg. Purification by flash chromatography (silica column, heptane 0% to 100% gradient) followed by evaporation of solvent from the appropriate fractions under reduced pressure and drying under vacuum afforded product, 80 mg (67.3%) of [2-[2-(6-methoxy-3-pyridyl)phenyl]pyrrolidin-1-yl]-(3-methyl-2-thienyl)methanone.

ESC1002083

Procedure 9 with (2-fluorophenyl)-[7-(methylamino)-3,4-dihydro-2H-quinolin-1-yl]methanone (130 mg, 0.46 mmol), 1H-imidazole-5-carbaldehyde (52.72 mg, 0.55 mmol) and acetic acid (0.04 ml, 0.69 mmol) and sodium triacetoxyborohydride (193.8 mg, 0.91 mmol). Crude product, 190mg. Product was isolated as 71 mg (42.6%) of (2-fluorophenyl)-[7-[1H-imidazol-5-ylmethyl(methyl)amino]-3,4-dihydro-2H-quinolin-1-yl]methanone.

Procedure 11 (ESC 1002089)

Suzuki cross coupling: aryl bromide, the desired boronic acid, the solvents (dioxane+water) and the mixture was added to a microwave vial and bubbled with argon for ~5 min. Then the catkit first choice was added to the reaction mixture and the vessel was sealed before heating it to 80°C for the required time. Once completed the mixture was diluted with EtOAc and Water and extracted with EtOAc (3x 15ml). The org. layers were dried over $Na_2SO_4$, and reduced before charging the mixture on a 10g silica cartridge. The mixture was purified on an Isolera and the desired fractions were collected. The solids obtained were further dried in a vac. oven (50°C overnight).
ESC1002332 was obtained by SFC chiral separation of ESC1002089.

Procedure 12 (ESC1002164)

tert-butyl 5-[[3-[(2-fluorobenzoyl)amino]-N-methyl-anilino]methyl]imidazole-1-carboxylate (44 mg, 0.1 mmol) was dissolved in DMF (1ml) and NaH (60%, 4.98 mg, 0.12 mmol) was added. The reaction was stirred at room temperature for 20 minutes. 1-bromo-2-methoxy-ethane (0.01 ml, 0.1 mmol) was added and stirring continued at room temperature for 4 hours then the reaction was heated to 60°C for 16 hours (LCMS). Additional NaH (60%, 4.98 mg, 0.12 mmol) and 1-bromo-2-methoxy-ethane (0.01 ml, 0.1 mmol) were added and heating to 60°C was continued for 5 hours. The reaction was allowed to cool to room temperature. The reaction mixture was diluted with EtOAc (20ml) and washed with water (3 x 20ml) and brine (10ml). Organics were dried over sodium sulphate, filtered and solvent was evaporated under reduced pressure to afford crude product as a pale yellow gum, 59mg. Purification by prep. HPLC (basic, 'late' focussed gradient) followed by evaporation of solvent under reduced pressure and drying afforded products EXP-16-FB5962A and EXP-16-FB5962B. EXP-16-FB5962B product of double alkylation: 4 mg (8.8%) of 2-fluoro-N-(2-methoxyethyl)-N-[3-[[1-(2-methoxyethyl)imidazol-4-yl]methyl-methyl-amino]phenyl]benzamide. Product EXP-16-FB5962B confirmed as ESC1002164-01.

Procedure 13 (ESC1002165)

tert-butyl 5-[[3-(isobutylamino)-N-methyl-anilino]methyl]imidazole-1-carboxylate (45 mg, 0.13 mmol) was dissolved in DCM (1ml) and DIEA (0.11 ml, 0.63 mmol) was added. The solution was cooled in an ice-bath and 2-methylpropanoyl chloride (0.02 ml, 0.19 mmol) was added. The reaction was allowed to warm to room temperature and stirred for 3 hours (LCMS). The reaction mixture was diluted with DCM (5ml) and washed with water (2ml). Organics were filtered through a hydrophobic frit and solvent was evaporated to afford crude intermediate. The intermediate was dissolved in HCl (4M diox., 1 ml) and stirred at room temperature for 3 hours. Solvent was evaporated under reduced pressure. The afforded residue was dissolved in 1:1 DMSO / MeOH and sodium carbonate added. The suspension was stirred for 30 minutes and filtered. Purification by prep. HPLC (basic) followed by evaporation of solvent from the appropriate fractions. The resulting residues were dissolved in DCM and combined in a vial. Solvent was evaporated and the resulting gum dried under vacuum. The afforded gum was difficult to handle so was dissolved in MeOH / water then solvent was evaporated (genevac, HPLC lyophilisation program). The afforded pale yellow gum was dried under vacuum

to afford product, 23 mg (55.8%) of N-[3-[1H-imidazol-5-ylmethyl(methyl)amino]phenyl]-N-isobutyl-2-methyl-propanamide.

ESC1002166

Procedure 13 with cyclohexanecarbonyl chloride (0.03 ml, 0.19 mmol) in place of 2-methylpropanoyl chloride. The product was obtained as 27 mg (58.4%) of N-[3-[1H-imidazol-5-ylmethyl(methyl)amino]phenyl]-N-isobutyl-cyclohexanecarboxamide.

ESC1002167

Procedure 13 with 2-phenylacetyl chloride (0.02 ml, 0.19 mmol) in place of 2-methylpropanoyl chloride. The product was obtained as a pale yellow gum, 25 mg (52.9%) of N-[3-[1H-imidazol-5-ylmethyl(methyl)amino]phenyl]-N-isobutyl-2-phenyl-acetamide.

ESC1002168

Procedure 13 with 3-phenylpropanoyl chloride (0.03 ml, 0.19 mmol) in place of 2-methylpropanoyl chloride. The product was obtained as a pale yellow gum, 39 mg (79.6%) of N-[3-[1H-imidazol-5-ylmethyl(methyl)amino]phenyl]-N-isobutyl-3-phenyl-propanamide.

ESC1002169

Procedure 13 with benzoyl chloride (0.02 ml, 0.19 mmol) in place of 2-methylpropanoyl chloride. The product was obtained as a yellow gum, 26 mg (57.1%) of N-[3-[1H-imidazol-5-ylmethyl(methyl)amino]phenyl]-N-isobutyl-benzamide.

ESC1002171

Procedure 13 with acetyl chloride (0.01 ml, 0.18 mmol) in place of 2-methylpropanoyl chloride. The product was isolated as a yellow gum, 22 mg (62.5%) of N-[3-[1H-imidazol-5-ylmethyl(methyl)amino]phenyl]-N-isobutyl-acetamide.

ESC1002172

Procedure 13 with naphthalene-1-carbonyl chloride (0.03 ml, 0.19 mmol) in place of 2-methylpropanoyl chloride. The product was isolated as a pale yellow solid, 22 mg (42.5%) of N-[3-[1H-imidazol-5-ylmethyl(methyl)amino]phenyl]-N-isobutyl-naphthalene-1-carboxamide.

ESC1002182

Procedure 13 with 4-methoxybenzoyl chloride (32.12 mg, 0.19 mmol) in place of 2-methylpropanoyl chloride. $^1H$ NMR / LCMS indicated product was not pure. Purification by prep. HPLC (basic, 'late' focussed gradient method) followed by evaporation of solvent from the appropriate fractions afforded the product as a sticky gum. The gum was dissolved in EtOH and water and solvent evaporated (genevac, lyophilisation program) to afford a solid. Drying the solid under vacuum caused the solid to melt and form a gum - product, 14 mg (28.4%) of N-[3-[1H-imidazol-5-ylmethyl(methyl)amino] phenyl]-N-isobutyl-4-methoxy-benzamide.

ESC1002183

Procedure 13 with 4-chlorobenzoyl chloride (0.02 ml, 0.19 mmol) in place of 2-methylpropanoyl chloride. The product was isolated as 9 mg (18.1%) of 4-chloro-N-[3-[1H-imidazol-5-ylmethyl(methyl)amino]phenyl]-N-isobutyl-benzamide.

Procedure 15 (ESC1002199 to ESC1002214)

2-fluoro-N-isobutyl-N-[3-(methylamino)phenyl]benzamide (198.25 mg, 0.66 mmol) was added to a vial containing the desired aldehyde (24.9 mg, 0.86 mmol) and dissolved in $CH_3CO_2H$ (1 ml, 17.48 mmol). To the mixture was added $(CH_3COO)_3BHNa$ (209.82 mg, 0.99 mmol). The mixture was stirred overnight at rt. The solutions were quenched with 2M NaOH (2ml) and extracted with EtOAc (3x10ml). The organic layers were dried over $Na_2SO_4$ then reduced. Some

compounds were purified on an Isolera (Biotage) whereas other were purified on a basic prep. Most compounds were gums, therefore it was decided to salt them by addition of 2ml $Et_2O$ and 1.2eq of 4M HCl in Dioxane. Most compounds seems to have salted successfully, although some showed sign of degradation. Therefore, they were re-purified by prep HPLC (basic) and submitted as free base.

Procedure 17 (ESC1002235)

To a solution of tert-butyl 5-[[3-(isobutylamino)-N-methyl-anilino]methyl]imidazole-1-carboxylate (42 mg, 0.12 mmol) in NMP (1ml) was added DIEA (0.06 ml, 0.35 mmol). The solution was cooled to 0°C and butanoyl chloride (18.73 mg, 0.18 mmol) added. The reaction was allowed to warm to room temperature and stirred for 90 minutes (LCMS). The reaction mixture was diluted with DCM (5ml) and washed with NaOH (2M aq., 4ml). Organics were filtered through a hydrophobic frit and the mixture concentrated to afford an NMP solution of crude product. The solution was diluted with HCl (4M diox., 2 ml) and the mixture stirred for 2 hours and concentrated to remove dioxane and excess HCl. Sodium carbonate was added to the resulting NMP solution and the mixture stirred for 30 minutes and then filtered. Purification by prep. HPLC (basic) followed by evaporation of solvent from the appropriate fractions and drying afforded product as a gum, 18mg. $^1H$ NMR indicates product is not sufficiently pure for submission for testing. Purification by prep. HPLC (basic, late focussed gradient) followed by evaporation of solvent from the appropriate fractions and drying under vacuum afforded product, 15 mg (39%) of N-[3-[1H-imidazol-5-ylmethyl(methyl)amino]phenyl]-N-isobutyl-butanamide.

Procedure 18 (ESC1002236)

tert-butyl 5-[[7-(isobutylamino)-3,4-dihydro-2H-quinolin-1-yl]methyl]imidazole-1-carboxylate (50%, 79 mg, 0.1 mmol) was dissolved in DCM (1ml) and DIEA (0.18 ml, 1.03 mmol) was added. The solution was cooled to 0°C and 2-fluorobenzoyl chloride (0.02 ml, 0.15 mmol) was added. The reaction was allowed to warm to room temperature and stirred for 3 hours (LCMS). The reaction mixture was diluted with DCM (5ml) and washed with water (2ml). Organics were filtered through a hydrophobic frit and solvent was evaporated to afford crude intermediate. Purification by prep. HPLC (basic) followed by evaporation of solvent from the appropriate fractions afforded product. $^1H$ NMR / LCMS indicates product is not sufficiently pure for submission for testing. Purification by flash chromatography (silica column, DCM 0% to 75% gradient of a solution of DCM:MeOH:$NH_4OH$ 90:10:1) followed by evaporation of solvent from the appropriate fractions and drying under vacuum afforded product, 10 mg (23.9%) of 2-fluoro-N-[1-(1H-imidazol-5-ylmethyl)-3,4-dihydro-2H-quinolin-7-yl]-N-isobutyl-benzamide.

Procedure 19 (ESC1002266)

2-fluoro-N-(2-methoxyethyl)-N-[3-(methylamino)phenyl]benzamide (200 mg, 0.66 mmol) and 1H-imidazole-5-carbaldehyde (76.27 mg, 0.79 mmol) were suspended in DCM (2ml) and acetic acid (0.06 ml, 0.99 mmol) was added. The mixture was stirred for 20 minutes then sodium triacetoxyborohydride (280.39 mg, 1.32 mmol) was added and stirring continued at room temperature for 18 hours (LCMS). The reaction mixture was diluted with DCM (30ml) and washed with NaOH (1M aq., 10ml). The aqueous wash was extracted with DCM (10ml) then organics were combined, washed with brine, dried over sodium sulphate, filtered and solvent was evaporated under reduced pressure to afford crude product, 362mg. Purification by flash chromatography (silica column, DCM 0% to 100% gradient of a solution of DCM:MeOH:NH4OH 100:10:1) followed by evaporation of solvent from the appropriate fractions afforded product 0500A as a colourless gum. Not sufficiently pure for submission for testing. Purification by prep. HPLC (basic) followed by evaporation of solvent from the appropriate fractions afforded product 0500B. Analysis confirms product is pure for testing but contains residual MeOH. Further drying under vacuum afforded product as a colourless gum, 180 mg (71.2%) of 2-fluoro-N-[3-[1H-imidazol-4-ylmethyl(methyl)amino]phenyl]-N-(2-methoxyethyl)benzamide.

ESC1002267

Procedure 19 with 2-fluoro-N-(2-isopropoxyethyl)-N-[3-(methylamino)phenyl]benzamide (151 mg, 0.46 mmol), 1H-imidazole-5-carbaldehyde (52.7 mg, 0.55 mmol), acetic acid (0.04 ml, 0.69 mmol) and sodium triacetoxyborohydride (193.72 mg, 0.91 mmol).

Procedure 20 (ESC1002338)

Suzuki Coupling: To a microwave vial was added the aryl bromide, the desired boronic acid the solvents (dioxane+water) and the mixture was bubbled with argon for ~5 min. Then the cat mix first choice was added to the reaction mixture and the vessel was sealed before heating it to 80°C overnight . The vial was let to cool to rt then, plugged on silica

gel eluting it with EtOAc. The solvents were removed via a Genevac then the mixture was purified by HPLC (acidic). The desired fraction were collected and re-purified if necessary. The mixtures were further dried overnight in a Vac oven (4mbar, at 50°C)
ESC1002421 was obtained by SFC chiral separation of ESC1002338.

Procedure 21 (ESC1002334)

N-[2-(dimethylamino)ethyl]-2-fluoro-N-[3-(methylamino)phenyl]benzamide (35 mg, 0.11 mmol) and 1H-imidazole-5-carbaldehyde (12.8 mg, 0.13 mmol) were combined in DCM (2ml) and acetic acid (0.01 ml, 0.17 mmol) was added. The mixture was stirred at room temperature for 20 minutes then sodium triacetoxyborohydride (47.04 mg, 0.22 mmol) was added and stirring was continued for 16 hours (LCMS). Additional 1H-imidazole-5-carbaldehyde (5.33 mg, 0.06 mmol) was added and stirring continued for 24 hours (LCMS). The reaction mixture was diluted with DCM (15ml) and washed with NaOH (1M aq., 10ml). The aqueous wash was extracted with DCM (10ml) then organics were combined, washed with brine, dried over sodium sulphate, filtered and solvent was evaporated under reduced pressure to afford crude product, 42mg. Purification by prep. HPLC (basic, late focussed gradient) followed by evaporation of solvent from the appropriate fractions and drying under vacuum afforded product as a colourless gum, 20 mg (45.6%) of N-[2-(dimethylamino)ethyl]-2-fluoro-N-[3-[1H-imidazol-4-ylmethyl(methyl)amino]phenyl]benzamide.

Procedure 24 (ESC1002399)

N-(cyclopropylmethyl)-4-methyl-N-[3-[methyl-[[1-(2-trimethylsilylethoxymethyl)imidazol-4-yl]methyl]amin o] phenyl]benzamide (27 mg, 0.05 mmol) was dissolved in MeOH (1ml) and THF (1ml) and 5M HCl (1 ml) was added. The reaction was heated in a microwave to 150°C for 30 minutes. The reaction mixture was concentrated under reduced pressure and the resulting brown oil partitioned between DCM and saturated sodium bicarbonate solution. Organics were filtered through a hydrophobic frit and solvent was evaporated under reduced pressure to afford crude product (18mg). Purification by prep. HPLC (basic, late focussed gradient) followed by evaporation of solvent from the appropriate fractions afforded product as a pale yellow gum, 2.6 mg (13%) of N-(cyclopropylmethyl)-N-[3-[1H-imidazol-4-ylmethyl(methyl) amino]phenyl]-4-methyl-benzamide.

Procedure 26 (ESC1002454)

2,4-difluoro-N-isobutyl-N-[3-[methyl-[[1-(2-trimethylsilylethoxymethyl)imidazol-4-yl]methyl]amino]phenyl] benzamide (37 mg, 0.07 mmol) was dissolved in MeOH (1ml) and 5M HCl (0.5 ml) added. The reaction was heated to reflux for 6 hours then stirred at room temperature for 16 hours, overnight. Heating was continued for 5 hours (LCMS) then the reaction mixture was concentrated under reduced pressure. The crude HCl salt was dissolved in DCM (2ml) and washed with saturated sodium bicarbonate solution (2ml). The aqueous wash was re-extracted with DCM (2ml) then organics were combined, filtered through a hydrophobic frit and solvent was evaporated under reduced pressure to afford crude product, 27mg. Purification by prep. HPLC (basic, late focussed gradient method) followed by evaporation of solvent from the appropriate fractions and drying afforded product, 15 mg (53.8%) of 2,4-difluoro-N-[3-[1H-imidazol-4-ylmethyl(methyl) amino]phenyl]-N-isobutyl-benzamide.

ESC1002455

Procedure 26 with 2-fluoro-N-isobutyl-N-[3-[methyl-[[1-(2-trimethylsilylethoxymethyl)imidazol-4-yl]methyl]amino]phenyl]-4-(t rifluoromethyl)benzamide (73%, 43 mg, 0.05 mmol) in place of 2,4-difluoro-N-isobutyl-N-[3-[methyl-[[1-(2-trimethylsilylethoxymethyl)imidazol-4-yl]methyl]amino]phenyl] benzamide. The product was obtained as 13 mg (53.4%) of 2-fluoro-N-[3-[1H-imidazol-4-ylmethyl(methyl)amino]phenyl]-N-isobutyl-4-(trifluoromethyl)benzamide.

ESC1002456

Procedure 26 with 2,6-difluoro-N-isobutyl-N-[3-[methyl-[[1-(2-trimethylsilylethoxymethyl)imidazol-4-yl]methyl] amino]phenyl] benzamide (25 mg, 0.05 mmol) in place of 2,4-difluoro-N-isobutyl-N-[3-[methyl-[[1-(2-trimethylsilylethoxymethyl)imidazol-4-yl]methyl]amino]phenyl] benzamide. The product was obtained as 8 mg (42.5%) of 2,6-difluoro-N-[3-[1H-imidazol-4-ylmethyl(methyl)amino]phenyl]-N-isobutyl-benzamide.

ESC1002457

Procedure 26 with 2-chloro-N-isobutyl-N-[3-[methyl-[[1-(2-trimethylsilylethoxymethyl)imidazol-4-yl]methyl]amino]phenyl]ben zamide (86%, 33 mg, 0.05 mmol) in place of 2,4-difluoro-N-isobutyl-N-[3-[methyl-[[1-(2-trimethylsilylethoxymethyl)imidazol-4-yl]methyl]amino]phenyl] benzamide. The product was obtained as 9 mg (42.1%) of 2-chloro-N-[3-[1H-imidazol-4-ylmethyl(methyl)amino]phenyl]-N-isobutyl-benzamide.

ESC1002459

Procedure 26 with 4-ethyl-N-isobutyl-N-[3-[methyl-[[1-(2-trimethylsilylethoxymethyl)imidazol-4-yl]methyl]amino]phenyl]benzamide (83%, 34 mg, 0.05 mmol) in place of 2,4-difluoro-N-isobutyl-N-[3-[methyl-[[1-(2-trimethylsilylethoxymethyl)imidazol-4-yl]methyl]amino]phenyl] benzamide. The product was obtained as 13.5 mg (63.8%) of 4-ethyl-N-[3-[1H-imidazol-4-ylmethyl(methyl)amino]phenyl]-N-isobutyl-benzamide.

ESC1002460

Procedure 26 with 5-chloro-N-isobutyl-N-[3-[methyl-[[1-(2-trimethylsilylethoxymethyl)imidazol-4-yl]methyl]amino]phenyl]thio phene-2-carboxamide (82%, 35 mg, 0.05 mmol) in place of 2,4-difluoro-N-isobutyl-N-[3-[methyl-[[1-(2-trimethylsilylethoxymethyl)imidazol-4-yl]methyl]amino]phenyl] benzamide. The product was obtained as 16.2 mg (74.7%) of 5-chloro-N-[3-[1H-imidazol-4-ylmethyl(methyl)amino]phenyl]-N-isobutyl-thiophene-2-carboxamide.

Procedure 29 (ESC1002164 to ESC1002460)

Suzuki cross coupling method. To a vial microwave vial were added the aryl bromide, the desired boronic acid the solvents (dioxane+water) and then the mixture was bubbled with argon for ~5 min. Cat mix first choice was added to the reaction mixture and the vessel was sealed before heating it to 80°C for 16h. The mixture was allowed to cool to rt then diluted with EtOAc and Water and extracted with EtOAc (3x 15ml). The org. layers were filtered through celite, dried over Na2SO4, reduced. To the crude intermediate was added TFA (17.1 mg, 0.15 mmol) and Dichloromethane (2 ml) and the mixture was stirred at rt for 3h. The mixtures were reduced (Genevac) then purified by HPLC prep. acidic (except reaction 4). the desired fractions were collected and reduced before flushing them through a 10g silica cartridge on an Isolera using Hep:EtOAc (0-100%). the desired fractions were collected, reduced and dried over Geneva and vac oven (3mbar at 52°C).

ESC1002491

Procedure 26 with N-isobutyl-2-methyl-N-[3-[methyl-[[1-(2-trimethylsilylethoxymethyl)imidazol-4-yl]methyl] amino]phenyl]benzamide (19 mg, 0.04 mmol) in place of 2,4-difluoro-N-isobutyl-N-[3-[methyl-[[1-(2-trimethylsilylethoxymethyl)imidazol-4-yl]methyl]amino]phenyl] benzamide. The product was obtained as 6.1 mg (43.2%) of N-[3-[1H-imidazol-4-ylmethyl(methyl)amino]phenyl]-N-isobutyl-2-methyl-benzamide.

ESC1002492

Procedure 26 with N-isobutyl-2,4-dimethyl-N-[3-[methyl-[[1-(2-trimethylsilylethoxymethyl)imidazol-4-yl]methyl] amino]phenyl] benzamide (13 mg, 0.02 mmol) in place of 2,4-difluoro-N-isobutyl-N-[3-[methyl-[[1-(2-trimethylsilylethoxymethyl)imidazol-4-yl]methyl]amino]phenyl] benzamide. The product was obtained as 3.9 mg (40%) of N-[3-[1H-imidazol-4-ylmethyl(methyl)amino]phenyl]-N-isobutyl-2,4-dimethyl-benzamide.

ESC1002493

Procedure 26 with N-isobutyl-N-[3-[methyl-[[1-(2-trimethylsilylethoxymethyl)imidazol-4-yl]methyl]amino]phenyl]-2-(trifluorom ethyl)benzamide (24 mg, 0.04 mmol) in place of 2,4-difluoro-N-isobutyl-N-[3-[methyl-[[1-(2-trimethylsilylethoxymethyl)imidazol-4-yl]methyl]amino]phenyl] benzamide. The product was obtained as 4.6 mg (25%) of N-[3-[1 H-imidazol 4-ylmethyl(methyl)amino]phenyl]-N-isobutyl-2-(trifluoromethyl)benzamide.

Procedure 30 (ESC1002597)

Amine and the desired halogen was added to a flask, and the mixture was diluted in tetrahydrofuran (2 ml) and TEA (0.07 ml, 0.5 mmol) and the mixture was heated to 70°C overnight. LCMS revealed >50% conversion. The mixture was diluted in EtOAc and sat $NaHCO_3$ solution then extracted with EtOAc (3*3ml). The org. phases were dried over $Na_2SO_4$ and reduced before being charged on a 10g silica cartridge and purified on an Isolera (Hep:EtOAc 0-50%). The purification was repeated. The compound was further purified on HPLC and the desired fractions were dried. The paste was slated using 0.1ml of 4M HCl in Dioxane, and the compound was dried before submission (Genevac and vac oven

3mbar at 52°C).

ESC1002516, reference compound not of the invention claimed herein

Procedure 26 with 2-fluoro-N-[(5-methylisoxazol-3-yl)methyl]-N-[3-[methyl-[[1-(2-trimethylsilylethoxymethyl) imidazol-4-yl]m ethyl]amino]phenyl]benzamide (20 mg, 0.04 mmol) in place of 2,4-difluoro-N-isobutyl-N-[3-[methyl-[[1-(2-trimethylsilylethoxymethyl)imidazol-4-yl]methyl]amino]phenyl] benzamide. Purification by prep. HPLC (acidic method) and SCX, product eluted with 2M methanolic ammonia followed by evaporation of solvent and drying afforded product as 5 mg (32.8%) of 2-fluoro-N-[3-[1H-imidazol-4-ylmethyl(methyl)amino]phenyl]-N-[(5-methylisoxazol-3-yl)methyl]benzamide. Trace MeOH impurity but sufficiently pure for submission for testing.

Procedure 32 (ESC1002575)

2-fluoro-N-isobutyl-4-methyl-N-[3-[methyl-[[1-(2-trimethylsilylethoxymethyl)imidazol-4-yl]methyl]amino]phenyl]benzamide (39 mg, 0.07 mmol) was dissolved in MeOH (1ml) and 5M HCl (0.5 ml) added. The solution was heated to 70°C for 18 hours. The reaction mixture was basified with addition of NaOH (2M aq.) and extracted with DCM (2 x 25ml). Organics were combined, dried over sodium sulphate, filtered and solvent was evaporated under reduced pressure to afford crude product, 30mg. LCMS indicated recovered starting material (suggests HCl also evaporated during reaction). The recovered material was dissolved in MeOH (1ml) and HCl (5M, 0.5ml) added. The reaction was heated to 70°C for 18 hours (LCMS) then concentrated in a genevac. The resulting residue was dissolved in DCM (2ml) and washed with saturated sodium bicarbonate solution (2ml). The wash was re-extracted with DCM (2ml) then organics were combined, filtered through a hydrophobic frit and solvent was evaporated in a genevac. The crude residue was purified by prep. HPLC (basic, late focussed gradient) followed by evaporation of solvent and drying to afford product, 11 mg (37.5%) of 2-fluoro-N-[3-[1H-imidazol-4-ylmethyl(methyl)amino]phenyl]-N-isobutyl-4-methyl-benzam

ESC1002614

Procedure 26 with N-isobutyl-N-[3-[methyl-[[1-(2-trimethylsilylethoxymethyl)imidazol-4-yl]methyl]amino]phenyl]-2-oxo-1H-p yridine-3-carboxamide (7 mg, 0.01 mmol) in place of 2,4-difluoro-N-isobutyl-N-[3-[methyl-[[1-(2-trimethylsilylethoxymethyl)imidazol-4-yl]methyl]amino]phenyl] benzamide. Product obtained as 1.2 mg (23%) of N-[3-[1H-imidazol-4-ylmethyl(methyl)amino]phenyl]-N-isobutyl-2-oxo-1H-pyridine-3-carboxamide.

Procedure 33 (ESC1002656)

N-isobutyl-N-[3-[methyl-[[1-(2-trimethylsilylethoxymethyl)imidazol-4-yl]methyl]amino]phenyl]pyrazine-2-carboxamide (54 mg, 0.11 mmol) was dissolved in TBAF (1M in THF, 2.5 ml) and the reaction heated to 120°C in a microwave for 60 minutes (LCMS). The reaction mixture was concentrated under reduced pressure and the afforded residue partitioned between DCM (10ml) and 10% citric acid solution (10ml). The acid solution was separated (organics discarded) and made basic with addition of NaOH (2M aq.) then extracted with DCM (2x10ml). Organics were combined, filtered through a hydrophobic frit and solvent was evaporated under reduced pressure to afford crude product, 95mg. Purification by prep. HPLC afforded the desired product. The product was purified by flash chromatography (silica column, elution with isocratic 90:10:1 DCM:MeOH:$NH_4OH$). Evaporation of solvent from the appropriate fractions and drying afforded desired product as a pale yellow solid, 18 mg (45.2%) of N-[3-[1H-imidazol-4-ylmethyl(methyl)amino]phenyl]-N-isobutyl-pyrazine-2-carboxamide.

Procedure 36 (ESC1002762)

To a solution of the appropriate N-Methylaniline analogue in toluene is added Cl-$CH_2$CN followed by $K_2CO_3$.The mixture is heated at reflux overnight. After cooling to RT The mixture is diluted with DCM and filtered. The filter residue is washed with DCM and the combined filtrates are evap. i.vac. The residual material is prepurified on a 5g Si-SPE column: Rf = 0.2 in heptane/Me$CO_2$Et = 2/1 (unreacted starting material elutes slightly before with an Rf = 0.3). Final purification is carried out on basic prepHPLC where the desired product elutes between 8-8.4min.

Procedure 37 (ESC1002764), reference compound not of the invention claimed herein 2-fluoro-{N}-[3-(1,2,4-triazol-4-yl)phenyl]benzamide (90.0mg, 0.319mmol) was dissolved in DMF (1ml). $Cs_2CO_3$ (0.312g, 0.957mmol), NaI (0.00956g, 0.0638mmol) and 1-bromo-2-methyl-propane (0.104ml) 0.957mmol) were added to a vial. The vial was sealed and heated to 80°C for 3 hours. LCMS indicated that product was forming. Further 1-bromo-2-methyl-propane (0.208ml) was added and the reaction heated for a further 3 hours. The reaction was diluted with ethyl acetate and water. The organic was washed with water, brine, dried over Na2SO4 and the solvent removed at reduced pressure. The resulting residue

was purified by flash chromatography (5g Biotage zip) eluting with a gradient 0 - 5% methanol in DCM. The appropriate fractions were combined, and the solvent removed at reduced pressure to afford product which was found to be impure. Product was further purified by acidic prep HPLC. The appropriate fractions were combined and the solvent removed in a Genevac to afford recovered starting amide and desired product.

Procedure 38 (ESC1002765), reference compound not of the invention claimed herein 2-fluoro-N-(3-imidazol-1-ylphenyl)benzamide (60 mg, 0.21 mmol) was stirred in DMF (1 mL). NaH disp. in mineral oil (60%, 12.8 mg, 0.32 mmol) was added. The reaction was placed under an argon atmosphere and stirred for 10 minutes. 1-bromo-2-methylpropane (46.39 μl, 0.43 mmol) was added and the reaction heated to 60°C for 1 hour. Further 1-bromo-2-methyl-propane (46.39 μl, 0.43 mmol) was added and the reaction heated for a further 30 minutes. The reaction was diluted with ethyl acetate and quenched with water. The organic layer washed with water (x3), brine, dried over $Na_2SO_4$ and concentrated at reduced pressure. The resulting residue was purified by flash chromatography (5g Biotage SiO2 zip) elutiing with a gradient 0 - 95:5:05 DCM:MeOH:$NH_4OH$. The appropriate fractions were combined and the solvent removed at reduced pressure to afford product.

Procedure 40 (ESC1002802)

2-fluoro-N-isobutyl-N-(5-(methylamino)pyridin-3-yl)benzamide (5 mg, 0.02 mmol) and 4-(chloromethyl)-4H-imidazole (0.01 g, 0.03 mmol) were dissolved in MeTHF. DIPEA (0.02 ml, 0.15 mmol) was added and the reaction mixture was allowed to stir at room temperature for 1 hour. The reaction mixture was heated to 70 °C and allowed to stir for three hours. MeCN (2 mL) was added to the reaction mixture which was allowed to stir at room temp for 2 hours. The reaction mixture was partitioned between water and EtOAc and the phases were separated. The aqueous layer was extracted with EtOAc and the combined organic layers were dried ($Na_2SO_4$), filtered and concentrated in vacuo giving 30 mg of residue. The mixture was purified by HPLC (acidic prep, rT = 5.22 min), the relevant fractions were collected and concentrated in vacuo giving <5 mg of product. The aqueous layer (pH 6-7) was basified to pH 9 with 2M NaOH (aq.) and extracted with EtOAc. The aqueous layer was further basified to pH 14 and extracted with EtOAc. The aqueous layer was concentrated in vacuo giving a solid residue. This was extracted with MeOH, filtered and concentrated in vacuo. The resulting residue was purified by HPLC (acidic prep, rT = 5.22 min), and the relevant fractions were concentrated in vacuo. This material was combined with the material from earlier HPLC runs, the combined material was dried at 50 °C for 18 h in vacuo giving a white solid (17 mg, 21%). NMR experiments indicated that this material exists as rotamers. High temperature NMR experiments confirmed that the structure was correct.

Procedure 41 (ESC1002804)

(R)-2-(3'-methoxy-[1,1'-biphenyl]-2-yl)pyrrolidine (330 mg, 1.3 mmol) and 3-chlorothiophene-2-carboxylic acid (0.42 g, 2.61 mmol) were dissolved in MeTHF (5 mL). DIPEA (0.53 ml, 3.91 mmol) and HATU (0.49 g, 1.3 mmol) were added to the reaction mixture which was allowed to stir at room temperature for 18 hours. The reaction mixture was diluted with water and EtOAc and the phases were separated. The aqueous phase was extracted with EtOAc (x2) and the combined organic layer was dried ($Na_2SO_4$), filtered and concentrated in vacuo onto silica gel. The material was purified by flash column chromatography (0-40% EtOAc in n-heptanes). Giving material with minor impurities (270 mg, 52%). An alliquot of material was purified further by HPLC (basic prep, rT = 9.33 min), the relevant fractions were collected and concentrated in vacuo. The material was dried at 50 °C in vacuo for 18 h giving the desired compound as a white solid.

ESC1002817

Procedure 37 with N-isobutyl-4-methyl-N-[4-(methylamino)pyrimidin-2-yl]benzamide (25 mg, 0.08 mmol), 4-(chloromethyl)-1H-imidazole;hydrochloride (25.64 mg, 0.17 mmol), $Cs_2CO_3$ (40.95 mg, 0.13 mmol) and NaI (0.31 mg, 0 mmol). Heating was continued over the weekend. An additional 1.5eq of base was added and heating continued. Additional base (3eq) and chloride (2eq) were added and heating continued overnight. The crude mix was filtered and purified on basic HPLC and the fraction corresponding to the product (by LCMS) was collected and concentrated in the genevac. NMR and LCMS suggest material is consistent with desired product and of sufficient purity for testing (though small baseline impurities are visible in the NMR). ~ 5mg obtained.

**Protein expression and purification for crystallography**

Human 17β-HSD10 (residues 1-261) was cloned into pNIC-CTHF vector with a TEV-cleavable C-terminal hexa-histidine tag and Flag tag. After transformation into *E. coli* (BL21(DE3) pRARE2), expression was performed in TB auto induction medium (FroMedium), supplemented with 20 g/L glycerol, anti-foam, 50 μg/mL kanamycin and 34 μg/mL chloramphenicol. Cultures were grown for four hours at 37 °C, then the temperature was dropped to 18 °C and the cultures

were grown for another 42 hours. Cells were spun at 4,000 x g for 20 minutes, and after discarding the supernatant, frozen for 2 hours. After thawing, the cell pellets were resuspended in a buffer (10 mM HEPES, 5% Glycerol, 500 mM NaCl, 0.5 mM TCEP, pH 7.5) supplemented with 0.5 mg/mL lysozyme and 1 μg/mL benzonase, vortexed and incubated at room temperature for 30 mins. 2% Triton X-100 was added and the cells were frozen overnight at -80°C. On thawing, the cells were supplemented with 10 mM imidazole and stirred for 1 hour at room temperature. Cells were then centrifuged for one hour at 4,000 × g and the supernatant applied to Hi GraviTrap column (GE healthcare) equilibrated with binding buffer (10 mM HEPES, 5 % glycerol, 500 mM NaCl, 0.5 mM TCEP, pH 7.5). The column was washed twice with the binding buffer supplemented with 10 mM imidazole. ABAD was eluted with the binding buffer supplemented with 300 mM imidazole. The eluted protein was applied to a PD-10 desalting column (GE Healthcare) and eluted with binding buffer supplemented with 10 mM imidazole. The C-terminal affinity tag was removed by TEV cleavage overnight and uncleaved protein was removed by reverse IMAC by applying it again to a His GraviTrap column. The flow-through of ABAD was supplemented with $1/10^{th}$ the volume of 1M Arginine/1M Glutamine mix (pH 7.5) and was concentrated and purified further by size exclusion chromatography using a YARRA SEC-2000 PREP column (Phenomenex), equilibrated with binding buffer. Fractions containing ABAD were pooled, concentrated and stored at -80 °C

**Crystallisation**

0.45 mM of 17β-HSD10 was incubated with 5 mM each of NADH and ESC1002033 compound at room temperature for 10 minutes. Sitting drop crystallization plate with 150 nL drop volume was setup with Hampton index screen (Hampton Research) at 20 °C. Co-crystals were obtained in condition containing 5 mM magnesium chloride, 5 mM cobalt chloride, 5 mM nickel chloride, 5 mM cadmium chloride, 12% PEG3350 and 0.1M HEPES, pH 7.5. All crystals were harvested with 20% ethylene glycol as cryoprotectant and flash cooled in liquid nitrogen.

**Synthesis of Compounds S2-Tn**

**LCMS Analytical Methods**

*Analytical Method A (LCMS monitoring)*

The HPLC measurement was performed using Waters Acquity H Class UPLC comprising a quaternary pump with degasser, a sample manager, a column oven (set at 50° C), a diode-array detector DAD and a column as specified in the respective methods below. Flow from the column was split to a MS spectrometer. The MS detector (Waters SQ Detector 2) was configured with an electrospray ionization source. Mass spectra were acquired by scanning from 160 to 1200 in 0.20 second. The capillary needle voltage was 3.50 kV in positive and negative ionization mode and the source temperature was maintained at 150 °C. Nitrogen was used as the desolvation gas, the flow was 750 L/Hour. Data acquisition was performed with Mass Lynx 4.2 Software. Reversed phase HPLC was carried out on a Waters Xbridge C18 column (3.5 μm, 50 x 3 mm) with a flow rate of 1.20 ml/min. Two mobile phases were used, mobile phase A: 5 Mm $NH_4CH_3CO_2$ in water; mobile phase B: 5 Mm $NH_4CH_3CO_2$ in acetonitrile (ACN): water (90:10)], and they were employed to run a gradient conditions from 5 % B for 0.75 minutes, from 5 % to 30 % in 1.25 minutes, and from 30 % to 98 % in 1.75 minutes, 98 % B for 2.25 minutes and 5 % B in 2.75 minutes and hold these conditions for 3.25 minutes in order to re-equilibrate the column (Total Run Time 3.25 minutes). An injection volume of 0.5 μl was used.The sample was monitored at 260 nm & 220 nm.

*Analytical Method B (LCMS final)*

The HPLC measurement was performed using Waters Acquity H Class UPLC comprising a quaternary pump with degasser, a sample manager, a column oven (set at 50° C), a diode-array detector DAD and a column as specified in the respective methods below. Flow from the column was split to a MS spectrometer. The MS detector (Waters SQ Detector 2) was configured with an electrospray ionization source. Mass spectra were acquired by scanning from 160 to 1200 in 0.20 seconds. The capillary needle voltage was 3.50 kV in positive and negative ionization mode and the source temperature was maintained at 150 °C. Nitrogen was used as the desolvation gas, the flow was 750 L/Hour. Data acquisition was performed with Mass Lynx 4.2 Software. Reversed phase HPLC was carried out on a Waters Acquity BEH C8 column (1.7 μm, 50 x 2.1 mm) with a flow rate of 0.800 ml/minute. Two mobile phases were used, mobile phase A: 0.05% HCOOH in water; mobile phase B: 0.05% HCOOH in ACN: water (90:10)], and they were employed to run at gradient conditions from 5 % B for 0.75 minutes, from 5 % to 25 % in 0.75 minutes, and from 25 % to 95 % in 1.50 minutes, 95 % B for 1.00 minutes and 5 % B in 0.50 minutes and hold these conditions for 0.60 minutes in order to re-equilibrate the column (Total Run Time 5.10 minutes). An injection volume of 0.5 μl was used. The sample was monitored at 260 nm & 220 nm.

**Preparative HPLC Method**

Preparative HPLC was done on a Waters auto purification instrument. Column name: YMC-Actus Triat (250 x 20 mm, 5μ) operating at ambient temperature and flow rate of 16 mL/minute. Mobile phase: A = 20 mM Ammonium bicarbonate in water, B=Acetonitrile; Gradient Profile: Mobile phase initial composition of 80% A and 20% B, then 70% A and 30% B in 3 minutes, then to 50% A and 50% B in 20 min., then to 5% A and 95% B in 21 minutes, held this composition up to 22 minutes. for column washing, then returned to initial composition in 23 minutes. and held till 25 minutes. UV detection e.g. 254 nM is used for the collection of fractions from HPLC. This description gives general method and variations in types of equipment, columns, mobile phase, detection wavelength, solvent gradient and run time also used to purify compounds.

**Preparative TLC Method**

In preparative TLC ,Silica gel 60 F254 analytical Prep thin layer chromatography (Prep-TLC) plates from Merck (Darmstadt, Germany) were used & crude material was run under a developed solvent system (30%-50% ethyl acetate hexane) separated are often applied as long streaks, rather than spots, in the sample application zone. After visualization under UV light, specific components were recovered by scraping the sorbent layer from the plate in the region of interest and eluting the separated material from the sorbent using a strong solvent (10% MeOH-DCM).

**Results and Discussion**

Around 350,000 drug-like molecules were screened through a primary screen assay to identify any hits which would inhibit 17β-HSD10. After further validation using orthogonal assays, dose response assays and assessing physicochemical properties a cluster of five molecules featuring an imidazole benzamide as the key structural feature and a singleton molecule were identified as starting points for obtaining lead molecules.

Further analogues were synthesised and tested through various screening assays. Primary screening results are shown in Table 1. Further analogue series were synthesised focussing on optimising each area of the molecule to increase target engagement, potency and simultaneously improving the physicochemical properties.

**Table 1:** Primary screening $pIC_{50}$ values for the resynthesized HTS hit compounds.

| Compound Origin | Structure | Compound identifier | ABAD $pIC_{50}$ |
|---|---|---|---|
| Cluster | 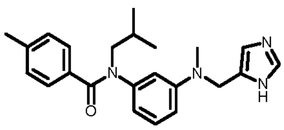 | ESC1002032 | 6.6 |
| Cluster | 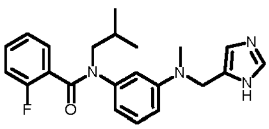 | ESC1002033 | 6.7 |
| Cluster | 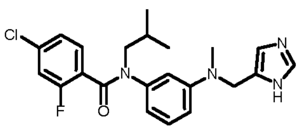 | ESC1002042 | 6.5 |
| Cluster | 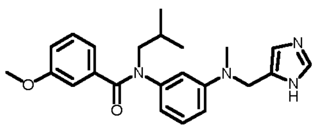 | ESC1002038 | 5.7 |
| Cluster | 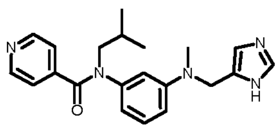 | ESC1002034 | 5.8 |

The physicochemical properties of the resynthesized hits are detailed in Table 2. The structures of the

compounds may be found in Table 6. Overall the compounds are lead-like, although the lipohilicities of some examples are higher than optimal. The physicochemical properties lie within the idealised limits required for brain exposure (see Lipinski, C. A., Lombardo, F. and Dominy, B. W., Adv. Drug Deliv. Rev. 1997, 23, 3-25..

**Table 2:** Physicochemical properties for the resynthesized HTS compounds. Where MW= molecular weight (Da), ALogP= atom additive partition coefficient, LogD= distribution coefficient, HBA= hydrogen bond acceptor, HBD= hydrogen bond donor, LipE=lipophilic efficiency.

| Identifier | MW | ALogP | LogD | TPSA | HBA | HBD | LipE |
|---|---|---|---|---|---|---|---|
| Optimal | <500 | <5 | <5 | <90 | <7 | <3 | pIC50-LogP |
| ESC1002033 | 380 | 3.93 | 4.37 | 52 | 3 | 1 | 2.33 |
| ESC1002032 | 376 | 4.21 | 4.65 | 52 | 3 | 1 | 1.95 |
| ESC1002042 | 415 | 4.6 | 5.04 | 52 | 3 | 1 | 1.46 |
| ESC1002034 | 363 | 2.58 | 3.02 | 65 | 4 | 1 | 2.78 |
| ESC1002038 | 392 | 3.71 | 4.15 | 61 | 4 | 1 | 1.55 |

**Cluster chemical modifications**

The cluster analogues identified by the primary screen were altered on the amide side of the molecule and initial structure activity relationships (SAR) within the hits suggested that both *ortho* and/or *para* substitution with an electron withdrawing group was favourable for potency. Initial analogues focussed on exploring this region further with a small number of additional aryl amides. As well as these changes, initial SAR exploration around the other parts of the hit were also planned to establish some initial SAR around these regions. Chemical alterations are shown in Figure 1.

Utilising the primary screen and dose response assays, results indicated that the replacement of the iso-propylmethyl results in some loss of potency with relatively subtle changes (Table 6). As well as these subtle changes, SAR would suggest that heteroatoms e.g. ESC1002334 and heteroaryl rings reduce potency e.g. ESC1002516.

Expansion of the SAR around the initial hits indicate that *ortho* and *para* substitution of the terminal phenyl ring (e.g. fluorophenyl of Figure 1) with both electron donating and withdrawing groups is well tolerated. Di-substitution was also well tolerated and may give rise to slightly improved inhibition of ABAD. Replacement of the terminal phenyl ring with an alkyl was also investigated. Although replacement with cyclohexyl (compound ESC1002166) resulted in a less potent compound, this compound may have improved physicochemical and drug metabolism and pharmacokinetic properties. Extending the terminal phenyl ring outward with insertion of methylenes gave rise to losses in potency. The box plot in Figure 2 summarises the compound type versus 17β-HSD10 activity.

It was also established that the appropriate positioning of a H-bond donor effects 17β-HSD10 activity, *c.f.* ESC1002199 and ESC1002033, and ESC1002205 and ESC1002206 (Table 6). Furthermore, comparing the imidazole/pyrazole pair, ESC1002033 and ESC1002205, suggests that an H-bond acceptor (likely to be protonatable) also effects activity. When comparing ESC1002033 and ESC1002208, the thiophene and pyrazole will be un-ionised at physiological pH. In addition, there is some space around the terminal imidazole group (see Figure 1) for further functionalisation with both methyl substituted compounds, ESC1002203 and ESC1002204 having only slightly reduced ABAD inhibition levels in comparison to ESC1002033. Expansion of the terminal imidazole to a 6-membered ring is also tolerated. It was also noted that when conformational constraints were implemented to lock the molecule into its bioactive conformation (see Figure 1 and compound ESC1002081 of Table 6), that potency was abolished.

Following the solving of the crystal structure of ESC1002033 within 17β-HSD10 (discussed in more detail later), key interactions between 17β-HSD10 and ESC1002033 were found to include an H-bond from the imidazole to GLN162 and possibly also a H-bond to a well-ordered water molecule (Figure 3a). In addition, an edge to face interaction between the central aryl ring and TYR168 of the catalytic triad is evident. With the crystal structure available, new opportunities presented themselves to develop further cluster analogues with increased potency. Specifically, a further set of analogues were prepared that maintained a H-bond donor in an equivalent position or increased the acidity of the donor itself.

The crystal structure revealed a small hydrophobic pocket close to the imidazole with a well-ordered water molecule present (Figure 3b). There was therefore an opportunity to fill the pocket and potentially displace the water molecule to gain potency. In the initial heterocyclic replacement set, the 2-methylsubstituted imidazole, ESC1002203 had already been prepared and was approximately 3-fold less potent than ESC1002033 (Table 6). Increasing the size of the 2-substituent to an *iso*-propyl, ESC1002633, resulted in further losses in potency. The slightly smaller and more $sp^2$-like cyclo-propyl was better tolerated than its iso-propyl congener (Table 6).

The crystal structure of ESC1002033 was superimposed with another crystal structure. At the time of experimentation, the only other reported crystal structure of a ligand bound to 17β-HSD10 is AG18051 (C. R. Kissinger et al., J. Mol. Biol., 2004, 342(3), 943-952). This ligand forms a covalent adduct with NADH, catalysed by 17β-HSD10, resulting in a highly potent inhibitor of the protein. Key interactions formed by the protein-ligand complex are a H-bond donor to TYR168 and a H-bond to GLN162. Superposition of ESC1002033 onto the AG18051 crystal structure (Figure 4) indicates that the TYR168 H-bond is not engaged by the ESC1002033 ligand. Analogues were prepared to try and exploit this interaction.

Attempts to target the H-bond of TYR168 resulted in losses in potency. Interestingly, the unsubstituted imidazole analogue ESC1002631, which was prepared as an intermediate, illustrates the importance of methyl substitution at imidazole (Table 6).

A potentially key interaction identified in the ligand bound crystal structure of ESC1002033 was the edge-to-face π-interaction between TYR168 and the central aryl ring (Figure 5). This interaction can be strengthened by making the interacting hydrogen atom(s) more acidic. Therefore, a set of heteroaromatic replacements were prepared which, due to their electronic nature, should increase the acidity of the interacting hydrogen. However, no improvement in potency was observed.

The last point of inquiry within this series was a potential H-bond interaction with the backbone carbonyl of LEU205. As detailed earlier in the report, an extensive array of both substituents and substitution patterns had already been prepared and tested. The overall conclusion from that work was that both 2-substitution and 2,6-disubstitution resulted in improved potencies, the original QHL hit ESC1002033 and ESC1002456 were identified as the most potent inhibitors within the series. Fluorine is a strongly electronegative group and will polarize the adjacent proton on the aromatic ring, making it more acidic. This would then be set-up for a weak H-bond between the Aryl-H and the carbonyl of LEU205 and indeed the distance between the Aryl-H and the carbonyl is consistent with a weak H-bond. To explore this further several analogues were prepared that either placed a H-bond donor in this position or a polarised hydrogen of a heterocycle. However, these modifications did not improve potency.

**Cluster chemical modification summary**

To summarise, an extensive range of analogues were prepared and tested to explore the SAR around the cluster hit series. In some cases, it was found that replacement of key features in ESC1002033 resulted in potency loses.

**Thermal shift analysis Results**

Results of a thermal shift assay (TSA) showed that some of the QHL compounds stabilised 17β-HSD10 in the presence of NADH, providing strong evidence of target engagement. The ΔTm for one compound from each series is presented in Figure 9 with a representative derivative plot of ESC1002033 and ESC1002082 (not of the invention claimed herein) in Figure 9a. A $\Delta T_m$ was analysed for each compound at 20 μM compound concentration and 1 mM NADH and Figure 9b shows the correlation between $pIC_{50}$, the cellular assay and $\Delta T_m$. None of the compounds could improve on the $\Delta T_m$= 6.75 °C exhibited by compound ESC1002033 with most $\Delta T_m$ shifts around 1-4 °C, therefore still displaying good target engagement. However, some of the compounds showed a degree of autofluorescence and as such are unsuitable for analysis using this technique.

**Mechanism of Action results**

Two compounds, ESC1002033 and ESC1002082 (not of the invention claimed herein), representative of the two different clusters were investigated for their mechanism of action against the substrates, NADH and AcetoAcetyl CoA (AcAc). The ESC inhibitors were tested as 12-point 1 in 1.5 dilution series with a maximal concentration of 2.5 μM. When investigating the effect of NADH concentration upon inhibition of 17β-HSD10 by the inhibitors AcAc was kept in excess at 800 μM and NADH diluted down from 150 μM. When investigating the effect of AcAc it was diluted from 1000 μM with an excess of 100 μM NADH.

Both inhibitors appear to have a similar mechanism of action. For NADH at increasing concentration of inhibitor, a decrease in Vmax is observed with no change in Km. This is characteristic of non-competitive(mixed) inhibition and is reflected by convergence of the data to the left of the y axis in a Lineweaver-Burke plot. In contrast, for AcAc, an increase in $Km_{app}$ at increasing inhibitor concentrations is observed, which is characteristic of competitive inhibition. This was supported by a Lineweaver-Burke plot. In conclusion, these data sugges that both ESC1002033 and ESC1002082 bind non-competitively with NADH but competitively with AcAc.

**Cell Viability Testing and Cytotoxicity Assays**

The effect upon cellular proliferation was tested for all compounds in a liver HepG2 cell line by using an Invitrogen™ CyQUANT™ Cell proliferation assay and a resazurin-resorufin metabolic assay. Two cytotoxicity assays,

CyQuant assay and CellVi assay were used on HepG2 cells to provide a measure of cytotoxicity and identify cytotoxic and cytostatic compounds. Overall, there were no issues identified with most compounds having no measurable $EC_{50}$. Lactate dehydrogenase assays (LDH) and Alamar blue assays in HEK293 cells were also used to probe cytotoxicity and cell viability, however no real cytotoxicity was measured with all compounds showing <15 % cytotoxicity (see Table 6).

**Enzyme Activity Cell Based Assay**

A selection of the most potent compounds were profiled in HEK 293 cells (human embryonic kidney) utilising a fluorogenic probe -(-)CHANA (Muirhead et al. 2010 REF: (-)-CHANA, a Fluorogenic Probe for Detecting Amyloid Binding Alcohol Dehydrogenase HSD10 Activity in Living Cells Kirsty E. A. Muirhead, Mary Froemming, Xiaoguang Li, Kamil Musilek, Stuart J. Conway, Dalibor Sames, and Frank J. Gunn-Moore ACS Chemical Biology 2010 5 (12), 1105-1114DOI: 10.1021/cb100199m) to monitor the oxidative activity of 17β-HSD10 in living cells. Cellular profiling of the compounds indicated a good correlation between the biochemical and cellular assays with the results of most compounds falling within 10-fold of the results of the biochemical assay. This indicates that cellular drop off is not an issue. Furthermore, several of the compounds were found to be very potent inhibitors of cellular 17β-HSD10, having $EC_{50}$ of <100 nM. ESC1002755, the most potent, had an $EC_{50}$ of 28 nM (see Figure 10).

**Crystallography and Molecular Modelling**

Crystal structures of representatives were obtained. The X-ray data for all complexes was of high quality with well-defined electron density for the ligands (see Table 4a, b, c (b and c not of the invention claimed herein)). Molecular replacement was carried out using Phaser and model building/refinement were carries out with CCP4/Coot/Phenix. Refinement statistics for the models are shown below. One representative of each series is discussed in more detail.

Table 4: X-ray crystallography data resolved for three ligand-compound structures (a, b, c).

| **ESC1002033** | |
|---|---|
| Space Group | $P6_1$ |
| Cell Dimensions | 133.25,133.25,133.89,90,90,120 |
| No. Unique Reflections | 86875 |
| Resolution | 66.6-2.03 |
| Rwork/Rfree | 18.0/20.6 |
| Ramachandran (favoured/outlier) | 95.4%/0.3% |
| Bond Length/Angle rmsd | 0.011/1.656 |
| **ESC1002421** (not of the invention claimed herein) | |
| Space Group | $P6_1$ |
| Cell Dimensions | 131.77,131.77,132.84,90,90,120 |
| No. Unique Reflections | 68115 |
| Resolution | 43.3-2.18 |
| Rwork/Rfree | 17.3/20.4 |
| Ramachandran (favoured/outlier) | 95.7%/0.2% |
| Bond Length/Angle rmsd | 0.008/1.202 |
| **ESC1002332** (not of the invention claimed herein) | |
| Space Group | $P6_1$ |
| Cell Dimensions | 131.33,131.33,132.60,90,90,120 |
| No. Unique Reflections | 69438 |
| Resolution | 113.7-2.16 |
| Rwork/Rfree | 17.8/20.9 |
| Ramachandran (favoured/outlier) | 95.9%/0.3% |

(continued)

| | |
|---|---|
| **ESC1002332** (not of the invention claimed herein) | |
| Bond Length/Angle rmsd | 0.007/1.047 |

All ligands showed convincing mFo-DFc difference density and also for the nicotinamide cofactor. The models were built with coot and refined iteratively with phenix and with the CCP4 package to a final Rfactor of below 20% and with reasonable stereochemistry. The ligands bound in the same deep, predominantly hydrophobic pocket and shared some notable binding interactions. Example electron density is shown in Figure 11 with initial electron density mFo-DFc in green on the left (ligand superimposed for reference) and the 2mFo-Dfc density for the fitted ligand and cofactor on the right. Ligand complexes were obtained either by cocrystallisation with ESC1002033 or by cocrystallising then soaking out the first ligand and replacing it with another. There are four molecules in the asymmetric unit and in some cases, the soaking procedure did not replace the original cocrystallisation ligand in all molecules. The relevant ligand was built and refined in each case.

**ESC1002033**

Binding site (left) and surface (right) representations of the ligand are shown in Figure 12. The fluorophenyl ring is almost perpendicular to the plane of the amide bond. Breaking conjugation between these two systems is energetically unfavourable and is likely to be caused by steric effects from the central phenyl ring and the *ortho* fluorine. Placing bulkier substituents here to pre-organise the ligand in the bound conformation resulted in equipotent or marginally more potent compounds. The imidazole ring is at the centre of a hydrogen bond network. This part of the side is deeply buried and will have a low relative permittivity and the effects of electrostatic interactions will be increased as a consequence. Another feature of this pocket is a well-ordered water molecule at the base of a sub-pocket and accessible through a vector from the imidazole ring. The methyl isopropyl group occupies a quite constricted pocket with a small amount of elaboration possible. Some substitution here may be beneficial to crowd the space around the amide group and force the fluorophenyl group into the unfavoured conformation.

Interaction of the ligand with the cofactor was mediated through a methyl group. Investigation of the small molecule database showed that this was quite a common interaction and presumably relies on the delta-positive hydrogen atoms of the methyl interacting with the delocalised electrons of the nicotinamide ring.

**ESC1002332** (not of the invention claimed herein)

The ligand bound in the same site as ESC1002033 (Figure 13a), sharing some interactions but others vary significantly (Figure 13b). A key shared feature is the edge/face interaction of the central aryl ring of the ligand with tyrosine 168 at the base of the active site pocket. The thiophene ring occupies a somewhat more buried space compared with the imidazole ring of ESC1002033 and both molecules have aromatic groups extending towards the solvent. ESC1002332 also forms a direct hydrogen bond to tyrosine 168.

A notable difference in the two complexes is the course of the ligands as they emerge from the binding site towards solvent. Both chemotypes superimpose well at the core phenyl, but diverge as they extend towards solvent.

**ADME Profiling**

Initial mouse hepatocyte stability and plasma protein binding experiments were undertaken on some of the most potent exemplars from the representative series and their results are summarised below in Table 5. The compounds were found to be either moderately or highly metabolised in mouse hepatocytes. The cluster exemplars appear more stable in comparison with the singleton exemplars.

Table 5: Initial mouse hepatocyte stability and plasma protein binding experiments were undertaken on some of the best exemplars from the representative series with AG18051 a previously published 17β-HSD10 inhibitor (C.R. Kissinger et al., J. Mol. Biol., 342 (2004), p. 943; Lim, Y.-A., Grimm, A., Giese, M. PLoS ONE (2011), 6, e28887.)

| | | | | | | |
|---|---|---|---|---|---|---|
| Ref: shown for comparison. Where k= absorption rate constant Cl= clearance, $T_{1/2}$= half-life, Heps= hepatocyte metabolism, PPB= plasma protein binding. | | | | | | |
| **ESC Code** | **Mean ABAD pIC50** | **k** | **Cl ml/minute /g liver** | **$T_{1/2}$ (minutes)** | **Heps Category** | **Mouse PPB (% free)** |
| ESC1002456 | 6.86 | 0.018 | 8.6 | 38.48 | High | 8.9 |
| **ESC Code** | **Mean ABAD pIC50** | **k** | **Cl ml/minute /g liver** | **$T_{1/2}$ (minutes)** | **Heps Category** | **Mouse PPB (% free)** |
| ESC1002033 | 6.7 | | 6.2 | 53.82 | Mod | |
| ESC1002575 | 6.57 | 0.012 | 5.8 | 57.15 | Mod | 7.6 |
| ESC1002204 | 6.4 | 0.11 | 54 | 6.16 | High | |
| ESC1002597 | 6.4 | 0.02 | 7.2 | 46.19 | Mod | |
| (reference, not of the invention claimed herein) | | 0.11 | AG18051 50.5 | 6.58 | High | |

**Table 6 - Compound structures and results of recombinant 17βHSD10 enzyme assays**

| Compound Structure | Compound Reference | Max . Fit | Min. Fit | Max. Effect (%) | Hill Slope | $pEC_{50}$ |
|---|---|---|---|---|---|---|
| 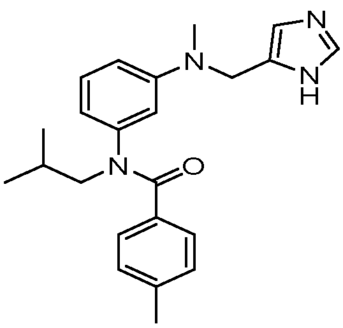 | ESC1002032 | 99 | 0 | 99 | 0.9 | 6.6 |
| 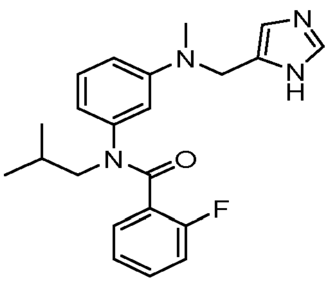 | ESC1002033 | 99 | -3 | 99 | 1 | 6.7 |

(continued)

| Compound Structure | Compound Reference | Max . Fit | Min. Fit | Max. Effect (%) | Hill Slope | $pEC_{50}$ |
|---|---|---|---|---|---|---|
| 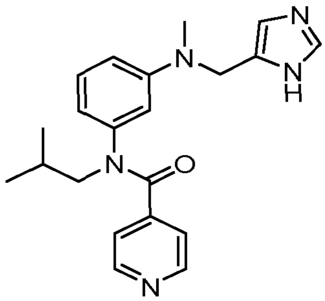 | ESC1002034 | 106 | -3 | 92 | 0.8 | 5.8 |
| 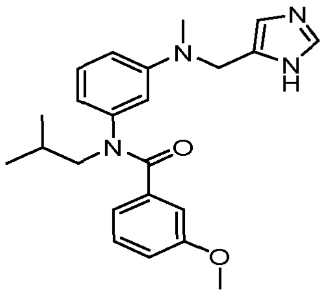 | ESC1002038 | 105 | -2 | 92 | 0.8 | 5.7 |
| 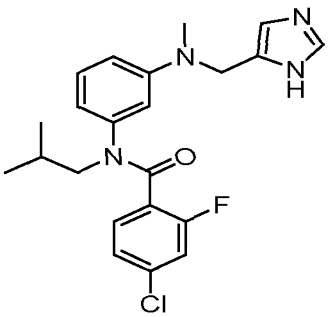 | ESC1002042 | 101 | -6 | 98 | 0.8 | 6.5 |
| 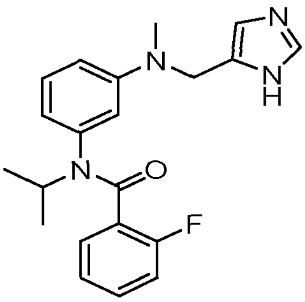 | ESC1002078 | 101 | -1 | 97 | 1 | 6.2 |
| 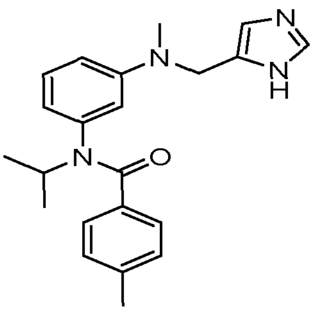 | ESC1002080 | 102 | 0 | 96 | 0.9 | 6 |

(continued)

| Compound Structure | Compound Reference | Max . Fit | Min. Fit | Max. Effect (%) | Hill Slope | $pEC_{50}$ |
|---|---|---|---|---|---|---|
| 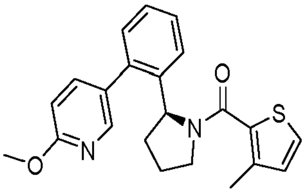 | ESC1002082 (not of the invention claimed herein) | 98 | 0 | 96 | 1.1 | 6.1 |
| 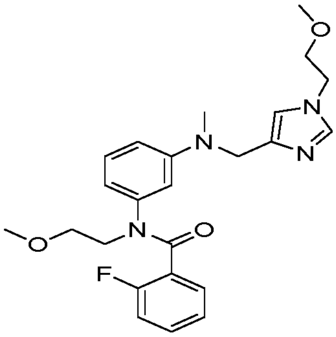 | ESC1002164 | | | 2 | | < 4.7 |
| 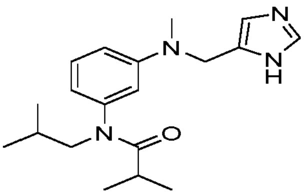 | ESC1002165 | 100 | 0 | 94 | 1 | 5.78 |
| 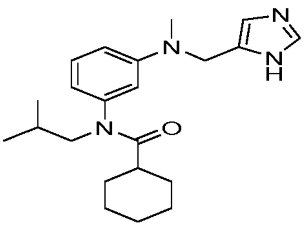 | ESC1002166 | 104 | 0 | 99 | 0.93 | 6.02 |
| 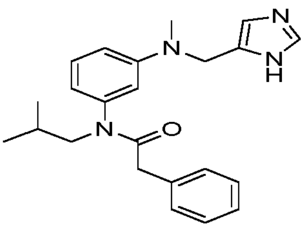 | ESC1002167 | 100 | 0 | 62 | 1 | 4.9 |
| 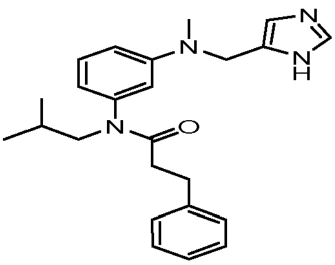 | ESC1002168 | 100 | 0 | 72 | 1.1 | 5.05 |

(continued)

| Compound Structure | Compound Reference | Max . Fit | Min. Fit | Max. Effect (%) | Hill Slope | $pEC_{50}$ |
|---|---|---|---|---|---|---|
| 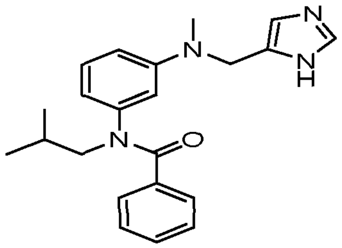 | ESC1002169 | 98 | 0 | 97 | 1.1 | 6.19 |
| 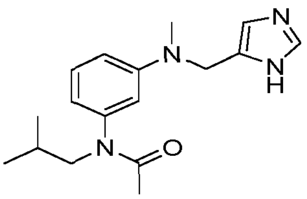 | ESC1002171 | | | 34 | | < 4.7 |
| 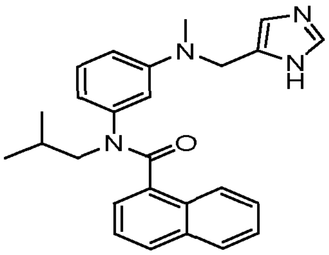 | ESC1002172 | 96 | 0 | 87 | 1.1 | 5.55 |
| 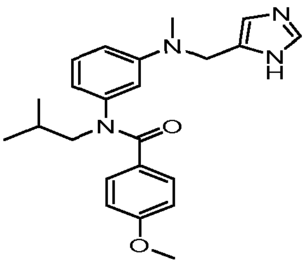 | ESC1002182 | 103 | 0 | 99 | 0.8 | 6.48 |
| 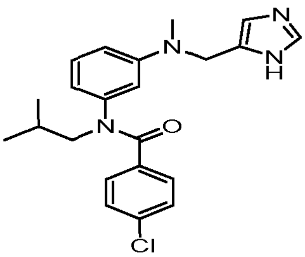 | ESC1002183 | 97 | 0 | 95 | 1.2 | 6.11 |
| 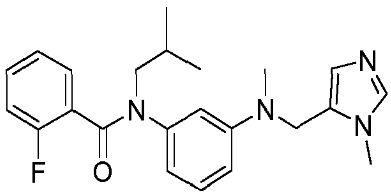 | ESC1002199 | | | 13 | | < 4.7 |

(continued)

| Compound Structure | Compound Reference | Max . Fit | Min. Fit | Max. Effect (%) | Hill Slope | $pEC_{50}$ |
|---|---|---|---|---|---|---|
| 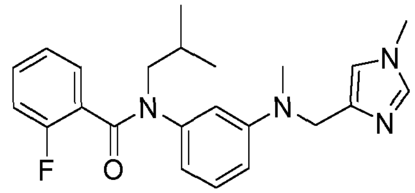 | ESC1002200 | 96 | 0 | 67 | 1 | 5.04 |
| 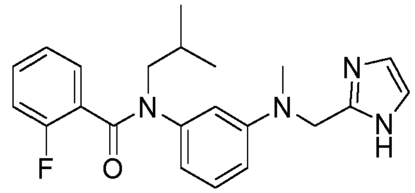 | ESC1002201 | | | 41 | | < 4.7 |
| 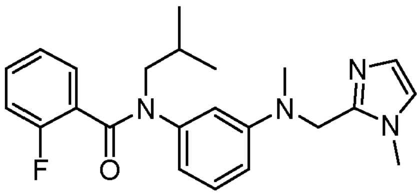 | ESC1002202 | 79 | 0 | 57 | 1.3 | 5 |
| 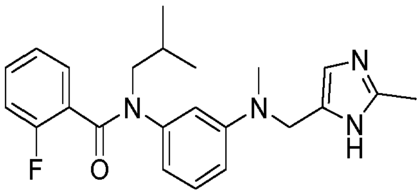 | ESC1002203 | 98 | 0 | 97 | 1.1 | 6.28 |
| 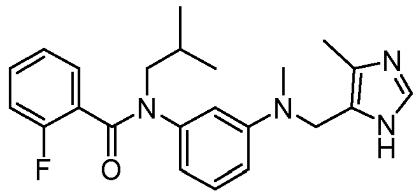 | ESC1002204 | 98 | 0 | 97 | 1.2 | 6.4 |
| 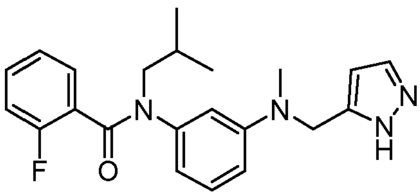 | ESC1002205 | 97 | 0 | 96 | 1.1 | 6.05 |
| 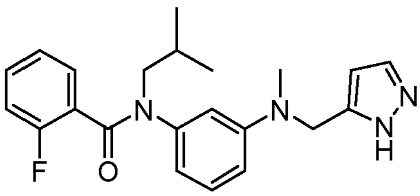 | ESC1002206 | 107 | 0 | 79 | 0.93 | 5.18 |

(continued)

| Compound Structure | Compound Reference | Max . Fit | Min. Fit | Max. Effect (%) | Hill Slope | $pEC_{50}$ |
|---|---|---|---|---|---|---|
| 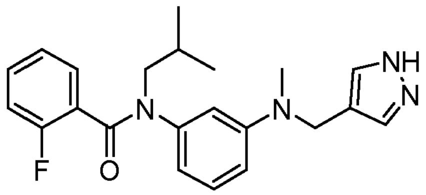 | ESC1002205 | 98 | 0 | 96 | 1.2 | 6.04 |
| 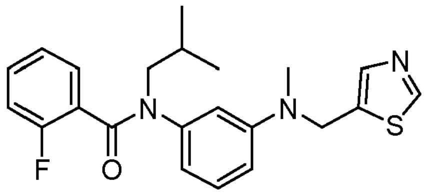 | ESC1002208 | | | 50 | | < 4.7 |
| 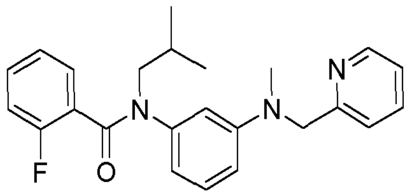 | ESC1002209 | 104 | 0 | 80 | 0.98 | 5.24 |
| 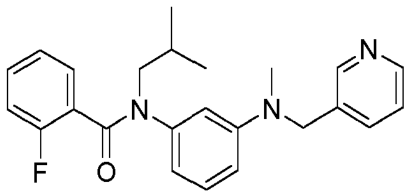 | ESC1002210 | 82 | 0 | 72 | 1.4 | 5.21 |
| 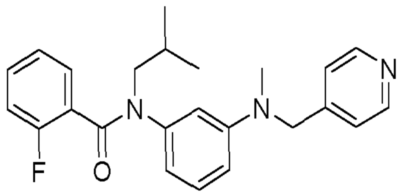 | ESC1002211 | | | 40 | | < 4.7 |
| 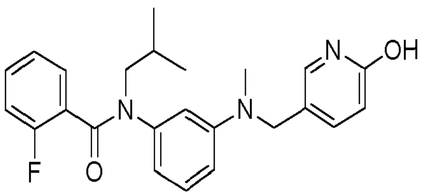 | ESC1002212 | | | 16 | | < 4.7 |
| 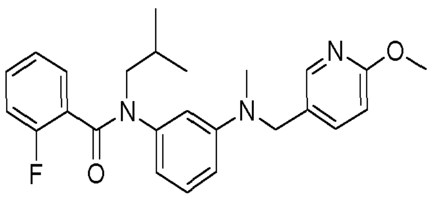 | ESC1002213 | | | 15 | | < 4.7 |

(continued)

| Compound Structure | Compound Reference | Max . Fit | Min. Fit | Max. Effect (%) | Hill Slope | $pEC_{50}$ |
|---|---|---|---|---|---|---|
| 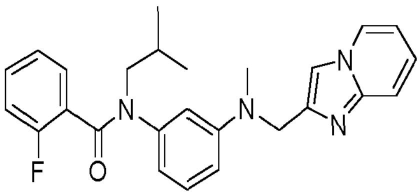 | ESC1002214 | | | 22 | | < 4.7 |
| 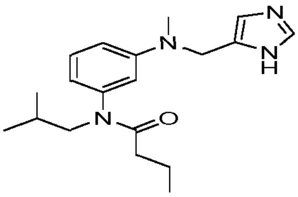 | ESC1002235 | 93 | -3 | 85 | 1.1 | 5.53 |
| 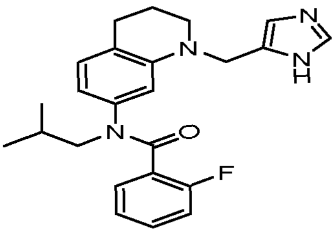 | ESC1002236 | | | 30 | | < 4.7 |
| 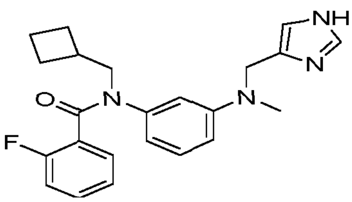 | ESC1002265 | 99 | 0 | 96 | 1.1 | 6.02 |
| 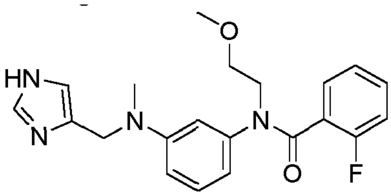 | ESC1002266 | 100 | -4 | 74 | 0.98 | 5.19 |
| 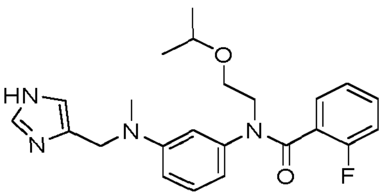 | ESC1002267 | 103 | -4 | 76 | 0.9 | 5.22 |
| 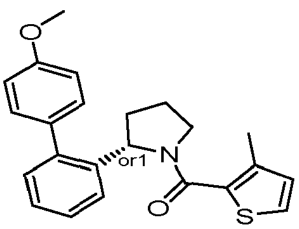 | ESC100233 2 (not of the invention claimed herein) | 100 | 0 | 99 | 1 | 6.78 |

(continued)

| Compound Structure | Compound Reference | Max . Fit | Min. Fit | Max. Effect (%) | Hill Slope | $pEC_{50}$ |
|---|---|---|---|---|---|---|
| 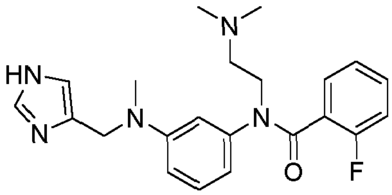 | ESC1002334 | | | 23 | | < 4.7 |
| 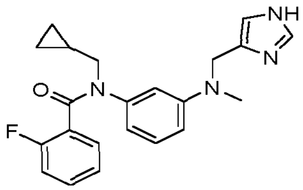 | ESC1002342 | 95 | -1 | 99 | 1.6 | 6.15 |
| 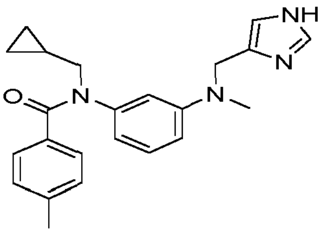 | ESC1002399 | 99 | 2 | 95 | 1.1 | 6 |
| 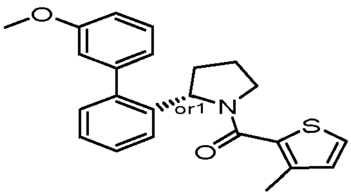 | ESC1002421 (not of the invention claimed herein) | 95 | 11 | 97 | 1.6 | 6.73 |
| 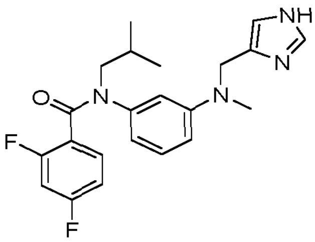 | ESC1002454 | 97 | -8 | 96 | 1.1 | 6.34 |
| 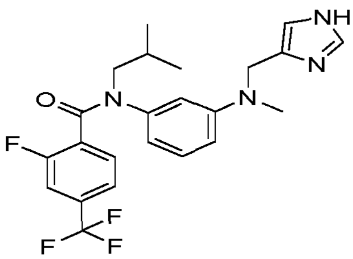 | ESC1002455 | 97 | -7 | 92 | 0.99 | 5.9 |
| 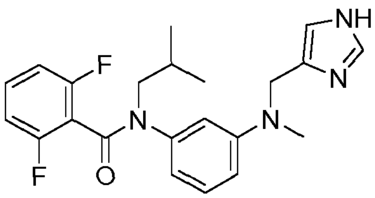 | ESC1002456 | 96 | 17 | 97 | 1.2 | 6.86 |

(continued)

| Compound Structure | Compound Reference | Max . Fit | Min. Fit | Max. Effect (%) | Hill Slope | pEC$_{50}$ |
|---|---|---|---|---|---|---|
| 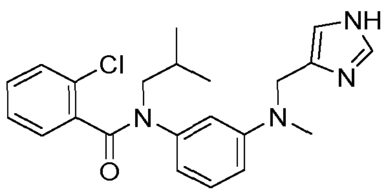 | ESC1002457 | 94 | -4 | 95 | 1.4 | 6.35 |
| 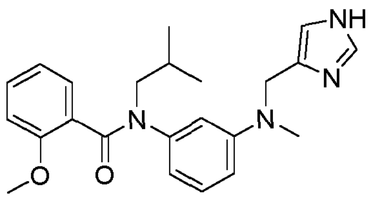 | ESC1002458 | 97 | -7 | 96 | 1 | 6.26 |
| 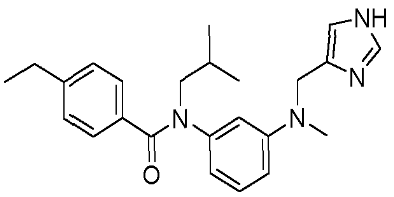 | ESC1002459 | 94 | -4 | 93 | 1.2 | 6.08 |
| 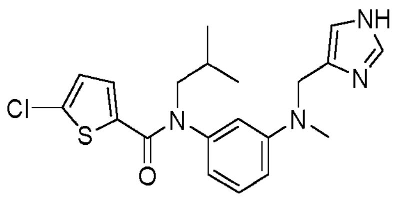 | ESC1002460 | 95 | -5 | 90 | 1 | 5.92 |
| 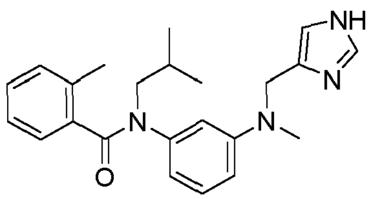 | ESC1002491 | 102 | 2 | 96 | 1 | 5.93 |
| 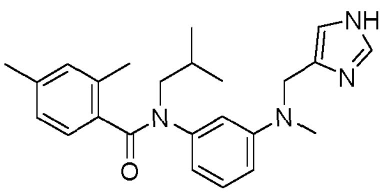 | ESC1002492 | 104 | -10 | 93 | 0.73 | 6.06 |
| 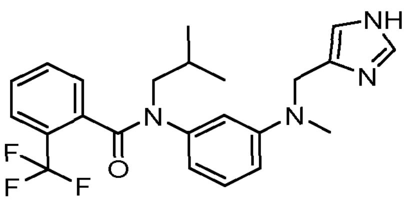 | ESC1002493 | 104 | -8 | 94 | 0.75 | 6.01 |

(continued)

| Compound Structure | Compound Reference | Max . Fit | Min. Fit | Max. Effect (%) | Hill Slope | $pEC_{50}$ |
|---|---|---|---|---|---|---|
| 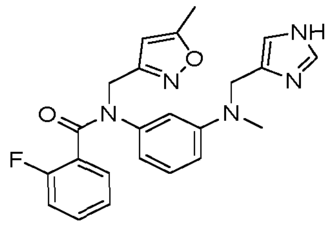 | ESC1002516 (not of the invention claimed herein) | | | 46 | | < 4.7 |
| 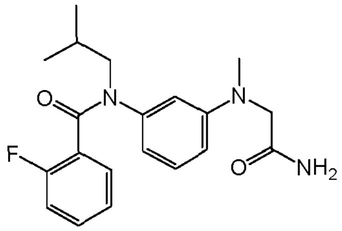 | ESC1002570 | | | 48 | | < 4.7 |
| 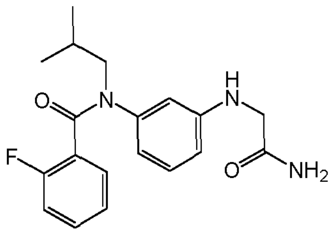 | ESC1002571 | | | 13 | | < 4.7 |
| 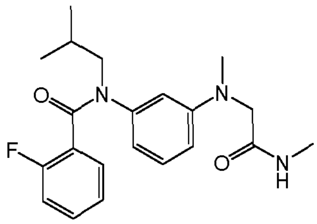 | ESC1002572 | 100 | 2 | 65 | 0.98 | 4.95 |
| 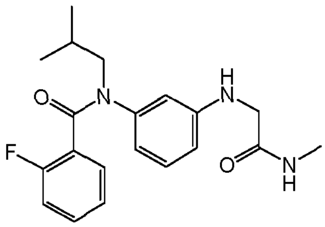 | ESC1002573 | | | 4 | | < 4.7 |
| 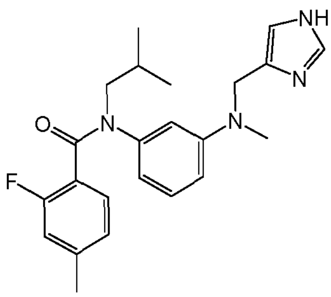 | ESC1002575 | 101 | 10 | 102 | 1.3 | 6.57 |

(continued)

| Compound Structure | Compound Reference | Max . Fit | Min. Fit | Max. Effect (%) | Hill Slope | $pEC_{50}$ |
|---|---|---|---|---|---|---|
| 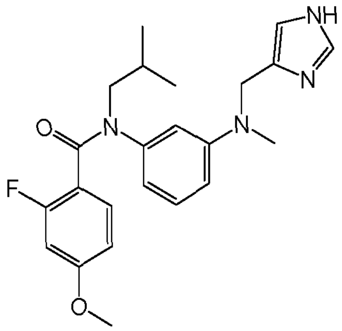 | ESC1002576 | 97 | -3 | 98 | 1.1 | 6.61 |
| 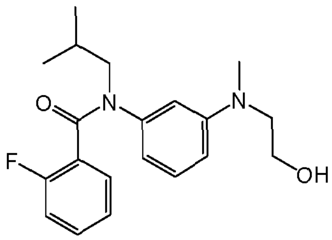 | ESC1002577 | 100 | -1 | 72 | 0.95 | 5.09 |
| 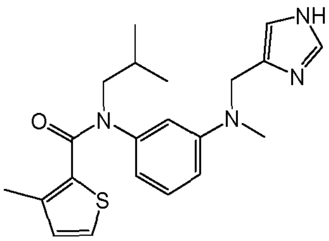 | ESC1002597 | 96 | 4 | 100 | 1.4 | 6.4 |
| 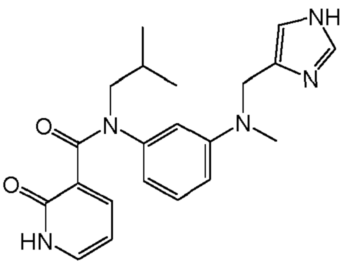 | ESC1002614 | | | 30 | | < 4.7 |
| 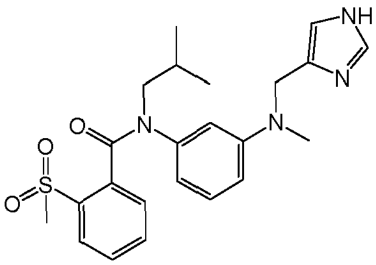 | ESC1002622 | 108 | -6 | 76 | 0.9 | 5.16 |

(continued)

| Compound Structure | Compound Reference | Max . Fit | Min. Fit | Max. Effect (%) | Hill Slope | $pEC_{50}$ |
|---|---|---|---|---|---|---|
| 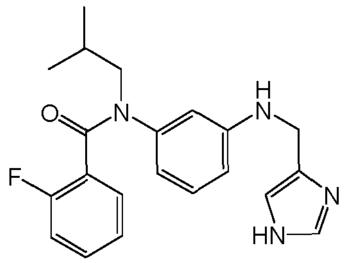 | ESC1002631 | 100 | -6 | 64 | 0.94 | 5.03 |
| 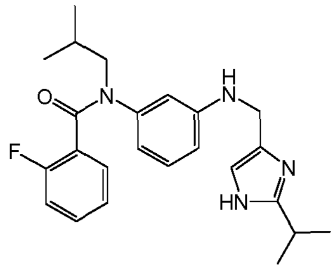 | ESC1002632 | | | 18 | | < 4.7 |
| 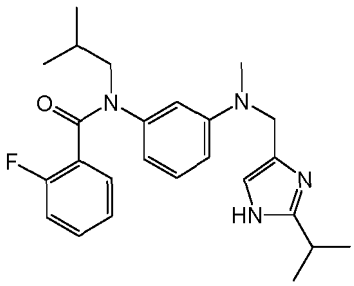 | ESC1002633 | 100 | -3 | 45 | 0.93 | < 4.7 |
| 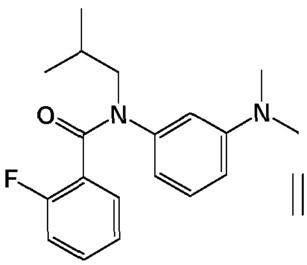 | ESC1002636 | 101 | -1 | 79 | 0.98 | 5.26 |
| 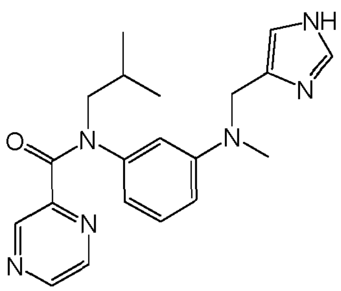 | ESC1002656 | | | 45 | | < 4.7 |

(continued)

| Compound Structure | Compound Reference | Max . Fit | Min. Fit | Max. Effect (%) | Hill Slope | $pEC_{50}$ |
|---|---|---|---|---|---|---|
| 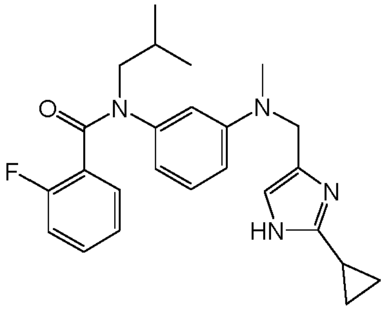 | ESC1002674 | 80 | -5 | 59 | 1.2 | 5.08 |
| 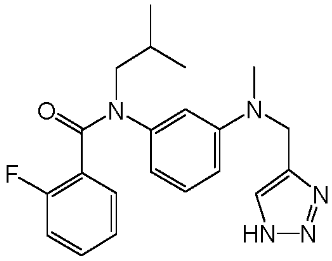 | ESC1002675 | 87 | -2 | 75 | 1.2 | 5.35 |
| 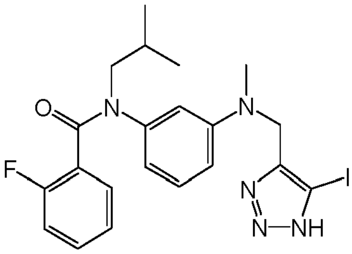 | ESC1002687 | 100 | -6 | 64 | 1 | 4.96 |
| 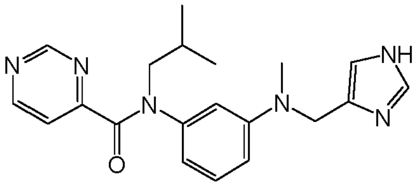 | ESC1002703 | 70 | -5 | 52 | 1.3 | 5.06 |
| 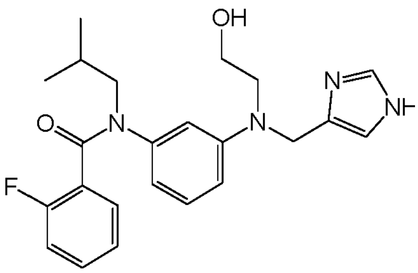 | ESC1002723 | 94 | 0 | 81 | 1.1 | 5.29 |
| 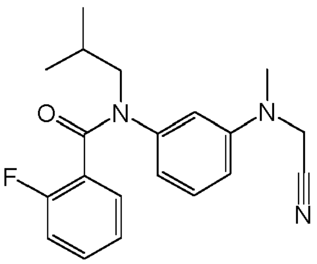 | ESC1002762 | | | 39 | | < 4.7 |

(continued)

| Compound Structure | Compound Reference | Max . Fit | Min. Fit | Max. Effect (%) | Hill Slope | pEC50 |
|---|---|---|---|---|---|---|
| 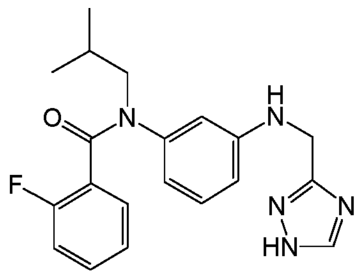 | ESC1002763 | | | 8 | | < 4.7 |
| 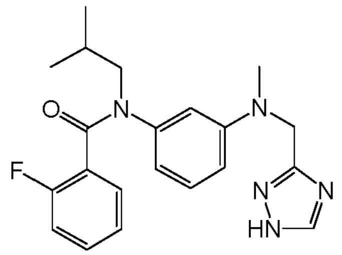 | ESC1002770 | 91 | -4 | 86 | 1.2 | 5.57 |
| 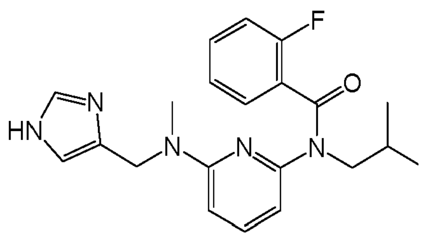 | ESC1002795 | 93 | 0 | 77 | 1.1 | 5.25 |
| 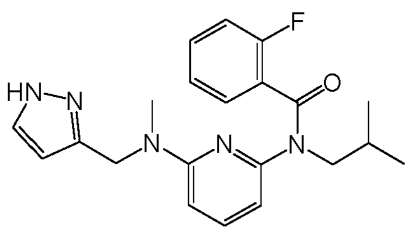 | ESC1002796 | 100 | -4 | 47 | 1.2 | < 4.7 |
| 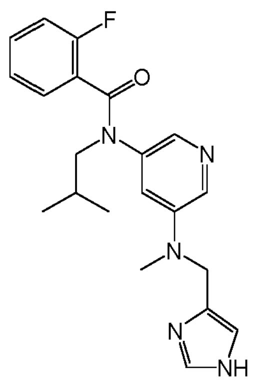 | ESC1002802 | | | 20 | | < 4.7 |

(continued)

| Compound Structure | Compound Reference | Max . Fit | Min. Fit | Max. Effect (%) | Hill Slope | $pEC_{50}$ |
|---|---|---|---|---|---|---|
| 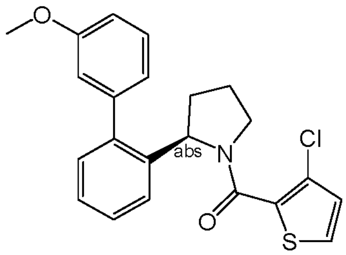 | ESC1002804 (not of the invention claimed herein) | | | 25 | | < 4.7 |

**Table 7 - Cytotoxicity and viability of compounds in mtsABAD HEK293 cells. A) Percentage cytotoxicity of mtsABAD HEK293 cells treated with 50 μM compound as measured by lactate dehydrogenase assay. B) Percentage of viable mtsABAD HEK293 cells treated with 50 μM compound as measured by Alamar Blue assay.**

| | | A) LDH % Toxicity (50 μM Dose) | B) Alamar Blue % Viability (50 μM Dose) |
|---|---|---|---|
| **Compound ID** | **MW** | | |
| ESC1002164-01 | 440.52 | 5.093 | 77.951 |
| ESC1002165-01 | 328.46 | 4.074 | 88.291 |
| ESC1002166-01 | 368.52 | 4.554 | 82.237 |
| ESC1002167-01 | 376.5 | 4.492 | 79.269 |
| ESC1002168-01 | 390.53 | 4.285 | 82.648 |
| ESC1002169-01 | 362.47 | 4.554 | 84.229 |
| ESC1002171-01 | 300.4 | 5.933 | 86.843 |
| ESC1002172-01 | 412.53 | 5.472 | 85.337 |
| ESC1002183-01 | 396.92 | 5.082 | 89.373 |
| ESC1002182-01 | 392.5 | 4.733 | 87.643 |
| ESC1002199-01 | 430.19 | 5.028 | 89.524 |
| ESC1002200-01 | 430.19 | 5.136 | 79.220 |
| ESC1002201-01 | 416.18 | 5.842 | 80.481 |
| ESC1002202-01 | 430.19 | 2.542 | 81.531 |
| ESC1002203-01 | 430.19 | 6.638 | 87.491 |
| ESC1002204-01 | 394.2 | 5.048 | 88.836 |
| ESC1002205-01 | 380.2 | 5.840 | 95.933 |
| ESC1002206-01 | 380.2 | 5918 | 95.609 |
| ESC1002205-02 | 416.18 | 5.243 | 89.638 |
| ESC1002208-01 | 433.14 | 20.084 | 65.577 |
| ESC1002209-01 | 427.18 | 5.698 | 80.514 |
| ESC1002210-01 | 427.18 | 5.509 | 93.200 |
| ESC1002211-01 | 391.21 | 5.501 | 82.553 |
| ESC1002212-01 | 407.2 | 5.576 | 94.278 |
| ESC1002213-01 | 421.2 | 8.010 | 81.860 |
| ESC1002214-02 | 430.2 | 5.902 | 82.439 |

(continued)

| | | A) LDH % Toxicity (50 μM Dose) | B) Alamar Blue % Viability (50 μM Dose) |
|---|---|---|---|
| Compound ID | MW | | |
| ESC1002235-01 | 328.46 | 5.54 | 84.09 |
| ESC1002236-01 | 406.5 | 3.94 | 82.69 |
| ESC1002265-01 | 392.48 | 4.52 | 80.52 |
| ESC1002267-01 | 410.49 | 4.81 | 89.57 |
| ESC1002266-01 | 382.44 | 5.22 | 89.86 |
| ESC1002332-02 (not of the invention claimed herein) | 377.49 | 3.52 | 87.68 |
| ESC1002334-01 | 395.48 | 517 | 92.96 |
| ESC1002342-01 | 378.45 | 4.76 | 8504 |

## Claims

**1.** A compound of formula (I):

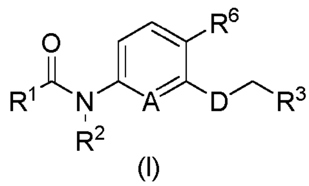

(I) ;

wherein $R^1$ is any one selected from the group consisting of $C_6$-$C_{10}$aryl, $C_3$-$C_5$heteroaryl, $C_{10}$-$C_{16}$biaryl, $C_6$-$C_{14}$biheteroaryl, $C_5$-$C_8$cycloalkyl, $C_3$-$C_7$heterocycloalkyl, $C_1$-$C_6$alkyl, $C_6$-$C_{10}$aryl$C_1$-$C_3$alkyl, $C_{10}$-$C_{16}$biaryl$C_1$-$C_3$alkyl and $C_3$-$C_8$Cycloalkyl$C_1$-$C_3$alkyl optionally substituted one or more times with any one or a combination selected from the group consisting of halo, $C_1$-$C_4$haloalkyl, $C_1$-$C_4$alkyl, $C_1$-$C_4$alkoxy, $C_1$-$C_4$haloalkoxy, hydroxy and methylsulfonyl;

$R^2$ is any one selected from the group consisting of iso-butyl, iso-propyl, cyclobutyl, cyclopropyl, cyclobutylmethyl, cyclopropylmethyl, methoxyethyl, isopropoxyethyl, and di($C_{1-4}$alkyl)amino$C_{1-3}$alkyl;
A is CH or N;
$R^3$ is any one selected from the group consisting of: $C_2$-$C_5$heteroaryl optionally substituted one or more times with any one or a combination selected from the group consisting of $C_1$-$C_4$alkyl, $C_3$-$C_5$cycloalkyl, halo, hydroxy and $C_{1-3}$alkoxy; ethyne, hydroxymethyl, amido, N-methylamido and cyano;
D is $NR^7$ or O, wherein $R^7$ is $C_{1-3}$alkyl or $C_{1-3}$alkylol; and
$R^6$ is H; or $R^6$ and D together form a 5 or 6 membered heterocycle optionally substituted one or more times with any one or a combination selected from the group consisting of oxo, halo, $C_{1-3}$alkyl, $C_{1-3}$haloalkyl, hydroxy and $C_{1-3}$alkoxy.

**2.** The compound of claim 1, wherein $R^1$ is:

(i) any one selected from the group consisting of $C_6$-$C_{10}$aryl, $C_3$-$C_5$heteroaryl, $C_{10}$-$C_{16}$biaryl, $C_5$-$C_8$cycloalkyl, $C_3$-$C_6$alkyl and $C_6$-$C_{10}$aryl$C_1$-$C_3$alkyl, optionally substituted one or more times with any one or a combination selected from the group consisting of halo, $C_1$-$C_4$haloalkyl, $C_1$-$C_4$alkyl, $C_1$-$C_4$alkoxy, hydroxy and methylsulfonyl;
(ii) any one selected from the group consisting of $C_6$-$C_{10}$aryl, $C_3$-$C_5$heteroaryl, $C_5$-$C_8$cycloalkyl and $C_3$-$C_6$alkyl, optionally substituted one or more times with any one or a combination selected from the group consisting of halo, $C_1$-$C_4$haloalkyl, $C_1$-$C_4$alkyl, $C_1$-$C_4$alkoxy and hydroxy; or
(iii) any one selected from the group consisting of phenyl, thiophenyl, pyridinyl, cyclohexyl and iso-propyl, optionally substituted one or more times with any one or a combination selected from the group consisting of fluoro, chloro, trifluoromethyl, methyl, ethyl and methoxy.

**3.** The compound of claim 1 or claim 2, wherein $R^2$ is any one selected from the group consisting of iso-butyl, iso-propyl,

cyclobutylmethyl and cyclopropylmethyl.

4. The compound of any one of claims 1 to 3, wherein A is CH.

5. The compound of any one of claims 1 to 4, wherein $R^3$ is an optionally substituted $C_2$-$C_5$heteroaryl comprising nitrogen and/or sulfur as heteroatoms.

6. The compound of claim 5, wherein $R^3$ comprises nitrogen as the only heteroatom.

7. The compound of any one of claims 1 to 6, wherein $R^3$ is a $C_3$heteroaryl, optionally substituted one or more times with methyl.

8. The compound of any one of claims 1 to 4, wherein $R^3$ is any one selected from the group consisting of imidazolyl, 1-methylimidazolyl, 2-methylimidazolyl, 4-methylimidazolyl and pyrazolyl.

9. The compound of any one of claims 1 to 4, wherein $R^3$ is any one selected from the group consisting of imidazol-5-yl, 1-methylimidazol-2-yl, 1-methylimidazol-4-yl, 2-methylimidazol-5-yl, 4-methylimidazol-5-yl and pyrazol-5-yl.

10. The compound of any one of claims 1 to 9, wherein D is $NCH_3$ and $R^6$ is H.

11. A composition comprising the compound of any one of claims 1 to 10 and one or more pharmaceutically acceptable excipients.

12. A compound of any one of claims 1 to 10 or a composition of claim 11 for use as an ABAD inhibitor.

13. A compound of any one of claims 1 to 10 or a composition of claim 11 for use as a medicament.

14. A compound of any one of claims 1 to 10 or a composition of claim 11 for use in the treatment or prophylaxis of dementia or cancer.

**Patentansprüche**

1. Verbindung der Formel (I):

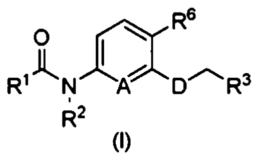

(I) ;

wobei $R^1$eines ausgewählt aus der Gruppe bestehend aus $C_6$ - $C_{10}$-Aryl, $C_3$ - $C_5$-Heteroaryl, $C_{10}$ - $C_{16}$-Biaryl, $C_6$ - $C_{14}$-Biheteroaryl, $C_5$ - $C_8$-Cycloalkyl, $C_3$ - $C_7$-Heterocycloalkyl, $C_1$ - $C_6$-Alkyl, $C_6$ - $C_{10}$-Ary19999rt5555555555555555555552$C_1$ - $C_3$-alkyl, $C_{10}$ - $C_{16}$-Biaryl$C_1$ - $C_3$-alkyl und $C_3$ - $C_8$-Cycloalkyl-$C_1$ - $C_3$-alkyl ist, das gegebenenfalls ein- oder mehrmals mit einem oder einer Kombination ausgewählt aus der Gruppe bestehend aus Halogen, $C_1$-$C_4$-Halogenalkyl, $C_1$ - $C_4$-Alkyl, $C_1$ - $C_4$-Alkoxy, $C_1$ - $C_4$-Halogenalkoxy, Hydroxy und Methylsulfonyl substituiert ist;
$R^2$ ein beliebiges ausgewählt aus der Gruppe bestehend aus iso-Butyl, iso-Propyl, Cyclobutyl, Cyclopropyl, Cyclobutylmethyl, Cyclopropylmethyl, Methoxyethyl, Isopropoxyethyl und Di($C_{1-4}$-alkyl)amino-$C_{1-3}$-alkyl ist;
A CH oder N ist;
$R^3$ eines ausgewählt aus der Gruppe ist, bestehend aus:

$C_2$ - $C_5$Heteroaryl, gegebenenfalls ein- oder mehrmals substituiert mit einem oder einer Kombination ausgewählt aus der Gruppe bestehend aus $C_1$ - $C_4$-Alkyl, $C_3$ - $C_8$-Cycloalkyl, Halogen, Hydroxy und $C_{1-3}$-Alkoxy; Ethin, Hydroxymethyl, Amido, N - Methylamido und Cyano;
D $NR^7$oder O ist, wobei $R^7$für $C_{1-3}$-Alkyl oder $C_{1-3}$-Alkylol steht; und
$R^6$ H ist; oder $R^6$ und D gemeinsam einen 5- oder 6-gliedrigen Heterocyclus bilden, der gegebenenfalls ein-

oder mehrmals mit einem oder einer Kombination, ausgewählt aus der Gruppe bestehend aus Oxo, Halogen, $C_{1-3}$-Alkyl, $C_{1-3}$-Halogenalkyl, Hydroxy und $C_{1-3}$-Alkoxy substituiert ist.

**2.** Verbindung nach Anspruch 1, wobei $R^1$ Folgendes ist:

(i) eines, ausgewählt aus der Gruppe bestehend aus $C_6$ - $C_{10}$-Aryl, $C_3$ - $C_5$-Heteroaryl, $C_{10}$ - $C_{16}$-Biaryl, $C_5$ - $C_8$-Cycloalkyl, $C_3$ - $C_6$-Alkyl und $C_6$ - $C_{10}$-Aryl-$C_1$ - $C_3$-alkyl, gegebenenfalls ein- oder mehrmals substituiert mit einem oder einer Kombination ausgewählt aus der Gruppe bestehend aus Halogen, $C_1$ - $C_4$-Halogenalkyl, $C_1$-$C_4$-Alkyl, $C_1$-$C_4$-Alkoxy, Hydroxy und Methylsulfonyl;
(ii) eines, ausgewählt aus der Gruppe bestehend aus $C_6$ - $C_{10}$-Aryl, $C_3$ - $C_5$-Heteroaryl, $C_5$ - $C_8$-Cycloalkyl und $C_3$-$C_6$-Alkyl, gegebenenfalls ein- oder mehrmals substituiert durch eine oder mehrere Verbindungen, ausgewählt aus der Gruppe bestehend aus Halogen, $C_1$ - $C_4$-Halogenalkyl, $C_1$ - $C_4$-Alkyl, $C_1$ - $C_4$-Alkoxy und Hydroxy; oder
(iii) eines, ausgewählt aus der Gruppe bestehend aus Phenyl, Thiophenyl, Pyridinyl, Cyclohexyl und iso-Propyl, gegebenenfalls ein- oder mehrmals substituiert mit einem oder einer Kombination ausgewählt aus der Gruppe bestehend aus Fluor, Chlor, Trifluormethyl, Methyl, Ethyl und Methoxy.

**3.** Verbindung nach Anspruch 1 oder Anspruch 2, wobei $R^2$ eines ausgewählt aus der Gruppe bestehend aus iso-Butyl, iso-Propyl, Cyclobutylmethyl und Cyclopropylmethyl ist.

**4.** Verbindung nach einem der Ansprüche 1 bis 3, wobei A für CH steht.

**5.** Verbindung nach einem der Ansprüche 1 bis 4, wobei $R^3$ ein gegebenenfalls substituiertes $C_2$-$C_5$-Heteroaryl ist, das Stickstoff und/oder Schwefel als Heteroatome umfasst.

**6.** Verbindung nach Anspruch 5, wobei $R^3$ Stickstoff als das einzige Heteroatom umfasst.

**7.** Verbindung nach einem der Ansprüche 1 bis 6, wobei $R^3$ ein $C_3$-Heteroaryl ist, gegebenenfalls ein- oder mehrmals mit Methyl substituiert.

**8.** Verbindung nach einem der Ansprüche 1 bis 4, wobei $R^3$ eines ausgewählt aus der Gruppe bestehend aus Imidazolyl, 1-Methylimidazolyl, 2-Methylimidazolyl, 4-Methylimidazolyl und Pyrazolyl ist.

**9.** Verbindung nach einem der Ansprüche 1 bis 4, wobei $R^3$ eines ausgewählt aus der Gruppe bestehend aus Imidazol-5-yl, 1-Methylimidazol-2-yl, 1-Methylimidazol-4-yl, 2-Methylimidazol-5-yl, 4-Methylimidazol-5-yl und Pyrazol-5-yl ist.

**10.** Verbindung nach einem der Ansprüche 1 bis 9, wobei D $NCH_3$ ist und $R^6$H ist.

**11.** Zusammensetzung, umfassend die Verbindung nach einem der Ansprüche 1 bis 10 und einen oder mehrere pharmazeutisch akzeptable Hilfsstoffe.

**12.** Verbindung nach einem der Ansprüche 1 bis 10 oder Zusammensetzung nach Anspruch 11 zur Verwendung als ABAD-Inhibitor.

**13.** Verbindung nach einem der Ansprüche 1 bis 10 oder Zusammensetzung nach Anspruch 11 zur Verwendung als Arzneimittel.

**14.** Verbindung nach einem der Ansprüche 1 bis 10 oder einer Zusammensetzung nach Anspruch 11 zur Verwendung bei der Behandlung oder Vorbeugung von Demenz oder Krebs.

## Revendications

**1.** Composé de formule (I) :

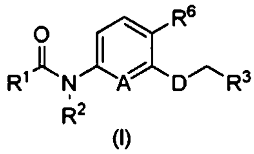

(I) ;

dans lequel $R^1$ est l'un quelconque choisi dans le groupe comprenant $C_6$ - $C_{10}$aryle, $C_3$ - $C_5$hétéroaryle, $C_{10}$ - $C_{16}$biaryle, $C_6$ - $C_{14}$bihétéroaryle, $C_5$ - $C_8$cycloalkyle, $C_3$ - $C_7$hétérocycloalkyle, $C_1$ - $C_6$alkyle, $C_6$ - $C_{10}$aryle $C_1$ - $C_3$alkyle, $C_{10}$ - $C_{16}$biary $C_1$ - $C_3$alkyle et $C_3$ - $C_8$cycioalkyle$C_1$ - $C_3$alkyle éventuellement substitué une ou plusieurs fois par l'un quelconque ou une combinaison choisi dans le groupe comprenant halo, $C_1$-$C_4$haloalkyle, $C_1$ - $C_4$alkyle, $C_1$ - $C_4$alcoxy, $C_1$ - $C_4$haloalcoxy, hydroxy et méthylsulfonyle ;
$R^2$ est l'un quelconque choisi dans le groupe comprenant iso-butyle, iso-propyle, cyclobutyle, cyclopropyle, cyclobutylméthyle, cyclopropylméthyle, méthoxyéthyle, isopropoxyéthyle et di($C_{1-4}$alkyl)amino$C_{1-3}$alkyle ;
A est CH ou N ;
$R^3$ est l'un quelconque choisi dans le groupe comprenant :

$C_2$ - $C_5$ hétéroaryle éventuellement substitué une ou plusieurs fois par l'un quelconque ou une combinaison choisi dans le groupe comprenant $C_1$ - $C_4$alkyle, $C_3$ - $C_8$cycloalkyle, halogéno, hydroxy et $C_{1-3}$alcoxy; éthyne, hydroxyméthyle, amido, N-méthylamido et cyano ;
D est $NR^7$ ou O, dans lequl $R^7$ est $C_{1-3}$alkyle ou $C_{1-3}$alkylol ; et
$R^6$ est H ; ou $R^6$ et D forment ensemble un hétérocycle à 5 ou 6 chaînons éventuellement substitué une ou plusieurs fois par l'un quelconque ou une combinaison choisi dans le groupe comprenant oxo, halo, $C_{1-3}$alkyl, $C_{1-3}$haloalkyle, hydroxy et $C_{1-3}$alcoxy.

**2.** Composé selon la revendication 1, dans lequel $R^1$ est :

(i) l'un quelconque choisi dans le groupe comprenant $C_6$ - $C_{10}$aryle, $C_3$ - $C_5$hétéroaryle, $C_{10}$ - $C_{16}$biaryle, $C_5$ - $C_8$cycloalkyle, $C_3$ - $C_6$alkyle et $C_6$ - $C_{10}$aryle$C_1$ - $C_3$alkyle, éventuellement substitué une ou plusieurs fois par l'un quelconque ou une combinaison choisi dans le groupe comprenant halo, $C_1$ - $C_4$haloalkyle, $C_1$-$C_4$alkyle, $C_1$-$C_4$alcoxy, hydroxy et méthylsulfonyle ;
(ii) l'un quelconque choisi dans le groupe comprenant $C_6$ - $C_{10}$aryle, $C_3$ - $C_5$hétéroaryle, $C_5$ - $C_8$cycloalkyle et $C_3$ - $C_6$alkyle, éventuellement substitué une ou plusieurs fois par l'un quelconque ou une combinaison choisi dans le groupe comprenant halo, $C_1$ - $C_4$haloalkyle, $C_1$ - $C_4$alkyle, $C_1$ - $C_4$alcoxy et hydroxy ; ou
(iii) l'un quelconque choisi dans le groupe comprenant phényle, thiophényle, pyridinyle, cyclohexyle et iso-propyle, éventuellement substitué une ou plusieurs fois par l'un quelconque ou une combinaison choisi dans le groupe comprenant fluoro, chloro, trifluorométhyle, méthyle, éthyle et méthoxy.

**3.** Composé selon la revendication 1 ou la revendication 2, dans lequel $R^2$ est l'un quelconque choisi dans le groupe comprenant iso-butyle, iso-propyle, cyclobutylméthyle et cyclopropylméthyle.

**4.** Composé selon l'une quelconque des revendications 1 à 3, dans lequel A est CH.

**5.** Composé selon l'une quelconque des revendications 1 à 4, dans lequel $R^3$ est un $C_2$-$C_5$hétéroaryle éventuellement substitué comprenant de l'azote et/ou du soufre sous forme d'hétéroatomes.

**6.** Composé selon la revendication 5, dans lequel $R^3$ comprend de l'azote comme seul hétéroatome.

**7.** Composé selon l'une quelconque des revendications 1 à 6, dans lequel $R^3$ est un $C_5$hétéroaryle, éventuellement substitué une ou plusieurs fois par du méthyle.

**8.** Composé selon l'une quelconque des revendications 1 à 4, dans lequel $R^3$ est l'un quelconque choisi dans le groupe comprenant imidazolyle, 1-méthylimidazolyle, 2-méthylimidazolyle, 4-méthylimidazolyle et pyrazolyle.

**9.** Composé selon l'une quelconque des revendications 1 à 4, dans lequel $R^3$ est l'un quelconque choisi dans le groupe comprenant imidazol-5-yle, 1-méthylimidazol-2-yle, 1-méthylimidazol-4-yle, 2-méthylimidazol-5-yle, 4-méthylimidazol-5-yle et pyrazol-5-yle.

**10.** Composé de l'une quelconque des revendications 1 à 9, dans lequel D est $NCH_3$ et $R^6$ est H.

**11.** Composition comprenant le composé selon l'une quelconque des revendications 1 à 10 et un ou plusieurs excipients pharmaceutiquement acceptables.

**12.** Composé selon l'une quelconque des revendications 1 à 10 ou composition selon la revendication 11 pour son utilisation comme inhibiteur d'ABAD.

**13.** Composé selon l'une quelconque des revendications 1 à 10 ou composition selon la revendication 11 pour son utilisation comme médicament.

**14.** Composé selon l'une quelconque des revendications 1 à 10 ou composition selon la revendication 11 pour son utilisation dans le traitement ou la prophylaxie de la démence ou du cancer.

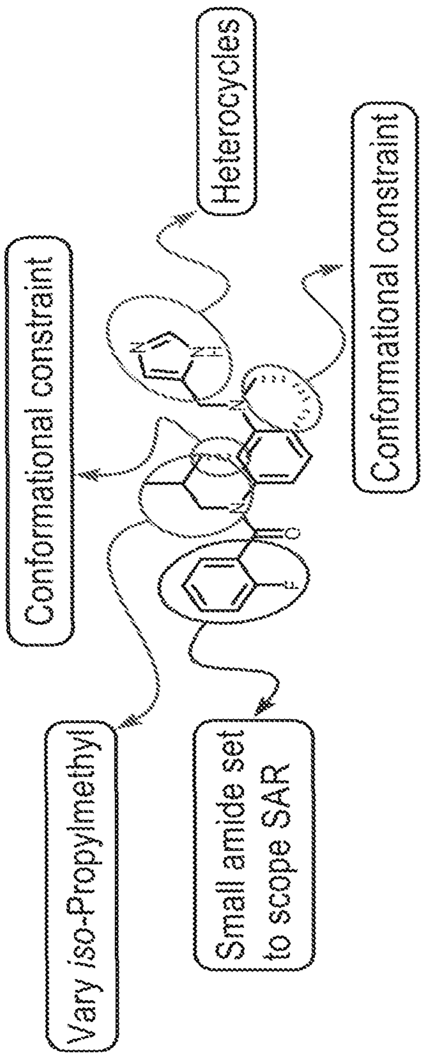
Conformational constraint
Heterocycles
Conformational constraint
Vary iso-Propylmethyl
Small amide set to scope SAR

FIG. 1

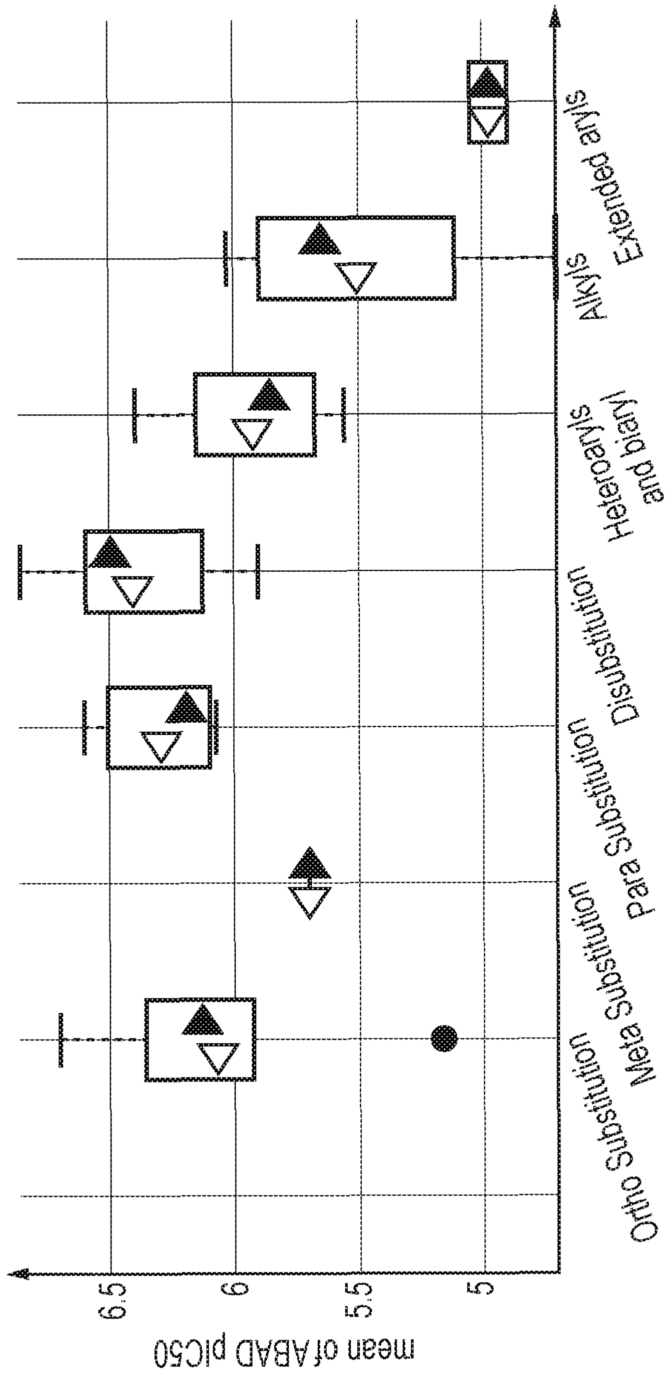
mean of ABAD pIC50
6.5
6
5.5
5
Ortho Substitution
Meta Substitution
Para Substitution
Disubstitution
Heteroaryls and biaryl
Alkyls
Extended aryls

FIG. 2

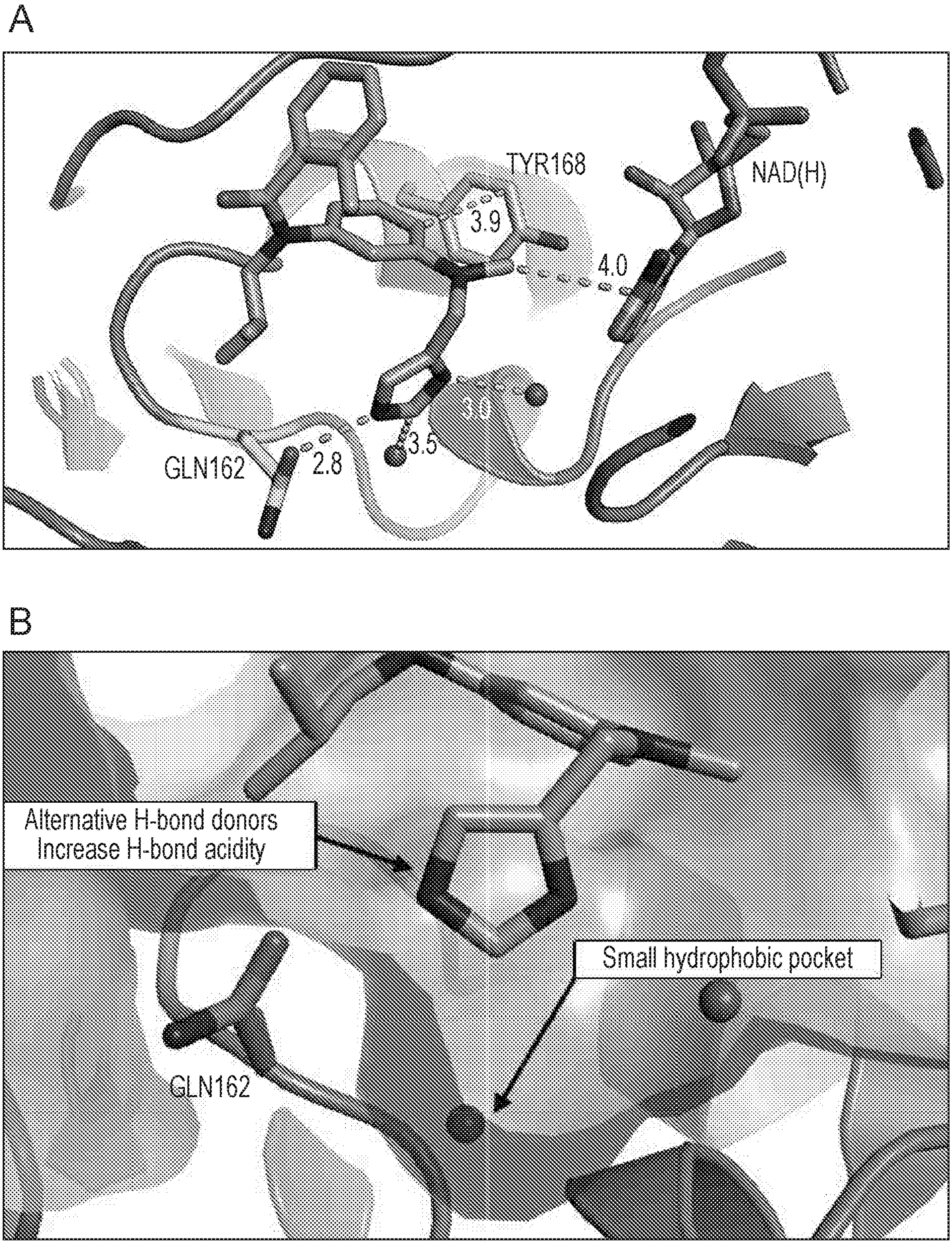
A
TYR168
NAD(H)
3.9
4.0
3.0
3.5
2.8
GLN162
B
Alternative H-bond donors
Increase H-bond acidity
Small hydrophobic pocket
GLN162

FIG. 3

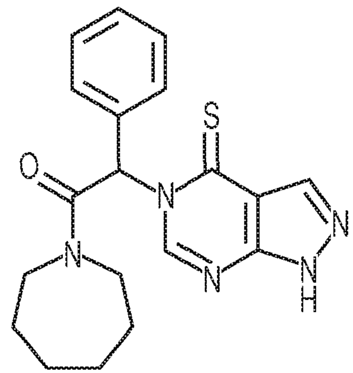

AG18051

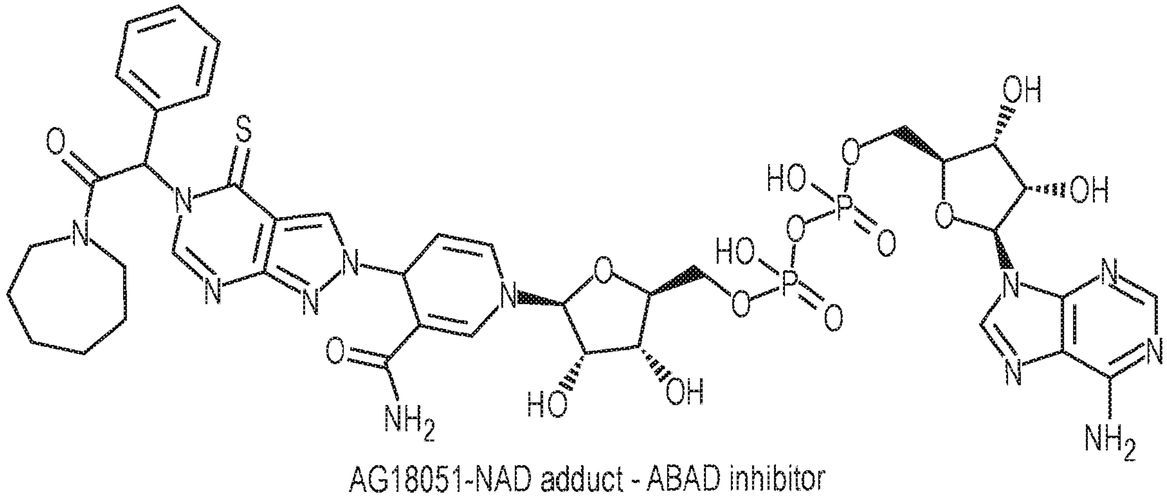

AG18051-NAD adduct - ABAD inhibitor

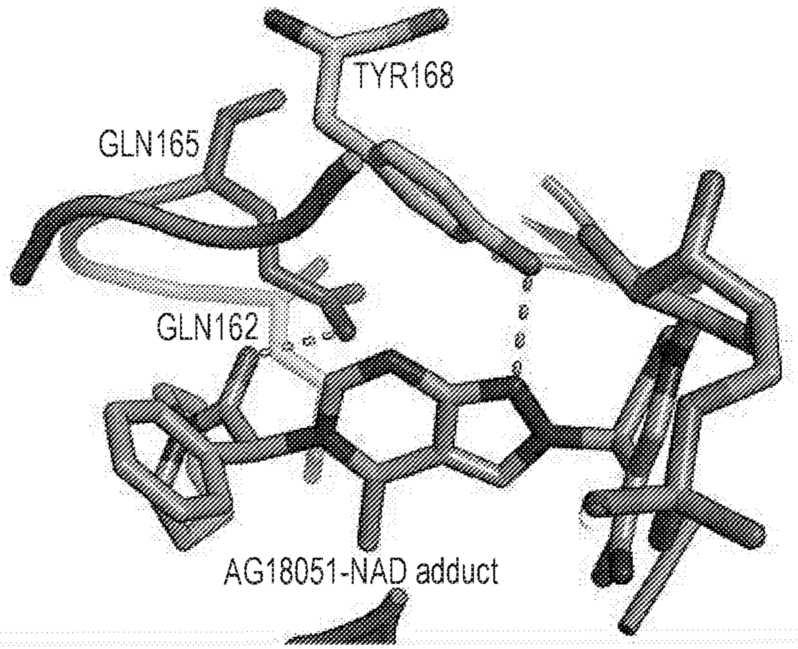


PDB ID 1u7t

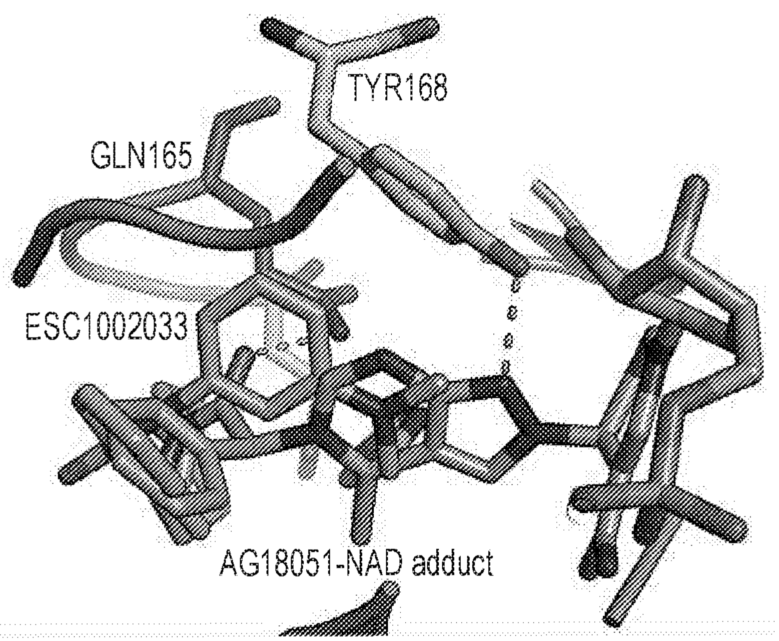


Superposition of ESC1002033

FIG. 4

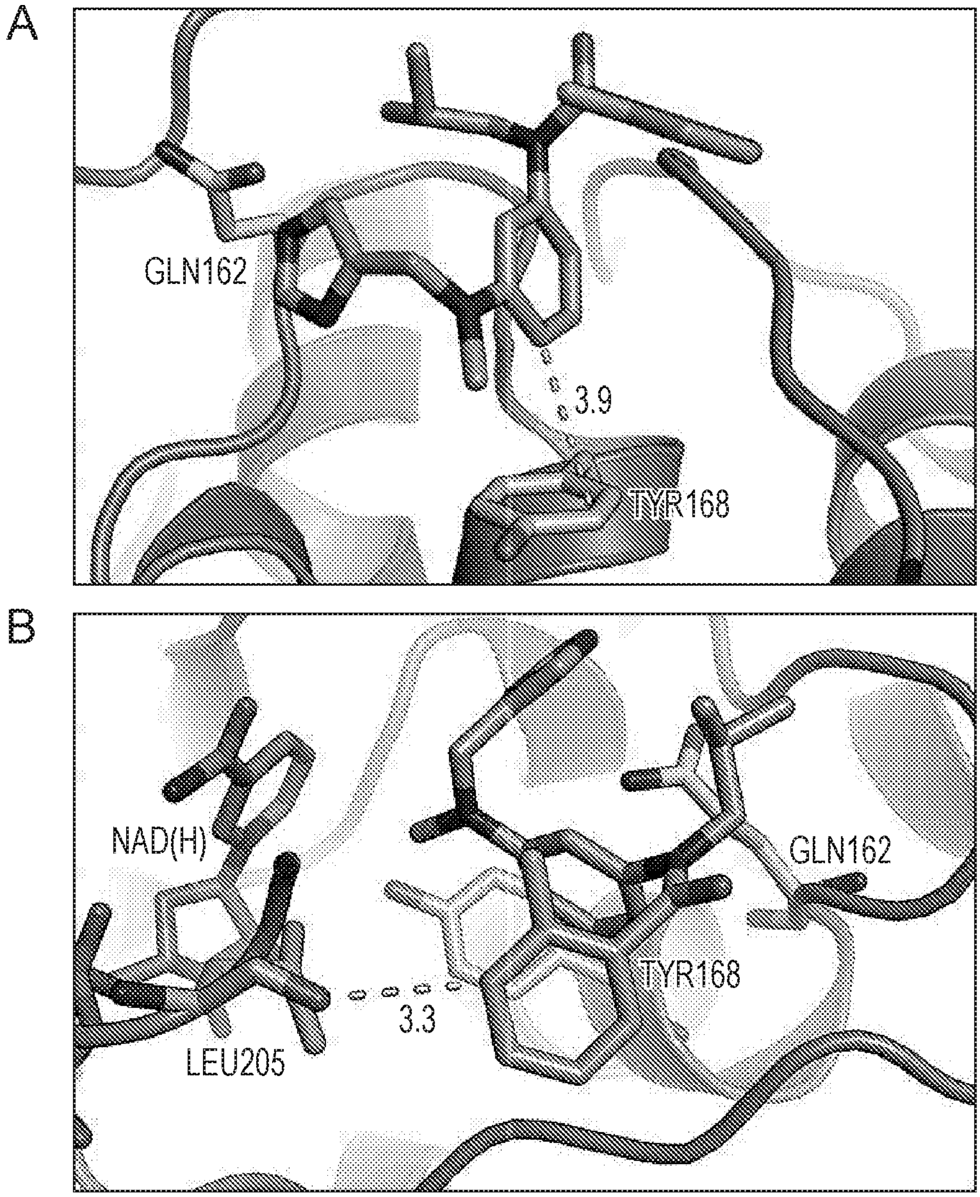


FIG. 5

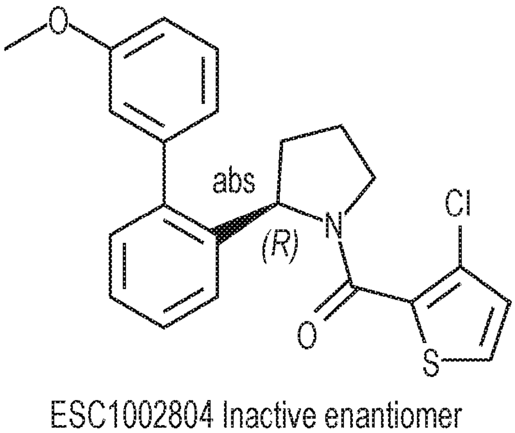

ESC1002804 Inactive enantiomer

FIG. 6

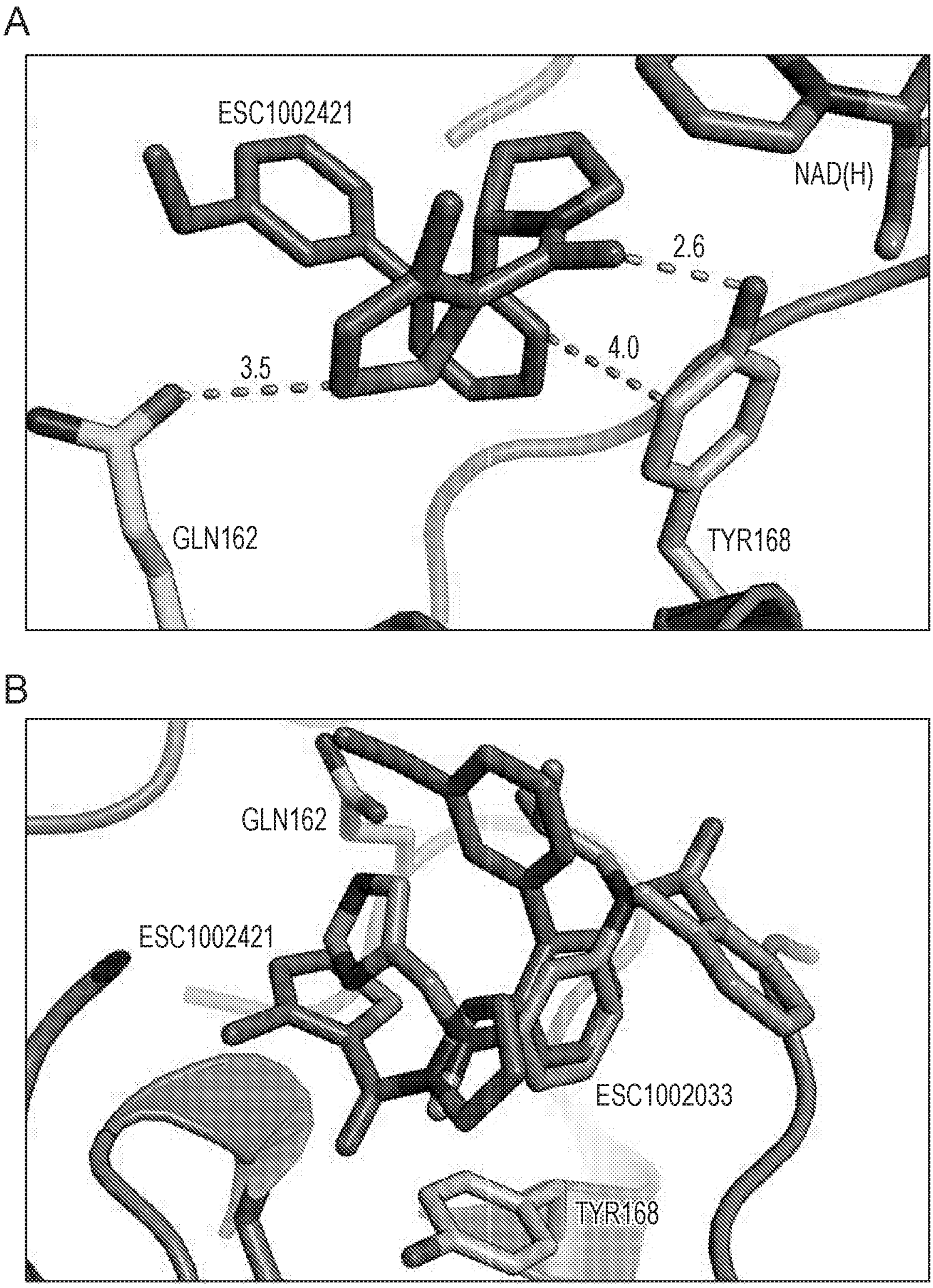
A
ESC1002421
NAD(H)
2.6
4.0
3.5
GLN162
TYR168
B
GLN162
ESC1002421
ESC1002033
TYR168

FIG. 7

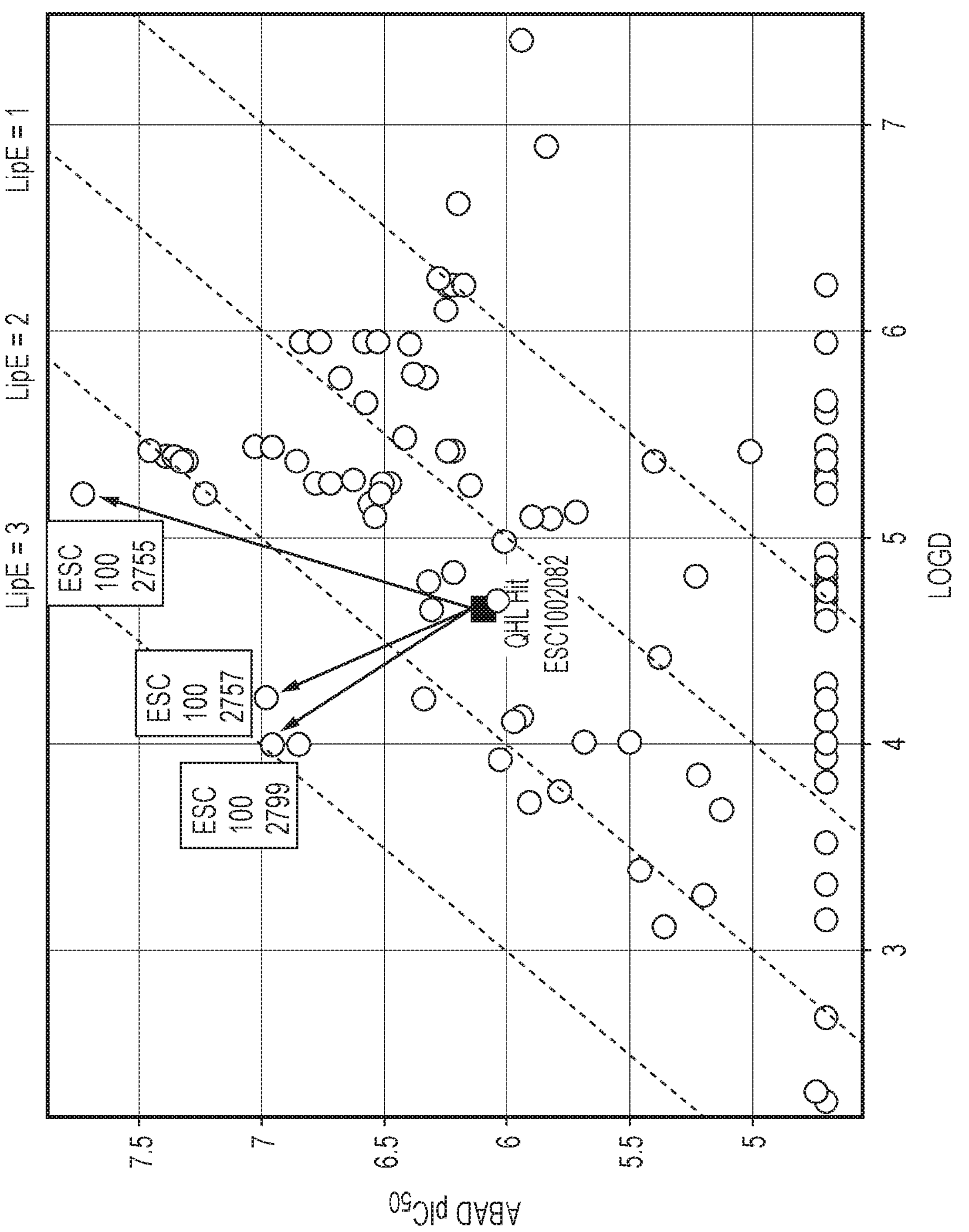
LipE = 3
LipE = 2
LipE = 1
ESC 100 2755
ESC 100 2757
ESC 100 2799
QHL Hit ESC1002082
7.5
7
6.5
6
5.5
5
ABAD $pIC_{50}$
3
4
5
6
7
LOGD

FIG. 8

A

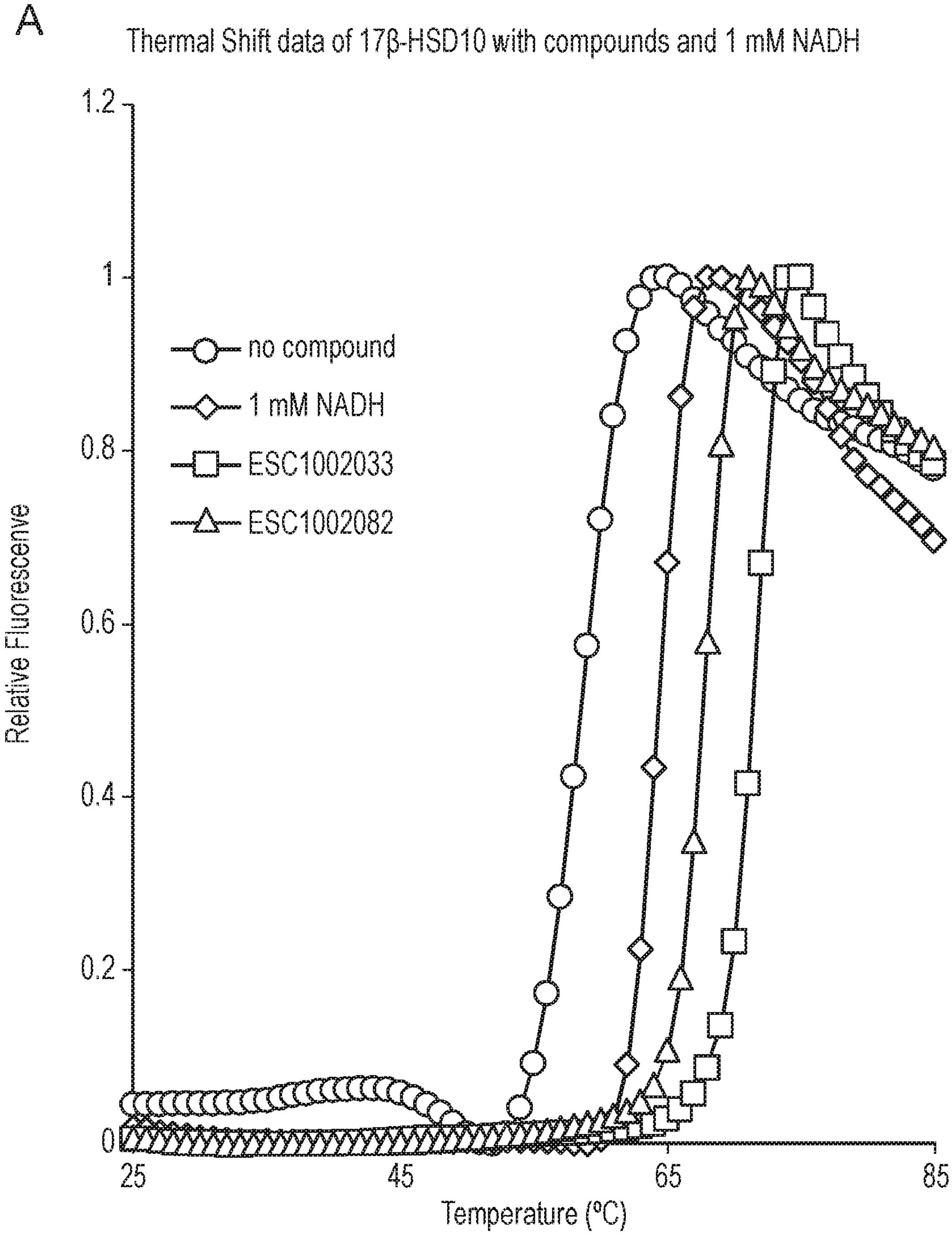


B

| Compound | ESC1002033 | ESC1002082 |
|---|---|---|
| ΔTm (ºC) | 6.75 | 3.714 |
| pEC50 | 6.7 | 6.1 |
| EC50 in CHANA | 2.24 | 4.56 |

FIG. 9

| Compound | CHANA EC50 (μM) | Compound | CHANA EC50 (μM) |
|---|---|---|---|
| ESC1002032 | 2.9 μM | ESC1002340 | 1.688 μM |
| ESC1002033 | 2.24 μM | ESC1002342 | 6.84 μM |
| ESC1002042 | 4.6 μM | ESC1002399 | 1.853 μM |
| ESC1002078 | 2.02 μM | ESC1002421 | 670.6 nM |
| ESC1002082 | 4.56 μM | ESC1002423 | 1.824 μM |
| ESC1002089 | 1.783 μM | ESC1002432 | 1.156 μM |
| ESC1002162 | 1.128 μM | ESC1002456 | 140.5 nM |
| ESC1002166 | 975.9 nM | ESC1002462 | 860.4 nM |
| ESC1002169 | 2.96 μM | ESC1002575 | 366.8 nM |
| ESC1002182 | 1.673 μM | ESC1002576 | 599.2 nM |
| ESC1002183 | 1.224 μM | ESC1002597 | 511.2 nM |
| ESC1002203 | 2.468 μM | ESC1002606 | 477.2 nM |
| ESC1002204 | 1.362 μM | ESC1002664 | 2.56 μM |
| ESC1002205-01 | 3.135 μM | ESC1002666 | 138 nM |
| ESC1002205-02 | 2.261 μM | ESC1002699 | 1.152 μM |
| ESC1002265 | 5.549 μM | ESC1002700 | 1.881 μM |
| ESC1002314 | 2.392 μM | ESC1002755 | 28.4 nM |
| ESC1002316 | 0.9933 μM | ESC1002757 | 265.6 nM |
| ESC1002317 | 3.368 μM | ESC1002767 | 873.1 nM |
| ESC1002320 | 2.564 μM | ESC1002769 | 713.1 nM |
| ESC1002321 | 1.531 μM | ESC1002775 | 448.9 nM |
| ESC1002323 | 1.677 μM | ESC1002776 | 228.3 nM |
| ESC1002324 | 8.643 μM | ESC1002788 | 351 nM |
| ESC1002325 | 5.736 μM | ESC1002799 | 170 nM |
| ESC1002326 | 5.605 μM | ESC1002801 | 50 nM |
| ESC1002332 | 7.221 μM | ESC1002838 | 231.6 nM |
| ESC1002337 | 1.745 μM | ESC1002840 | 878.7 nM |
| ESC1002338 | 1.147 μM | ESC1002842 | 40 nM |
| ESC1002339 | 1.853 μM | | |

FIG. 10

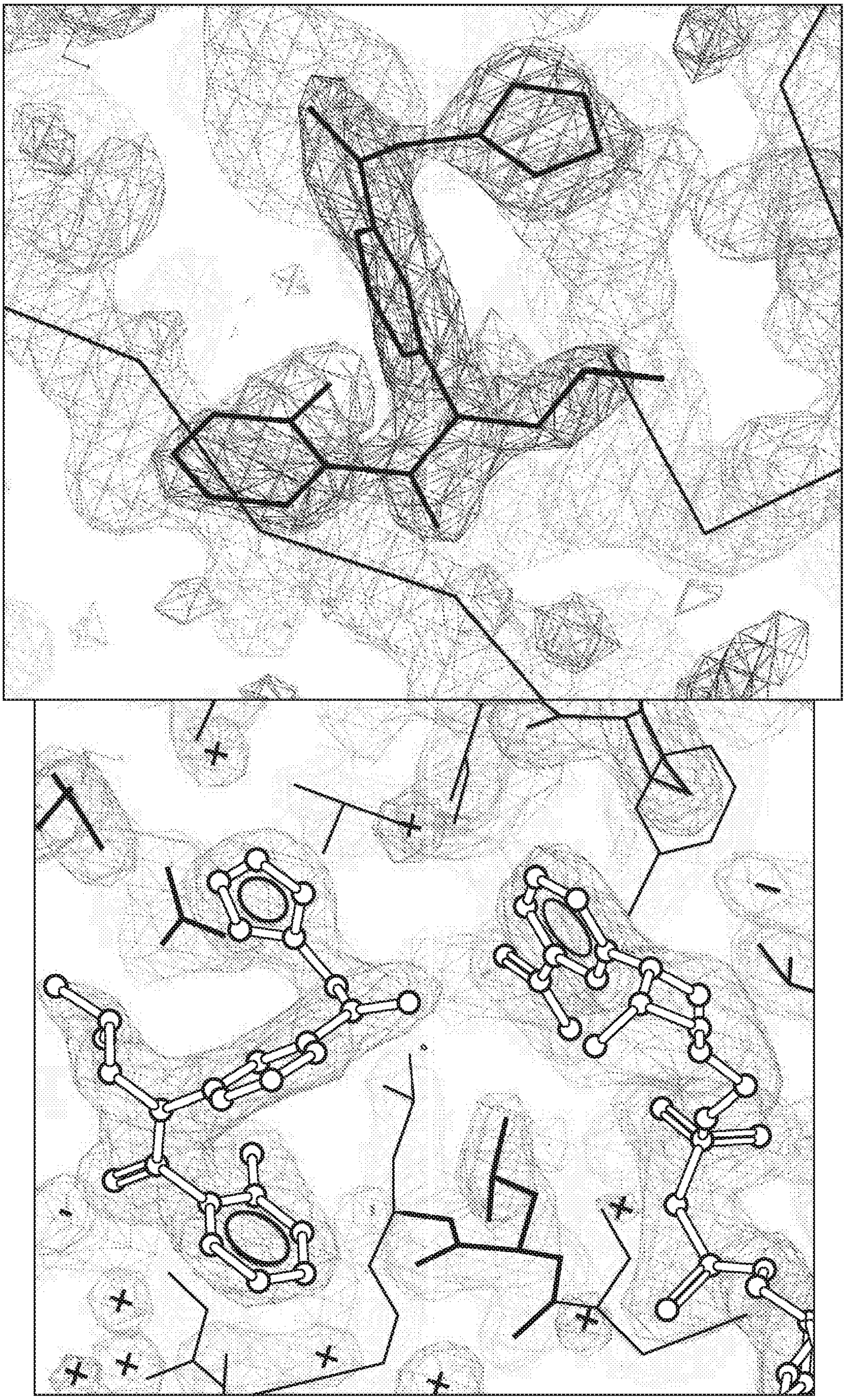

FIG. 11

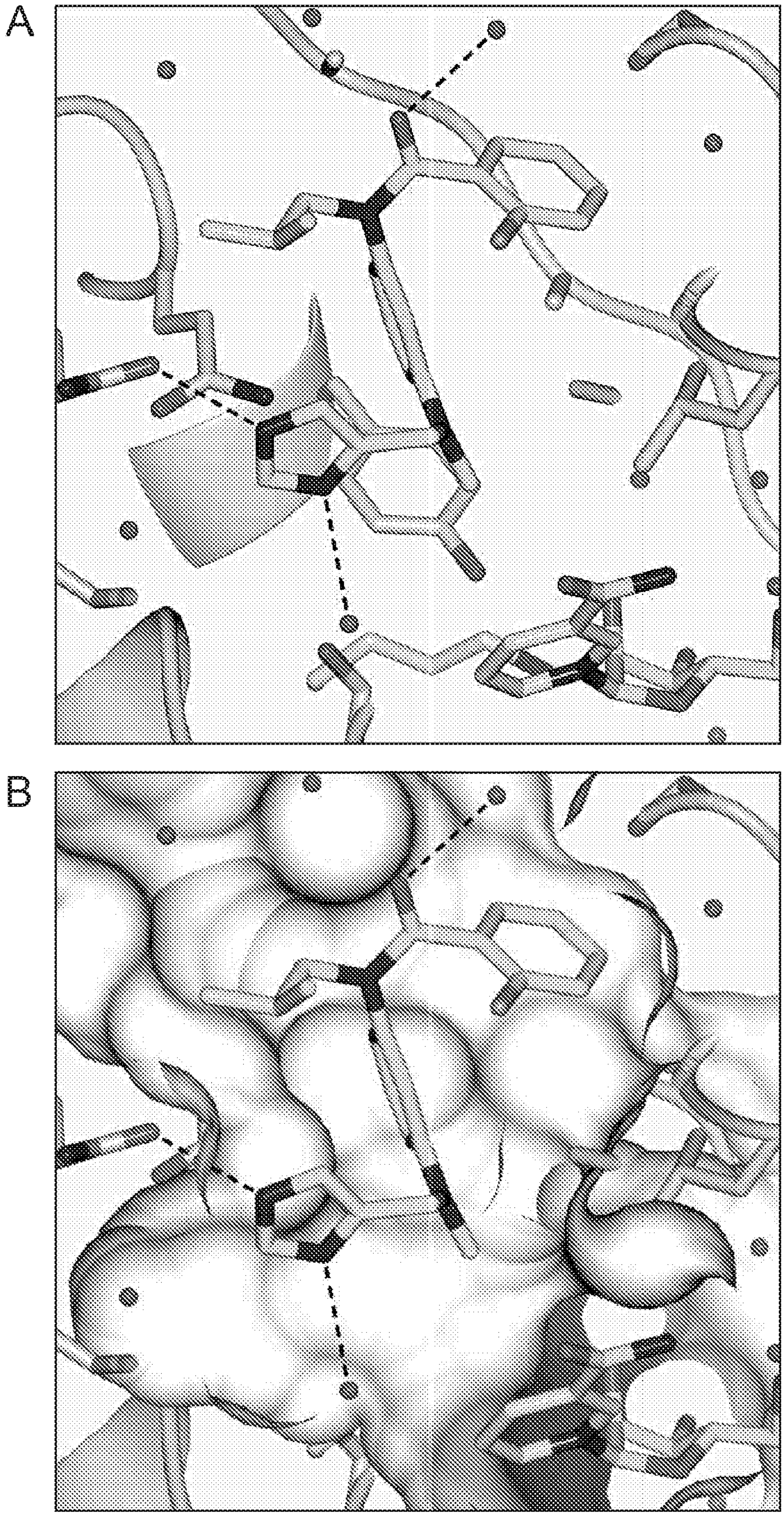

FIG. 12

A B

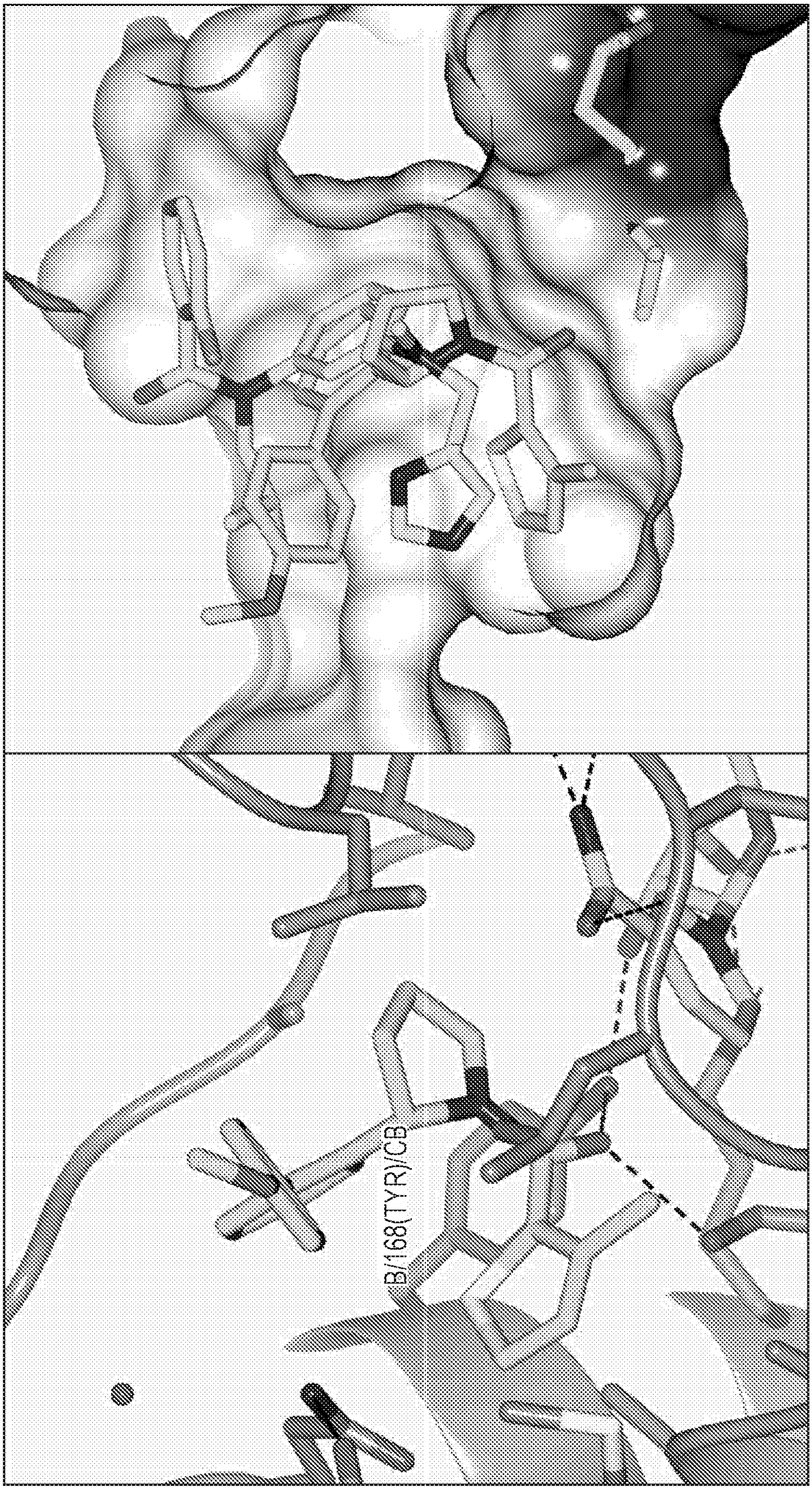


FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008100457 A
- WO 2007024944 A
- WO 2013173206 A


### Non-patent literature cited in the description


- **S. D. YAN et al.** *Nature*, 1997, vol. 389, 689-695
- **J. W. LUSTBADER et al.** *Science*, 2004, vol. 304 (5669), 448-452
- **J. YAO et al.** *Mol. Cell. Neurosci*, 2007, vol. 35 (2), 377-382
- **U. C. OPPERMANN et al.** *FEBS lett.*, 1999, vol. 451 (3), 238-242
- **S. D. YAN et al.** *J. Biol. Chem.*, 1999, vol. 274, 2145-2156
- **LIM et al.** *PLoS One*, 2011, vol. 6 (12), e28887
- **P. R. DILLARD** ; **M. F. LIN** ; **S. A. KHAN**. *Mol. Cel. Endocrinol.*, 2008, vol. 295 (1-2), 115-120
- **E. JERNBERG et al.** *PLoS One*, 2013, vol. 8 (11), e77407
- **E. CARLSON et al.** *BMC Cancer*, 2015, vol. 15, 166
- **A. ZHANG et al.** *Horm. Cancer*, 2016, vol. 7 (2), 104-113
- **L. HROCH**. *Bioorganic & Medicinal Chemistry*, 2017, vol. 25, 1143-1152
- **Y. XIE**. *Bioorganic & Medicinal Chemistry*, 2005, vol. 16, 4657-4660
- Handbook of Pharmaceutical Excipients. The Pharmaceutical Press, 2009
- **AITKEN, L et al.** *ChemBioChem*, 2016, vol. 17 (11), 1029-1037
- **AITKEN, L et al.** *SLAS Discovery*, 2017, vol. 22 (6), 676-685
- **LIPINSKI, C. A.** ; **LOMBARDO, F** ; **DOMINY, B. W.** *Adv. Drug Deliv. Rev.*, 1997, vol. 23, 3-25
- **C. R. KISSINGER et al.** *J. Mol. Biol.*, 2004, vol. 342 (3), 943-952
- **MUIRHEAD et al.** Fluorogenic Probe for Detecting Amyloid Binding Alcohol Dehydrogenase HSD10 Activity in Living Cells Kirsty. *REF: (-)-CHANA*, 2010
- **E. A. MUIRHEAD** ; **MARY FROEMMING** ; **XIAO-GUANG LI** ; **KAMIL MUSILEK** ; **STUART J. CONWAY** ; **DALIBOR SAMES** ; **FRANK J. GUNN-MOORE**. *ACS Chemical Biology*, 2010, vol. 5 (12), 1105-1114